(19) Europäisches Patentamt / European Patent Office / Office européen des brevets

(11) **EP 1 848 389 B1**

(12) **EUROPÄISCHE PATENTSCHRIFT**

(45) Veröffentlichungstag und Bekanntmachung des Hinweises auf die Patenterteilung:
**20.07.2011 Patentblatt 2011/29**

(21) Anmeldenummer: 06706967.4

(22) Anmeldetag: **15.02.2006**

(51) Int Cl.:
*A61F 9/01* *(2006.01)*  *B23K 26/36* *(2006.01)*
*B23K 26/40* *(2006.01)*  *B23K 26/00* *(2006.01)*
*B23K 26/03* *(2006.01)*  *B23K 26/073* *(2006.01)*
*B23K 26/10* *(2006.01)*

(86) Internationale Anmeldenummer:
**PCT/EP2006/001364**

(87) Internationale Veröffentlichungsnummer:
**WO 2006/087180 (24.08.2006 Gazette 2006/34)**

(54) **VERFAHREN ZUR HERSTELLUNG EINES ABLATIONSPROGRAMMS, IN ABHÄNGIGKEIT VON DER FORM EINES LASERSTRAHLPROFILS UND VON EINER NEIGUNG DER ZU ABLATIERENDEN OBERFLÄCHE ; MITTEL ZUR DURCHFÜHRUNG DER VERFAHREN**

METHOD FOR THE ESTABLISHMENT OF AN ABLATION PROGRAM AND MEANS FOR CARRYING OUT SAID METHODS

PROCEDE POUR ETABLIR UN PROGRAMME D'ABLATIO ET SYSTEME POUR METTRE EN OEUVRE LES PROCEDES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **15.02.2005 DE 102005006897**
**22.03.2005 DE 102005013252**

(43) Veröffentlichungstag der Anmeldung:
**31.10.2007 Patentblatt 2007/44**

(73) Patentinhaber: **Carl Zeiss Meditec AG**
**07745 Jena (DE)**

(72) Erfinder:
• **SLUYTERMAN VAN LANGEWEYDE, Georg**
**07749 Jena (DE)**
• **CABEZA-GUILLEN, Jesus, Miguel**
**73434 Aalen (DE)**

(74) Vertreter: **Geyer, Fehners & Partner**
**Patentanwälte**
**Perhamerstrasse 31**
**80687 München (DE)**

(56) Entgegenhaltungen:
WO-A-01/50945    WO-A-01/85075
WO-A-2005/011544    US-A- 5 186 184
US-A- 5 642 287

**Beschreibung**

Die vorliegende Erfindung bezieht sich auf ein Verfahren zur Erstellung eines Ablationsprogramms für die Ablation von Material von einer Oberfläche eines Körpers und Mittel zur Durchführung dieser Verfahren gemäß dem obenbegriff der Ansprüche 1, 33 und 35 (siehe, z.B.

WO 01/85075).

Die Ablation, das heißt die Abtragung, von Material von einer Oberfläche eines Körpers mittels eines gepulsten Laserstrahls ist grundsätzlich bekannt. Bei der Ablation wird Laserstrahlung bzw. ein Laserstrahl auf die zu ablatierende Oberfläche gelenkt, an der Material des Körpers wenigstens einen Teil der Laserstrahlung absorbiert und bei hinreichender Intensität bzw. hinreichendem Energieeintrag Material von der Oberfläche entfernt wird. Die Laserablation kann daher dazu eingesetzt werden, einen Körper berührungslos mit hoher Genauigkeit, insbesondere auch bei nur geringen Abtragstiefen, zu formen.

Zur Formgebung sind verschiedene Verfahren der Laserablation bekannt.

Bei einer Variante, die sich zum Abtrag von einem im Ablationsbereich näherungsweise sphärischen Körper eignet, werden Laserstrahlpulse auf die Oberfläche gelenkt, wobei gemäß einem vorgegebenen Ablationsprogramm der Zielort, auf den der jeweilige Puls gelenkt werden soll, und die Form und Größe des Strahlquerschnitts auf der Oberfläche, eingestellt werden. Häufig ist dabei der Zielort für alle Pulse konstant und wird dann nicht explizit angegeben.

Bei einer anderen, besonders wichtigen Variante, die auch als "spot-scanning"-Verfahren bezeichnet wird, wird Material von der Oberfläche abgetragen, indem ein gepulster Laserstrahl gemäß einem vorgegebenen Ablationsprogramm über die Oberfläche geführt wird. Unter dem Ablationsprogramm wird dabei eine Folge von Zielorten auf der Oberfläche bzw. von entsprechenden die Lagen der Zielorte wiedergebende Daten verstanden, auf die jeweils wenigstens ein Puls des Laserstrahls gelenkt werden soll. Sind die Strahl- bzw. Pulseigenschaften der verwendeten Laserstrahlung veränderbar, kann das Ablationsprogramm weiter wenigstens eine Angabe enthalten, die eine Strahl- oder Pulseigenschaft, insbesondere die Energie des Pulses oder die Fluence, das heißt die Energie des Pulses bezogen auf die bestrahlte Fläche ermittelt auf einer orthogonal zu der Richtung des Laserstrahls an der Oberfläche des Körpers angeordneten Ebene, angibt. Arbeitet der Laser während einer Ablation mit einer konstanten Pulsenergie bzw. Fluence, brauchen Daten, die die Pulsenergie oder Fluence angeben, nicht für jeden Puls bzw. Zielort angegeben zu werden.

Das Ablationsprogramm wird ausgehend von einem vorgegebenen Soll-Ablationsprofil ermittelt, d.h. der Angabe gewünschter Ablationstiefen bzw. Tiefen des Abtrags des zu ablatierenden Materials durch die Pulse in Abhängigkeit von dem Ort auf der Oberfläche. Zur Erstellung des Ablationsprogramms wird häufig davon ausgegangen, daß jeder Puls ein Einzelpuls-Ablationsvolumen abträgt, das durch den Querschnitt des Laserstrahls an der für diesen Zweck als orthogonal zur der Strahlrichtung angenommenen Oberfläche und durch die Ablationstiefe gegeben ist. Treffen mehrere Pulse an demselben Ort auf, summieren sich die Ablationstiefen, so daß insgesamt eine größere Tiefe erzielt wird. Das Ablationsprogramm wird nun so bestimmt, daß durch Abgabe der Pulse entsprechende Ablationsvolumina oder Einzelpulsvolumina an den durch das Ablationsprogramm vorgegebenen Zielorten von der Oberfläche abgetragen werden, so daß insgesamt möglichst gut das gewünschte Soll-Ablationsprofil erreicht wird.

Ein wichtiger Anwendungsbereich der Laserablation nach dem sogenannten "spot-scanning"-Verfahren ist die Laserablation von Kunststofflinsen, beispielsweise Kontaktlinsen, oder insbesondere auch von Hornhautgewebe bei der photorefraktiven Keratektomie bzw. LASIK zur Korrektur von Fehlsichtigkeit insbesondere des menschlichen Auges.

Um durch die Ablation das gewünschte Ablationsprofil, das heißt das Soll-Ablationsprofil, möglichst genau zu erhalten, ist es wesentlich, daß bei der Erstellung des Ablationsprogramms die abtragende Wirkung eines einzelnen Pulses gut bekannt ist.

Wie bereits erwähnt wird der Prozeß der Ablation durch die auf die zu bearbeitende Oberfläche auftreffende Energie pro Fläche bzw. die wirksame Fluence bestimmt, wobei eine Ablation typischerweise erst oberhalb eines materialabhängigen Schwellwertes für die Energie pro Fläche tatsächlich eintritt.

Bei Oberflächen, die gegenüber dem auf sie auftreffenden Laserstrahl geneigt sind, wurde beobachtet, daß die Ablation geringer ist als bei orthogonalem Auftreffen zu erwarten wäre. Dies kann teilweise dadurch erklärt werden, daß der von dem Laserstrahl auf der Oberfläche erzeugte Fleck bzw. Spot bedingt durch die Neigung eine größere Fläche aufweist als bei senkrechtem Einfall, wodurch die tatsächliche Fluence auf der Oberfläche gegenüber der Fluence bei senkrechtem Einfall herabgesetzt wird.

In WO 01/85075 A1 ist ein Verfahren zur Erzeugung eines Steuerprogramms beschrieben, gemäß dem ein Laserspot räumlich und zeitlich gesteuert über eine photorefraktiv zu korrigierende Hornhaut bzw. Cornea geführt wird, um ein vorbestimmtes Soll-Ablationsprofil von der Hornhaut abzutragen. Bei der Erstellung des Steuerprogramms wird der Einfluß des Winkels zwischen dem Laserstrahl und der Oberfläche der Hornhaut auf die Energiedichte des Laserspots, der auf die Oberfläche der Hornhaut auftrifft, und/oder des von der Oberfläche weg reflektierten Teils der auf die Oberfläche der Hornhaut auftreffenden Laserstrahlenergie berücksichtigt.

In WO 01/87201 A1 ist ein System zur Korrektur von optischen Fehlern eines Auges beschrieben, das einen Wellenfrontanalysator umfaßt, der auf eine von dem Auge ausgehenden Wellenfront zur Bestimmung einer optischen Wegdifferenz zwischen einer Referenzwelle und der Wellenfront anspricht. Ein Konverter liefert eine optische Korrektur auf der Basis der Wegdifferenz und einer radial abhängigen Ablationseffektivitätsfunktion. Die Ablationseffektivitätskorrektur verwendet ein kompensierendes Polynom der Form $A+B\rho+C\rho^2+D\rho^3+...+X\rho^n$, wobei $\rho$ einen normierten Radius bezeichnet, der von einem mittleren Bereich der Hornhaut gemessen wird und einen Wert von 1 an der äußeren Kante des zu korrigierenden Bereichs annimmt. Die Koeffizienten des Polynoms werden dabei durch einen Vergleich der gewünschten und der erzielten Ablationstiefe, also experimentell, bestimmt.

WO 01/50945 A1 bescheiben ein Laser-Ablationsystem und-Verfahren bei dem Fluence- oder Bestrahlungsvariationen ausgeglichen werden. Die erwähnten Verfahren lassen jedoch noch Raum für die Verbesserung der Genauigkeit der Ablation durch Verwendung eines verbesserten Ablationsprogramms.

Bei der Behandlung der Fehlsichtigkeit des menschlichen Auges durch Ablation mit einem Excimerlaser wird die Cornea bzw. Hornhaut des Auges durch Ablation so geformt, daß ein refraktiver Fehler, der die Fehlsichtigkeit bedingt, möglichst weitgehend beseitigt wird. Mit konventionellen Verfahren kann die gewünschte Refraktion in etwa 95% der Fälle bis auf etwa +/- 1 Dioptrie genau erreicht werden.

In Einzelfällen können jedoch Nachtsichtprobleme und eine Reduktion der Kontrastempfindlichkeit beim Dämmerungssehen auftreten, die auf Änderungen in der Asphärizität der Hornhaut durch die laserchlrurgische Behandlung zurückzuführen sind. Die Cornea ist bei einem gesunden bzw. nicht fehlsichtigen Auge eher gestreckt und weist eine negative Asphärizität mit Werte des Asphärizitätsparameters Q von etwa -0,25 auf. Diese Asphärizität kompensiert sphärische Aberrationen der Linse des Auges. Nach einer laserchirurgischen Behandlung von Kurzsichtigkeit ist die Cornea tendenziell eher abgeflacht, wobei die Asphärizität wesentlich größer ist als bei dem gesunden bzw. nicht fehlsichtigen Auge. Diese Abweichungen können wenigstens teilweise dadurch bedingt sein, daß das durch die Ablation erreichte Ist-Ablationsprofil von dem vorgegebenen Soll-Ablationsprofil abweicht.

Zur Verbesserung der Ablation sind in WO 95/27534 ein Verfahren und ein System zur Durchführung photorefraktiver Keratektomie zur Herstellung einer gewünschten refraktiven Korrektur des Cornea-Gewebes beschrieben. Das Verfahren und das System verwenden eine Steuerung der Wirkung von Flüssigkeit auf der Oberfläche der Cornea, um den störenden Einfluß der Flüssigkeit auf den gewünschten Ablationsprozeß unter gleichzeitiger Aufrechterhaltung des Wasserhalts der Cornea zu reduzieren. Es wird vorgeschlagen, die mittlere Wiederholrate von auf die Cornea-Oberfläche abgegebenen Pulsen zu steuern, um ein Ansammlung der Flüssigkeit zwischen den Pulsen zu reduzieren, ohne die Cornea zu dehydrieren, oder eine erhöhte Fluence des auf die Cornea-Oberfläche abgegebenen Pulses zu wählen, um die Wirkung einer Ansammlung von Flüssigkeit an der Cornea-Oberfläche zu reduzieren. Weiter wird vorgeschlagen, daß vor der Abgabe eines zur Ablation vorgesehenen Pulses auf einen Ort diesem Verdampfungsenergie zugeführt wird.

Auch diese bekannten Verfahren lassen noch Raum für die Verbesserung der Genauigkeit der Ablation durch Verwendung eines verbesserten Ablationsprogramms.

Der vorliegenden Erfindung liegt daher die Aufgabe zugrunde, ein Verfahren zur Erstellung eines Ablationsprogramms zu schaffen, das eine Ablation mit hoher Genauigkeit erlaubt, und Mittel zur Durchführung des Verfahrens bereitzustellen. Eine weitere Aufgabe besteht in der Bereitstellung eines entsprechenden Verfahrens zur Ablation von Material von einer Oberfläche eines Körpers, das eine hohe Genauigkeit der Ablation erlaubt, und von Mitteln zur Durchführung des Ablationsverfahrens.

Die Aufgabe wird nach der Erfindung gelöst durch ein Verfahren zur Erstellung eines Ablationsprogramms gemäß Anspruch 1.

Unter einem Ablationsprogramm zur Ablation zu verwendenden Ablationsprogramm wird, wie eingangs beschrieben, wenigstens eine Angabe einer Folge von Zielorten oder -richtungen verstanden, auf die bzw. in die Pulse des Laserstrahls abgegeben werden. Zusätzlich kann das Ablationsprogramm entweder für die gesamte Folge einen Wert der Pulsenergie bzw. Fluence oder für jeden Puls oder Gruppen von Pulsen jeweils einen Wert der für den Puls bzw. die Pulsgruppe zu verwendenden Pulsenergie bzw. Fluence enthalten oder vorgeben. In dem zweiten Fall können die Werte abhängig vom Zielort sein. Unter dem Ablationsprofil wird dabei eine Angabe der Tiefe der Ablation in Abhängigkeit vom Ort auf der Oberfläche oder von einer Richtung des Laserstrahls bezogen auf den Körper verstanden. Es kann sich also um ein gewünschtes, theoretisches oder tatsächliches Profil handeln.

Bei dem Verfahren wird optional die Neigung der Oberfläche verwendet. Darunter wird die Neigung der Oberfläche relativ zu der Richtung des Laserstrahls an der Oberfläche oder zu einer fest vorgegebenen Bezugsrichtung verstanden. Die Bezugsrichtung kann beispielsweise durch eine vorgegebene mittlere Richtung des Laserstrahls sein, von der der Laserstrahl um nur geringe Winkel ausgelenkt wird, um unterschiedliche Zielorte auf der Oberfläche des Körpers zu erreichen. Die Neigung der Oberfläche des Körpers ist im allgemeinen ortsabhängig und wird vorzugsweise wenigstens näherungsweise für jeden Puls ortsabhängig vom Zielort des Pulses berücksichtigt.

Die Erfindung bzw. der geschilderte Aspekt der Erfindung beruht unter anderem auf dem Gedanken, daß die tatsächlich für die Ablation wirksame Energie pro Fläche, das heißt die wirksame Fluence, eines auf die Oberfläche des Körpers auftreffenden Pulses des gepulstes Laserstrahls nicht allein von der Neigung der Oberfläche relativ zu der Richtung des Laserstrahls an der Oberfläche abhängt, sondern auch von dem Strahlprofil des Laserstrahls, insbesondere an der Oberfläche des Körpers. Unter dem Strahlprofil wird dabei der Verlauf der Intensität bzw. der auf die Fläche bezogenen Energie oder Fluence des Pulses über den Strahlquerschnitt nahe der Oberfläche verstanden. Die Form des Strahlprofils beinhaltet dabei keine absoluten Werte der Intensität oder der Energie bzw. der auf eine zum Laserstrahl senkrechte Querschnittsfläche bezogenen Fluence, sondern nur den Verlauf dieser Größen. Insbesondere eignet sich das Verfahren daher für die Ablation mit Laserstrahlen mit einem nicht-konstanten Strahlprofil.

Unter Fluence wird im folgenden die Energie der Laserstrahlung bezogen auf eine Fläche orthogonal zu der Richtung des Laserstrahls verstanden. Davon ist die für die Ablation wirksame Fluence zu unterscheiden, die im folgenden als wirksame Fluence bezeichnet wird und die Energie pro Fläche der gegebenenfalls geneigten Oberfläche bezeichnet. Die wirksame Fluence fällt mit der Fluence nur im Falle einer orthogonal zu der Oberfläche verlaufenden Richtung des Laserstrahls zusammen. Unter der Fluence eines Pulses wird der Wert an einem vorgegeben Punkt des Strahlprofils, vorzugsweise dem Zentrum, verstanden. Da die Strahlform als bekannt vorausgesetzt wird, genügt diese Angabe zur Spezifizierung des Strahlprofils.

Eine Ablation findet häufig nur statt, wenn die wirksame Fluence lokal einen materialabhängigen Schwellwert der Fluence für eine Materialabtragung überschreitet. Teile des Strahlprofils mit Werten für die wirksame Fluence unterhalb dieses Schwellwertes führen zu keiner Ablation, so daß die tatsächlich Ablation durch einen Puls signifikant von der bei bekannten Verfahren zur Erstellung eines Ablationsprogramms angenommenen Ablation abweichen kann. Bei einer Berücksichtigung allein der Neigung der Oberfläche relativ zu dem Laserstrahl und einer entsprechenden Vergrößerung des von dem Laserstrahl erzeugten Flecks bzw. Spots auf der Oberfläche wird dieser Effekt nicht berücksichtigt und kann daher zu einer ungenauen Vorhersage des durch einen einzelnen Puls ablatierten Ablationsvolumens führen. Die Berücksichtigung der Abhängigkeit der Ablation vom Strahlprofil ist deshalb insbesondere bei Verwendung von Laserstrahlen vorteilhaft, bei denen die. Fluence in Bereichen des Strahlquerschnitts den materialabhängigen Schwellwert der Fluence unterschreitet. Durch die gleichzeitige Berücksichtigung der Neigung der zu ablatierenden Oberfläche und der Form des Strahlprofils kann so ein zur Ablation zu verwendendes Ablationsprogramm erstellt werden, das bei einer Ablation gemäß dem Ablationsprogramm zu einem Ablationsprofil führt, das dem gewünschten Soll-Ablationsprofil sehr nahe kommt oder idealerweise mit diesem übereinstimmt.

Selbstverständlich kann das Ablationsprogramm noch von weiteren Größen abhängen, beispielsweise weiteren Strahlparametern wie dem Strahldurchmesser an der Oberfläche des Körpers, dem Schwellwert für die Fluence für das Einsetzen einer Ablation und weiteren Größen.

Das Erstellungsverfahren zur Erstellung eines Ablationsprogramms kann durchgeführt werden mittels einer Erstellungsvorrichtung entsprechend Anspruch 35 der Erfindung. Die Vorrichtung zur Erstellung eines Ablationsprogramms für die Ablation von Material von einer Oberfläche gemäß einem Soll-Ablationsprofil durch Abgabe von Pulsen eines gepulsten Laserstrahls auf die Oberfläche weist eine Datenverarbeitungseinrichtung auf, die zur Durchführung des erfindungsgemäßen Erstellungsverfahrens ausgebildet ist.

Die Datenverarbeitungseinrichtung kann insbesondere einen Prozessor zur Abarbeitung eines Computerprogramms aufweisen, mittels dessen das Ablationsprogramm entsprechend dem erfindungsgemäßen Erstellungsverfahren erstellbar ist, wenn das Computerprogramm auf dem Computer ausgeführt wird, sowie einen Speicher, in dem das Programm gespeichert ist.

Das Soll-Ablationsprofil kann in beliebiger Weise wiedergegeben werden. Beispielsweise kann es durch Punkte auf einem vorgegebenen Punktraster in einer Bezugsebene und den Punkten jeweils zugeordnete Ablationstiefenangaben gegeben sein. Es ist jedoch auch möglich das Soll-Ablationsprofil durch wenigstens eine durch wenigstens einen Funktionsparameter parametrisierte Funktion und einen Wert des Funktionsparameters anzugeben, wobei die Funktion und der Wert des Funktionsparameters so gewählt sind, daß die Funktion in Abhängigkeit von dem Wert des Funktionsparameters und vom Ort in der Bezugsebene die Ablationstiefe angibt. Bei einer Darstellung durch Funktionen können beispielsweise Polynome, Splines, Zernike-Polynome oder andere Darstellungen verwendet werden.

Die zur Erstellung des Ablationsprogramms notwendigen Oberflächendaten, aus denen die Neigung ermittelbar ist, können unmittelbar die Neigung der Oberfläche in Abhängigkeit von dem Ort, die Gradienten der Höhe der Oberfläche über einer vorgegebenen Bezugsebene, in der die Orte angegeben sind, oder auch die Höhen der Oberfläche über der Bezugsebene als Funktion des Ortes angeben, wobei insbesondere auch die in Bezug auf das Soll-Ablationsprofil erwähnten Darstellungsmöglichkeiten verwendet werden können. Falls diese Daten die Neigung, beispielsweise gegeben durch entsprechende Winkel, nicht explizit enthalten, kann diese Information durch Gradientenbildung und/oder die Verwendung trigonometrischer Beziehungen einfach ermittelt werden.

Je nach Anwendung können in der Datenverarbeitungseinrichtung das Soll-Ablationsprofil und/oder die Daten in Bezug auf die Oberflächenneigung gespeichert sein, was sich insbesondere anbietet, wenn mehrere gleiche Körper nacheinander ablatiert werden sollen. Wird bei verschiedenen Ablationen jeweils ein anderes Soll-Ablationsprofil verwendet, kann die Datenverarbeitungseinrichtung eine Schnittstelle zur Erfassung des Soll-Ablationsprofils umfassen, die durch entsprechende Schaltungen und/oder durch eine Softwareschnittstelle gegeben sein kann. Das Soll-Ablationsprofil kann dann z.B. über die Schnittstelle eingegeben bzw. eingelesen werden.

Können sich auch die Oberflächendaten in Bezug auf die Neigung der Oberfläche ändern, kann die erfindungsgemäße Erstellungsvorrichtung weiter über eine Schnittstelle zur Erfassung von Oberflächendaten verfügen, die die Neigung der Oberfläche direkt oder indirekt wiedergeben. Die Schnittstelle kann wie die Schnittstelle zur Erfassung des Soll-Ablationsprofils ausgebildet sein.

Die Aufgabe der Bereitstellung eines Ablationsverfahrens wird gelöst durch ein Verfahren zur Ablation, nämlich ein Verfahren zur Ablation von Material von einer Oberfläche eines Körpers gemäß einem vorgegebenen Soll-Ablationsprofil mittels eines über die Oberfläche geführten, gepulsten Laserstrahls mit einer vorgegebenen Form des Strahlprofils des Laserstrahls, bei dem mit dem erfindungsgemäßen Erstellungsverfahren ein Ablationsprogramm erstellt wird, und Pulse des Laserstrahls entsprechend dem erstellten Ablationsprogramm auf die Oberfläche gelenkt werden.

Die Pulse können dabei vorzugsweise periodisch abgegeben werden, jedoch braucht dies nicht unbedingt der Fall zu sein.

Darüber hinaus wird die Aufgabe gelöst durch eine Vorrichtung zur Ablation, nämlich eine Vorrichtung zur Ablation von Material von einer Oberfläche eines Körpers, die einen Laser zur Abgabe eines gepulsten Laserstrahls, eine Ablenkeinrichtung zur gesteuerten Ablenkung des Laserstrahls und eine erfindungsgemäße Erstellungsvorrichtung umfaßt.

Die erfindungsgemäße Ablationsvorrichtung umfaßt neben der erfindungsgemäßen Erstellungsvorrichtung einen zur Ablation geeigneten Laser und eine Ablenkeinrichtung, mittels derer ein von dem Laser abgegebener Laserstrahl auf die zu ablatierende Oberfläche gerichtet werden kann. Die Ablenkeinrichtung und der Laser sind dabei in Abhängigkeit von dem von der Erstellungsvorrichtung erstellten Ablationsprogramm steuerbar, so daß Pulse des gepulsten, von dem Laser abgegebenen Laserstrahls auf entsprechende Zielorte auf der Oberfläche gelenkt werden.

Zur Ansteuerung der Ablenkeinrichtung und/oder des Lasers kann die Erstellungsvorrichtung vorzugsweise über eine Steuereinrichtung zur Ansteuerung eines Lasers zur Abgabe des Laserstrahls und/oder einer Ablenkeinrichtung zur Ablenkung des Laserstrahls gemäß einem erstellten Ablationsprogramm verfügen. Die Steuereinrichtung kann dabei unabhängig von der Datenverarbeitungseinrichtung ausgebildet und mit dieser durch eine Datenverbindung zur Übermittlung des Ablationsprogramms verbunden sein. Vorzugsweise ist sie wenigstens teilweise durch Einrichtungen der Datenverarbeitungseinrichtung realisiert, mittels derer durch das Ablationsprogramm in Verbindung mit entsprechenden Schaltungen der Steuereinrichtung Steuerbefehle an den Laser und/oder die Ablenkeinrichtung abgegeben werden können. Vorzugsweise ist daher bei der Ablationsvorrichtung der Laser und/oder die Ablenkeinrichtung mit der Steuereinrichtung über eine entsprechende Steuerverbindung verbunden. Hierdurch kann ein besonders einfacher Aufbau erreicht werden.

Die Berücksichtigung der Neigung der Oberfläche und der Form des Strahlprofils des Laserstrahls kann auf unterschiedliche Art und Weise erfolgen. Um konventionelle Verfahren zur Erstellung von Ablationsprogrammen und insbesondere Verfahren zur Erstellung von Ablationsprogrammen, die von einem konstanten Strahlprofil, einem sogenannten "top-hat"-Profil, ausgehen, verwenden zu können, kann die Berücksichtigung des Neigung der Oberfläche und der Form des Strahlprofils im Wege einer Vorkompensation der bei einfachen Erstellungsverfahren auftretenden Fehler erfolgen, bei der das Soll-Ablationsprofil in geeigneter Weise modifiziert wird. Dazu ist es bevorzugt, daß zur Berücksichtigung der Neigung der Oberfläche und der Form des Strahlprofils aus dem Soll-Ablationsprofil in Abhängigkeit von wenigstens der Form des Strahlprofils und der Neigung der Oberfläche an einem jeweiligen Zielort auf der Oberfläche ein modifiziertes bzw. vorkompensiertes Soll-Ablationsprofil ermittelt wird, und aus dem modifizierten bzw. vorkompensierten Soll-Ablationsprofil das Ablationsprogramm erstellt wird. Zur Ermittlung des Ablationsprogramms aus dem modifizierten bzw. vorkompensierten Soll-Ablationsprofil können einfache Erstellungsverfahren verwendet werden, unter denen im Rahmen der vorliegenden Erfindung Erstellungsverfahren verstanden werden, die zur Erstellung des Ablationsprofils nicht zugleich das Strahlprofil und die Oberflächenneigung verwenden. Beispiele für solche einfache Erstellungsverfahren sind in DE 197 27 573 C1 oder EP 1060710 A2 beschrieben, deren Inhalt hiermit insoweit durch Bezugnahme in die Beschreibung aufgenommen wird.

Das Soll-Ablationsprofil wird dabei vorzugsweise so modifiziert, daß die Ablation gemäß dem auf der Basis des modifizierten Soll-Ablationsprofil erstellten Ablationsprogramm wenigstens näherungsweise zu dem gewünschten Soll-Ablationsprofil führt. Die Fehler, die bei Verwendung eines einfachen Erstellungsverfahrens zur Erstellung eines Ablationsprogramms durch die Nichtberücksichtigung des Strahlprofils und der Neigung der Oberfläche relativ zu dem Strahl auftreten, können durch eine entsprechende Modifikation des Soll-Ablationsprofils, das heißt der Ausgangsdaten zur Erstellung des Ablationsprogramms, so kompensiert werden, daß in der Folge ein einfaches Erstellungsverfahren verwendet werden kann. Dabei können die Art und der Umfang der Vorkompensation bzw. Modifikation von den Eigenschaften des verwendeten einfachen Erstellungsverfahrens bzw. von den diesem zugrundeliegenden Modellannahmen abhängen. Auf diese Art kann eine Erhöhung der Genauigkeit der Ablation erzielt werden, wobei auf bekannte einfache Erstellungsverfahren zurückgegriffen werden kann.

Dazu ist es bevorzugt, das für wenigstens zwei zu ablatierende Bereiche der Oberfläche jeweils ein Wert einer Modifikationsfunktion in Abhängigkeit von wenigstens der Form des Strahlprofils und der Neigung der Oberfläche in dem jeweiligen Bereich ermittelt werden und daß das modifizierte bzw. vorkompensierte Soll-Ablationsprofil unter Verwendung des Soll-Ablationsprofils und der Werte der Modifikationsfunktion ermittelt wird. Die Erstellung des Ablationsprogramms kann dabei insbesondere durch ein einfaches Erstellungsverfahren erfolgen. Die Werte der Modifikationsfunktion werden vorzugsweise für jeden Puls bzw. für jeden Ort auf der Oberfläche, auf den ein Puls gelenkt werden soll, berechnet. Die Werte können für jeden Bereich bzw. Zielort jeweils neu berechnet werden. Es ist jedoch auch möglich, die Werte nur einmal zu berechnen und dann in einer Tabelle, beispielsweise in Abhängigkeit von der Neigung, abzulegen, aus der sie dann jeweils bei Bedarf ausgelesen werden können.

Vorzugsweise hängt die Modifikationsfunktion zusätzlich von der der Intensität, der Energie oder der Fluence der bei der Ablation zu verwendenden Pulse ab. Insbesondere kann die Modifikationsfunktion auch von einem Schwellwert für die Energie oder wirksame Fluence abhängen, der angibt, ab welcher Energie bzw. wirksamen Fluence eine Ablation des Materials auftritt.

Insbesondere kann die Modifikationsfunktion von dem dimensionslosen Verhältnis der Fluence und des Fluence-Schwellwerts und/oder dem Neigungswinkel abhängen.

Gemäß einer anderen Alternative, bei der ebenfalls einfache Verfahren zur Erstellung von Ablationsprogrammen verwendet werden können, ist vorgesehen, daß aus dem Soll-Ablationsprofil ein vorläufiges Ablationsprogramm ermittelt wird, für wenigstens einen der abzugebenden Pulse ein Soll-Wert für dessen Energie oder Fluence in Abhängigkeit von der Form des Strahlprofils, der Neigung der Oberfläche in dem Bereich, auf den der Puls abgegeben werden soll, und von einer Vorhersage der Ablationstiefe bei Verwendung des vorläufigen Ablationsprogramms ermittelt wird, und das erstellte Ablationsprogramm den Zielort des Pulses gemäß dem vorläufigen Ablationsprogramm und den ermittelten Soll-Wert für die Energie oder Fluence des Pulses umfaßt. Dabei wird die Pulsenergie oder Fluence nahe der Oberfläche ermittelt. Das bedeutet, daß nicht das Soll-Ablationsprofil modifiziert zu werden braucht. Vielmehr wird eine auftretende Reduktion der tatsächlichen Ablationstiefe gegenüber einer Ablationstiefe, die bei senkrechtem Auftreffen des Laserstrahls auf die Oberfläche und einem konstanten Strahlprofil angenommen wird, durch eine Änderung, beispielsweise Erhöhung, der Energie bzw. Fluence der Pulse berücksichtigt: Pulse werden also später bei der Ablation auf einen vorgegebenen Bereich mit einer dessen Neigung und dem verwendeten Strahlprofil entsprechenden Energie abgegeben. Die Angabe der Pulsenergie zusammen mit dem jeweiligen Ort, auf den der Puls zu lenken ist, ist dann ein Teil des abschließend erstellten, für die Ablation zu verwendenden Ablationsprogramms.

Die Einstellung der Energie oder Fluence eines Pulses kann dabei auf wenigstens drei Weisen erfolgen, die alternativ oder in Kombination angewandt werden können. Bei einer ersten Variante wird zur Einstellung der Energie oder Fluence eines Pulses der Laserstrahl abgeschwächt. Hierzu ist vorzugsweise die Steuereinrichtung zur Ansteuerung eines den Laserstrahl schwächenden Modulators ausgebildet. Die erfindungsgemäße Ablationsvorrichtung weist dazu neben der Steuereinrichtung einen mit der Steuereinrichtung verbundenen Modulator auf, der den Laserstrahl auf Ansteuerung durch die Steuereinrichtung abschwächt. Als Modulator kann dabei jegliche Einrichtung dienen, mit der die Intensität des von dem Laser abgegebenen Laserstrahls reduziert werden kann. Beispielsweise können Pockels-Zellen oder Flüssigkristallelemente mit steuerbarem Transmissionsvermögen verwendet werden. Der Modulator kann dabei beispielsweise polarisationsabhängig oder auch wellenlängenabhängig wirken. Die Verwendung von Modulatoren erlaubt eine einfache Einstellung der Pulsenergie bei beliebigen Lasertypen.

Bei der zweiten Variante kann zur Einstellung der Energie oder Fluence eines Pulses der zur Abgabe des gepulsten Laserstrahls verwendete Laser gesteuert werden. Hierzu kann insbesondere die Steuereinrichtung entsprechend zur Ansteuerung des Lasers ausgebildet sein. Bei der erfindungsgemäßen Abtationsvorrichtung ist dann vorzugsweise der Laser mittels der Steuereinrichtung steuerbar. Bei Verwendung von Excimerlasern kann die Steuereinrichtung insbesondere die Hochspannung zur Aufladung eines Kondensators bzw. mehrerer Kondensatoren zur Speicherung von Energie zur Erzeugung eines Laserpulses steuern. Die Steuerung der Pulsenergie oder Fluence des Lasers hat den Vorteil, daß weniger Energie benötigt wird.

Als dritte Variante kann zur Einstellung der Fluence eines Pulses der Strahlquerschnitt des Laserstrahls an der Oberfläche geändert werden. Die erfindungsgemäße Ablationsvorrichtung kann hierzu eine entsprechende strahlformende optische Einrichtung besitzen, die von der Steuereinrichtung angesteuert wird.

Soll eine möglichst hohe Genauigkeit bei der Ablation, das heißt eine möglichst geringe Abweichung zwischen Soll-Ablationsprofil und Ist-Ablationsprofil, erreicht werden, ist es bevorzugt, daß aus dem Soll-Ablationsprofil ein vorläufiges Ablationsprogramm ermittelt wird, unter Verwendung des vorläufigen Ablationsprogramms ein vorhergesagtes Ablationsprofil in Abhängigkeit von der Form des Strahlprofils und der Neigung der Oberfläche vorhergesagt wird, und unter Verwendung des vorhergesagten Ablationsprofils das zu verwendende Ablationsprogramm erstellt wird. Die Vorhersage des Ablationsprofils bzw. der Ablationstiefe kann dabei mit einem geeigneten Modell erfolgen. Dieses Vorgehen erlaubt eine hohe Genauigkeit, da ein Vergleich des vorhergesagten Ablationsprofils und des Soll-Ablationsprofil erfolgen kann. Insbesondere bei Verwendung einer Modifikationsfunktion oder einer geänderten Fluence oder Pulsenergie kann die Modifikationsfunktion bzw. die Fluence oder Pulsenergie vorzugsweise für jeweils einen Zielort eines Pulses und damit den gesamten Puls ermittelt werden. Die Modifikationsfunktion wird dann als für den gegebenen Puls räumlich konstant angenommen, was eine gute Näherung darstellt, wenn die Änderung der Neigung relativ zu der Größe des Abstands benachbarter Auftreffpunkte von Laserpulsen gering ist. Dies fügt sich insbesondere in das Vorgehen bei einfachen Erstellungsverfahren ein, bei denen nur Einzelpuls-Ablationsvolumina betrachtet werden. Dieses Vorgehen hat den Vorteil einfach zu sein. Insbesondere bei starken Änderungen der Oberflächenneigung ist es jedoch bevorzugt, daß das vorhergesagte Ablationsprofil an wenigstens zwei Punkten ermittelt wird, die weniger als der Durchmesser des Laserstrahls auf der Oberfläche des Körpers voneinander beabstandet sind. Mit anderen Worten wird das vorhergesagte Ablationsprofil mit einer Ortsauflösung ermittelt, die besser ist als der Durchmesser des Laserstrahls auf der Oberfläche des Körpers. Das vorhergesagte Ablationsprofil kann beispielsweise für Punkte eines Punktgitters verwendet werden, dessen Gitterabstand kleiner als der Durchmesser des Laserstrahls ist. Diese Verfahrensvariante hat den Vorteil, daß jegliche Änderung der Neigung der Oberfläche berücksichtigt werden kann.

Bei der Verfahrensvariante, bei der ein Ablationsprofil vorhergesagt wird, wird vorzugsweise unter Verwendung des vorhergesagten Ablationsprofils und des vorgegebenen Soll-Ablationsprofils ein vorkompensiertes Soll-Ablationsprofil ermittelt und das zu verwendende Ablationsprogramm aus dem vorkompensierten Soll-Ablationsprofil erstellt. Dies Alternative hat den Vorteil, daß nach Erstellung des vorkompensierten Soll-Ablationsprofil ein einfaches, beispielsweise bekanntes Erstellungsverfahren zur Erstellung des zu verwendenden Ablationsprogramms verwendet werden kann. Die bei einfachen Erstellungsverfahren nicht berücksichtigten Einflüsse der Form des Strahlprofils und/oder der Neigung der Oberfläche werden durch die entsprechende Vorkompensation, die von der Form des Strahlprofils und der Neigung der Oberfläche abhängt, berücksichtigt, so daß bei Ablation gemäß dem zu verwendenden Ablationsprogramm wenigstens näherungsweise das Soll-Ablationsprofil ablatiert wird.

Das geschilderte Vorgehen unter Verwendung eines vorhergesagten Ablationsprofils erlaubt bereits eine gute Vorkompensation. Zur Erzielung einer besonders hohen Genauigkeit erfolgt bevorzugt die Erstellung des zu verwendenden Ablationsprogramms iterativ, indem in wenigstens einer aktuellen Iterationsschleife aus einem in einer vorhergehenden Iterationsschleife ermittelten modifizierten Soll-Ablationsprofil ein vorläufiges Ablationsprogramm ermittelt wird, ausgehend von dem vorläufigen Ablationsprogramm in Abhängigkeit von der Form des Strahlprofils und der Neigung der Oberfläche ein aktuelles vorhergesagtes Ablationsprofil vorhergesagt wird, unter Verwendung des aktuellen vorhergesagten Ablationsprofils ein aktuelles modifiziertes Soll-Ablationsprofil ermittelt wird und nach der letzten Iterationsschleife das zu verwendende Ablationsprogramm in Abhängigkeit von dem vorgegebenen Soll-Ablationsprofil und wenigstens einem der modifizierten Soll-Ablationsprofile erstellt wird. Bei diesem iterativen Verfahren kann eine sukzessive Verbesserung des Ablationsprogramms und damit eine besonders hohe Genauigkeit erzielt werden. In der ersten Iterationsschleife kann statt eines modifizierten Soll-Ablationsprofils das Soll-Ablationsprofil verwendet werden.

Vorzugsweise wird bei der Verwendung eines vorkompensierten bzw. modifizierten Soll-Ablationsprofils für wenigstens zwei zu ablatierende Bereiche der Oberfläche jeweils ein Wert einer Modifikationsfunktion in Abhängigkeit von wenigstens dem Wert des vorhergesagten Ablationsprofils und des vorgegebenen Soll-Ablationsprofils ermittelt. Das vorkompensierte Soll-Ablationsprofil wird dann unter Verwendung des vorgegebenen Soll-Ablationsprofils und der Werte der Modifikationsfunktion ermittelt. Bei einem iterativen Vorgehen kann die Modifikationsfunktion insbesondere in Abhängigkeit von dem vorgegebenen Soll-Ablationsprofil und den modifizierten Ablationsprofilen ermittelt werden, so daß nach der letzten Iterationsschleife zunächst das vorkompensierte Soll-Ablationsprofil unter Verwendung der Modifikationsfunktion und über die Modifikationsfunktion in Abhängigkeit von dem vorgegebenen Soll-Ablationsprofil und den modifizierten Soll-Ablationsprofilen ermittelt und dann aus dem vorkompensierten Soll-Ablationsprofil das zu verwendende Ablationsprogramm, beispielsweise unter Verwendung eines einfachen Erstellungsverfahrens, erstellt wird. Die Verwendung einer Modifikationsfunktion erlaubt eine besonders einfache Berücksichtigung der Einflüsse der Form des Strahlprofils und der Neigung der Oberfläche. Die Modifikationsfunktion kann dabei in analytischer Form gegeben sein und dann insbesondere explizit von der Intensität, der Energie oder der Fluence der bei der Ablation zu verwendenden Pulse abhängen oder durch Werte an Stützstellen des Soll-Ablationsprofils oder Stützstellen zur Beschreibung der Oberfläche, die numerisch ermittelt werden.

Die Iteration kann beispielsweise nach einer vorgegebenen Anzahl von Iterationsschleifen abgebrochen werden. Vorzugsweise wird jedoch in wenigstens einer Iteration als Abbruchkriterium geprüft, ob das in der einer vorhergehenden Iteration ermittelte vorhergesagte Ablationsprofil gemäß einem vorgegebenen Kriterium mit dem in der aktuellen Iterationsschleife vorhergesagten Ablationsprofil übereinstimmt, und bei Feststellung einer Übereinstimnung werden keine weiteren Iterationsschleifen durchlaufen. Als Kriterium kann dabei beispielsweise geprüft werden, ob das Verhältnis aus den vorhergesagten Ablationsprofilen für alle Orte in dem zu ablatierenden Bereich in zwei aufeinanderfolgenden Iterationsschleifen um weniger als ein vorgegebener Grenzwert von 1 abweicht. Alternativ kann beispielsweise geprüft werden, ob die Differenzen der vorhergesagten Ablationsprofile für alle Orte in dem zu ablatierenden Bereich in zwei aufeinanderfolgenden Iterationsschleifen geringer als ein anderer vorgegebener Grenzwert sind.

Das vorhergesagte Ablationsprofil ist unter Umständen, bedingt durch die Berechnung an diskreten Orten, beispielsweise auf einem Gitter, nicht glatt, sondern weist Spitzen auf, die Artefakte darstellen. Es ist daher bevorzugt, daß vor der Erstellung des aktuellen Ablationsprogramms das entsprechende vorhergesagte Ablationsprofil oder eine aus diesem ermittelte Funktion, insbesondere das vorkompensierte Soll-Ablationsprofil oder die durch die Werte der Modifikationsfunktion gegeben Funktion, geglättet wird. Hierzu können insbesondere entsprechende Ausgleichsverfahren oder Tiefpaßfilter verwendet werden.

Um den Einfluß der Form des Strahlprofils und der Oberflächenneigung, z.B. in Form der Modifikationsfunktion oder der Ermittlung des vorhergesagten Ablationsprofils, möglichst genau berücksichtigen zu können, wird bevorzugt ein Modell verwendet, mittels dessen für einen Puls in Abhängigkeit von der Form des Strahlprofils der Verlauf der Ablationstiefe vorhersagbar ist. Dabei können Modelle Verwendung finden, die entweder das gesamte durch einen Puls ablatierte Volumen oder sogar lokal die Ablationstiefe als Funktion wenigstens der Fluence an dem Ort mit einer Auflösung besser als der Strahlquerschnitt wiedergeben. Solche Modelle sind grundsätzlich bekannt.

Insbesondere in dem Fall, daß die Erstellung des Ablationsprogramms mit dem erfindungsgemäßen Verfahren im Vergleich zu der Dauer der eigentlichen Ablation relativ viel Zeit erfordert, empfiehlt es sich, daß das Ablationsprogramm vor Beginn der Ablation erstellt wird. Das Ablationsprogramm, das heißt die Folge der Zielorte, auf die Pulse gelenkt werden sollen, und gegebenenfalls die entsprechenden Pulsenergien, kann dann zwischengespeichert werden, um später zur Durchführung der eigentlichen Ablation sukzessive ausgelesen zu werden.

Erfolgt eine Änderung der Fluence bzw. Energie der Pulse in Abhängigkeit von der Neigung der Oberfläche und der Form des Strahlprofils, ist es bevorzugt, daß für wenigstens zwei Pulse deren Energie oder Fluence in Abhängigkeit von der Form des Strahlprofils und der Neigung der Oberfläche in dem jeweiligen Bereich, auf den der jeweilige Puls abgegeben wird, während der Ablation ermittelt wird. Insbesondere kann zunächst nur ein vorläufiges, beispielsweise mit einem einfachen Erstellungsverfahren erstelltes Ablationsprogramm vorliegen, das die Zielorte, auf die Pulse zu lenken sind, angibt. Für jeden der Zielorte kann dann entsprechend die Pulsenergie bzw. Fluence ermittelt und die Pulsenergie bzw. Fluence gesteuert werden. Das Ablationsprogramm umfaßt also die Zielorte des vorläufigen Ablationsprogramms ergänzt durch die entsprechenden Vorgaben für die Pulsenergien bzw. Fluencewerte. Ein Vorteil dieser Ausführungsform liegt darin, daß sie eine teilweise Erstellung des zu verwendenden Ablationsprogramms während der Ablation ermöglicht, wobei insbesondere auch Formänderungen des Strahlprofils während der Ablation berücksichtigt werden können.

Das Strahlprofil braucht nicht unbedingt zeitlich variabel zu sein. Vielmehr kann die Form des Strahlprofils für eine gegebene Ablationsvorrichtung gleichbleibend sein, so daß bei dem Erstellungsverfahren immer die gleiche Form des Strahlprofils verwendet werden kann, die dann z.B. in der Erstellungsvorrichtung gespeichert sein kann. Es ist alternativ möglich, daß die funktionale Form des Strahlprofils, beispielsweise entsprechend einer parametrisierten Gauß-Funktion, fest vorgegeben ist, wobei jedoch ein die Form bestimmender Parameter, beispielsweise bei einer Gauß-Funktion die Halbwertsbreite, eingelesen wird. Um jedoch eine beliebige Variabilität der Form des Strahlprofils während der Ablation oder auch bei aufeinanderfolgenden Ablationen berücksichtigen zu können, ist es bei der erfindungsgemäßen Erstettungsvorrichtung bevorzugt, daß die Datenverarbeitungseinrichtung eine Schnittstelle zur Eingabe von Daten, die die Form des Strahlprofils des Laserstrahls charakterisieren, umfaßt. Auch hier kann die Schnittstelle eine physische und/oder Software-Schnittstelle umfassen. Das Strahlprofil kann dabei wiederum entweder in Form von Werten für die Intensität, Energie oder Fluence in Abhängigkeit von der Lage im Strahlprofil oder durch Angabe von Werten von Formparametern einer durch die Formparameter parametrisierten Funktionen, die die Form des Strahlprofils wiedergibt, beschrieben werden. Die Schnittstelle kann insbesondere zur manuellen Eingabe von Daten und/oder zur automatischen Übertragung von Daten über eine Datenverbindung ausgebildet sein.

Um die Erfassung eines Strahlprofils zu erleichtern, ist es bevorzugt, daß die Erstellungsvorrichtung eine Einrichtung zur Erfassung der Form des Strahlprofils des Laserstrahls umfaßt. Diese Vorrichtung erfaßt nicht nur die Form des Strahlprofils, sondern ermöglicht vorzugsweise auch die Erfassung wenigstens eines weiteren Strahlparameters, beispielsweise des Querschnitts des Laserstrahls. Bei dem Verfahren ist es dann bevorzugt, daß zur Erstellung des Ablationsprogramms das Strahlprofil, vorzugsweise automatisch, vermessen wird, wozu die Einrichtung zur Erfassung des Strahlprofils verwendet werden kann. Die Erfassung oder Vermessung kann vor der Erstellung des Ablationsprogramms oder auch, wenn die Erstellung des Ablationsprogramms schnell genug erfolgt, während der Erstellung des Ablationsprogramms bzw. während der Ablation durchgeführt werden. Wird ein Computerprogramm zur Durchführung des Erstellungsverfahrens verwendet, weist dieses hierzu entsprechende Befehle zur Ansteuerung der Einrichtung zur Erfassung des Strahlprofils und zum Einlesen entsprechender Daten auf.

Besonders bevorzugt verfügt die Einrichtung hierzu über einen mit der Datenverarbeitungseinrichtung verbundenen ortsauflösenden Detektor für die Laserstrahlung und einen in dem Strahlengang des Laserstrahls angeordneten Strahlteiler, der einen Teil des Laserstrahls auf den Detektor ablenkt.

Alternativ können Erfassungsverfahren mit einer bewegter Schlitz-, Loch- oder Kantenblende verwendet werden, die in geeigneter Weise vor einem dahinter befindlichen Strahlungsdetektor scannend bewegt wird, wobei aus den Meßwerten dann das Strahlprofil ermittelt wird. Ein solches Verfahren ist beispielsweise in US 6,666,855 beschrieben.

Zur Erstellung des Ablationsprogramms ist es notwendig, die Neigung der Oberfläche in dem zu ablatierenden Bereich zu kennen. Liegen Oberflächen mit einfacher Geometrie vor, so ist eine manuelle Eingabe entsprechender Parameter der Oberfläche, aus denen die Neigung ortsabhängig ermittelbar ist, möglich. Beispielsweise können bei einer Kugelgeometrie, wie sie bei der Cornea vorliegt, der Krümmungsradius, bei einer torischen Fläche die beiden Hauptkrümmungen, bei einem lateralen Versatz der oben genannten Flächen auch die Koordinaten der Mittenpositionen (Kugel- bzw. Torusachse), bei einer einfachen geneigten ebenen Fläche Neigungswinkel und Neigungsrichtung und bei asphärischen Flächen wie Oberflächenbereichen eines Ellipsoids ein Asphärizitätsparameter angegeben werden.

Die entsprechenden Daten können einfach über die entsprechende Schnittstelle eingelesen werden. Es ist jedoch bevorzugt, daß bei dem erfindungsgemäßen Erstellungsverfahren zur Erstellung des Ablationsprogramms Topographiedaten der Oberfläche erfaßt werden. Die erfaßten Oberflächentopographiedaten geben die Neigung der Oberfläche direkt wieder oder erlauben eine Ermittlung der Neigung. Grundsätzlich kann die Schnittstelle zur Erfassung der Oberflächendaten zur manuellen Eingabe der Daten oder zum Einlesen der Daten von einem vorgegebenen Datenträger bzw. einer entsprechenden Einrichtung ausgebildet sein. Vorzugsweise werden die Daten jedoch automatisch erfaßt. Dies hat den Vorteil, daß es dann leichter möglich ist, ein Koordinatensystem, in dem das Soll-Ablationsprofil vorgegeben ist, und ein Koordinatensystem, in dem die Oberflächendaten angegeben sind, zueinander in Beziehung zu setzen. Die Erstellungsvorrichtung weist hierzu vorzugsweise eine Einrichtung zur Erfassung von Topographiedaten der zu ablatierenden Oberfläche auf. Diese kann von der Datenverarbeitungseinrichtung angesteuert werden, um die Daten automatisch zu erfassen. Insbesondere ist die Einrichtung zur Erfassung von Topographiedaten zur Erfassung von Daten in Bezug auf die Neigung der zu ablatierenden Oberfläche geeignet. Oberflächentopographiedaten bzw. Neigungen können beispielsweise mit Placido-Ring-Verfahren bzw. -Einrichtungen, Streifenprojektionsverfahren bzw. -einrichtungen oder auch mit optischen Kohärenztomographen ermittelt werden.

Das Soll-Ablationsprofil kann, wie die Oberflächentopographiedaten auch, grundsätzlich nach vorheriger Ermittlung manuell, von einem Datenträger oder über eine Datenverbindung eingelesen werden. Es ist jedoch bevorzugt, daß zur Erstellung des Ablationsprogramms Daten zur Ermittlung des Soll-Ablationsprofils, vorzugsweise automatisch, erfaßt werden. Die Erstellungsvorrichtung umfaßt hierzu vorzugsweise eine Einrichtung zur Erfassung von Daten zur Ermittlung des Soll-Ablationsprofils. Diese wird vorzugsweise von der Datenverarbeitungseinrichtung angesteuert. Hierbei können beispielsweise im Fall der Korrektur von Fehlsichtigkeit entsprechende diagnostische Geräte und Verfahren zur Ermittlung der Brechkraft des Auges und insbesondere auch Aberrometer bzw. Wellenfrontanalysatoren, beispielsweise nach Hartmann-Shack, verwendet werden. Berechnungen können entweder über einen separaten, geeignet programmierten Prozessor oder vorzugsweise unter Verwendung der ohnehin in der Vorrichtung vorhandenen Datenverarbeitungseinrichtung mittels entsprechender Computerprogrammmodule ausgeführt werden.

Dabei kann es sich als vorteilhaft erweisen, wenn Daten eingelesen werden, die die Lage eines Bezugspunkts eines Koordinatensystems für die zu ablatierende Oberfläche und/oder eines Bezugspunkts eines Koordinatensystems für das Soll-Ablationsprofil wiedergeben. Insbesondere können auch Relativlagen dieser Bezugspunkte zueinander oder die Lagen in einem gemeinsamen Koordinatensystem angegeben werden. Vorzugsweise werden als Bezugspunkte die entsprechenden Koordinatenursprünge verwendet. Die Vorrichtung umfaßt hierzu vorzugsweise eine Eingabeschnittstelle, mittels derer ein Bezugspunkt eines Koordinatensystems für die zu ablatierenden Oberfläche und/oder ein Bezugspunkt eines Koordinatensystems für das Soll-Ablationsprofil eingebbar sind. Ist die zu ablatierende Form nicht rotationssymmetrisch, können darüber hinaus gegebenenfalls auch noch Daten in Bezug auf die Ausrichtung der Koordinatensysteme aufeinander eingelesen werden. Die Datenverarbeitungseinrichtung ist vorzugsweise dazu ausgebildet, die Daten des Soll-Ablationsprofils bzw. der Oberflächentopographie bzw. -neigung in ein gemeinsames Koordinatensystem zu überführen.

Werden die die Oberflächentopographie bzw. -neigung wiedergebenden Daten und die Soll-Ablationsprofildaten automatisch ermittelt, kann das erfindungsgemäße Verfahren insbesondere auch bei Ablationsverfahren verwendet werden, bei denen während der Ablation die Oberflächentopographie und optische Eigenschaften der Oberfläche ermittelt und in Abhängigkeit davon ein temporäres Soll-Ablationsprofil ermittelt und wenigstens teilweise ablatiert wird. Ein solches Verfahren ist beispielsweise in der WO 01/12113 A1 beschrieben, deren Inhalt insoweit hiermit durch Bezugnahme in die Beschreibung aufgenommen wird.

Neben dem Strahlprofil und der Neigung können, insbesondere bei biologischem Material wie beispielsweise der Hornhaut des Auges, noch individuelle Unterschiede in den Eigenschaften des Hornhautmaterials und gegebenenfalls bei oder nach der Behandlung durch die Behandlung auftretende Änderungen der Form Oberfläche auftreten. Es ist daher bevorzugt, daß die Vorkompensation in Abhängigkeit von einer von dem individuellen Körper abhängigen Ablationseigenschaft des Materials und/oder einer zu erwartenden Änderung der Oberflächen- und/oder Materialeigenschaften bei und/oder infolge der Ablation durchgeführt wird. Unter einer Oberflächeneigenschaft wird hierbei auch eine Formänderung verstanden. Insbesondere kann hierzu ein entsprechender Korrekturfaktor zusätzlich zu der neigungsabhängigen Modifikationsfunktion verwendet werden. Es können damit in vorteilhafter Weise Effekte wie der Wassergehalt des Materials oder, insbesondere im Falle einer Behandlung von Material in einem lebenden Körper, eine Änderung der Oberflächenform nach der Ablation durch Heilungseffekte berücksichtigt werden.

Um eine noch genauere Ablation durchführen zu können, ist es bei dem Erstellungsverfahren, bei dem das Material dann wasserhaltig ist, bevorzugt, daß bei der durchzuführenden Ablation der Laserstrahl über die Oberfläche geführt wird, und daß das Ablationsprogramm ausgehend von dem Soll-Ablationsprofil zusätzlich unter Berücksichtigung eines Wassergehalts des zu ablatierenden Materials erstellt wird. Auf diese Weise kann der Einfluß des Wassers auf das Ablationsverhalten zusätzlich berücksichtigt werden.

Zur Estellung des Ablationsprogramms eignet sich ein Computerprogramm mit Programmcode gemäß Anspruch 33, um das erfindungsgemäße Erstellungsverfahren durchzuführen, wenn das Programm auf einem Computer ausgeführt wird.

Darüber hinaus ist Gegenstand der Erfindung ein Computerprodukt mit Programmcode, der auf einem computerlesbarem Datenträger gespeichert ist, um das erfindungsgemäße Erstellungsverfahren durchzuführen, wenn das Computerprogrammprodukt auf einem Computer ausgeführt wird. Unter einem Datenträger wird hierbei ein beliebiger Datenträger verstanden, insbesondere ein magnetische Datenträger, wie beispielsweise eine Diskette oder Festplatte, ein magnetooptische Datenträger, ein optische Datenträger wie beispielsweise ein CD, DVD oder Blue-Ray-Disc, oder auch ein anderer nicht-flüchtige Speicher, wie beispielsweise ein sogenannter Flash-ROM.

Unter einem Computer wird hierbei eine beliebige Datenverarbeitungsvorrichtung, insbesondere eine Datenverarbeitungseinrichtung der Erstellungsvorrichtung, verstanden, mit der das Verfahren ausgeführt werden kann, wenn das Programm darauf ausgeführt wird. Insbesondere kann diese einen digitalen Signalprozessor und/oder einen programmierbaren Mikroprozessor aufweisen, mit dem das Verfahren ganz oder in Teilen ausgeführt wird. Der Prozessor kann mit einer Speichereinrichtung verbunden sein, in der das Computerprogramm und/oder Daten zur Ausführung des Computerprogramms gespeichert sind.

Wie eingangs bemerkt, kann beispielsweise bei Verwendung eines Laserstrahls mit einem stark variierenden Strahlprofil, beispielsweise einem Gaußschen Strahlprofil, in einigen Bereichen, beispielsweise an den Rändern, des durch den Laserstrahl auf der Oberfläche erzeugten Laserspots bzw. des Strahlprofils ein nicht unerheblicher Teil der Fluence unter dem Schwellwert für die Ablation von Material liegen. Diese Pulsanteile führen dann nicht zur Ablation, sondern nur zu einer häufig unerwünschten Erwärmung des Materials.

Es ist daher bevorzugt, daß bei dem erfindungsgemäßen Ablationsverfahren ein Laserstrahl verwendet wird, der wenigstens nahe der Oberfläche des Körpers ein Strahlprofil aufweist, das über den gesamten Querschnitt eine vorgegebene Mindestintensität oder -fluence nicht unterschreitet. Vorzugsweise wird als Mindestintensität bzw. Mindestfluence näherungsweise der Schwellwert für die Ablation von Material des zu bearbeitenden Körpers verwendet. Besonders bevorzugt, da einfach zu erzeugen, wird ein Strahlprofil mit einer Gaußschen Form verwendet, das an den Flanken abgeschnitten ist, und auch als "Truncated-Gauß"-Profil bezeichnet wird. Die Ablationsvorrichtung weist dazu vorzugsweise eine strahlformende Einrichtung auf, mittels derer das Strahlprofil des Laserstrahls so formbar ist, daß wenigstens nahe der Oberfläche des Körpers das Strahlprofil über den gesamten Querschnitt die vorgegebene Mindestintensität oder Mindestfluence nicht unterschreitet.

Weiterer Gegenstand der Erfindung ist ein Verfahren zur Bildung von Steuersignalen zur Steuerung eines Lasers einer Laserablationsvorrichtung zur Abgabe eines gepulsten Laserstrahls und/oder einer Ablenkeinrichtung der Laserablationsvorrichtung zur Ablenkung des Laserstrahls, um wasserhaltiges Material von einer Oberfläche eines Körpers gemäß einem vorgegebenen Soll-Ablationsprofil mittels Pulsen eines gepulsten Laserstrahls zu ablatieren, bei dem mit dem Erstellungsverfahren ein Ablationsprogramm für das vorgegebene Soll-Ablationsprofil erstellt wird und entsprechend dem Ablationsprogramm Steuersignale an den Laser und/oder die Ablenkeinrichtung abgegeben werden.

Dementsprechend ist Gegenstand der Erfindung auch eine Vorrichtung zur Bildung von Steuersignalen für einen Laser und/oder eine Ablenkeinrichtung einer Laserablationsvorrichtung, um wasserhaltiges Material von einer Oberfläche eines Körpers gemäß einem vorgegebenen Soll-Ablationsprofil mittels Pulsen eines gepulsten Laserstrahls zu ablatieren, wobei die Vorrichtung eine erfindungsgemäße Erstellungsvorrichtung zur Erstellung eines Ablationsprogramms aus dem vorgegebenen Soll-Ablationsprofil und eine Steuereinrichtung zur Abgabe von Steuersignalen gemäß dem erstellten Ablationsprogramm an den Laser und/oder die Ablenkeinrichtung zur Ablenkung des von dem Laser abgegebenen Laserstrahls umfaßt.

Die Steuereinrichtung, mittels derer die Steuersignale gebildet und vorzugsweise abgegeben werden, kann dabei unabhängig von der Datenverarbeitungseinrichtung ausgebildet und mit dieser durch eine Datenverbindung zur Übermittlung des Ablationsprogramms verbunden sein. Vorzugsweise ist sie wenigstens teilweise durch Einrichtungen der Datenverarbeitungseinrichtung realisiert, mittels derer durch das Ablationsprogramm in Verbindung mit entsprechenden Schaltungen der Steuereinrichtung Steuerbefehle an den Laser und/oder die Ablenkeinrichtung abgegeben werden können, wodurch sich in vorteilhafter Weise ein besonders einfacher Aufbau ergibt. Die Ablenkeinrichtung und der Laser sind dabei in Abhängigkeit von dem von der Erstellungsvorrichtung erstellten Ablationsprogramm durch die gebildeten Steuersignale so steuerbar, daß Pulse des gepulsten, von dem Laser abgegebenen Laserstrahls auf entsprechende Zielorte auf der Oberfläche gelenkt werden.

Die Laserablationsvorrichtung kann insbesondere die Steuersignalbildungsvorrichtung umfassen, wodurch sich der Vorteil eines besonders kompakten Aufbaus ergibt. Vorzugsweise ist daher bei der Laserablationsvorrichtung der Laser und/oder die Ablenkeinrichtung mit der Steuereinrichtung über eine entsprechende Steuerverbindung verbunden.

Eine Ablation nach dem eingangs bereits erwähnten "spot scanning"-Verfahren hat den Vorteil, daß einfach weitgehend beliebige Ablationsprofile ablatiert werden können. Das Erstellungsverfahren und die Erstellungsvorrichtung dienen daher vorzugsweise zur Erstellung eines Ablationsprogramms für die Ablation von wasserhaltigem Material von einer Oberfläche eines Körpers gemäß einem vorgegebenen Soll-Ablationsprofil durch die Abgabe von Pulsen eines über die Oberfläche geführten gepulsten Laserstrahls auf die Oberfläche. Das Ablationsprogramm umfaßt dann vorzugsweise eine Folge von Zielorten oder -richtungen, mit denen bzw. auf die bzw. in die Pulse des Laserstrahls abgegeben werden. Werden nacheinander Pulse auf den gleichen Zielort abgegeben, braucht der Zielort nur einmal angegeben zu werden, wenn gleichzeitig die Anzahl der auf den Zielort abzugebenden Pulse angegeben wird.

Im folgenden sind bevorzugte Ausführungsformen und Weiterbildungen der Erfindung geschildert , bei denen die Strahlform und die Neigung berücksichtigt wird.

Prinzipiell kann es bei dem Erstellungsverfahren genügen, den Wassergehalt des Materials als konstant für das gesamte zu ablatierende Volumen anzunehmen. Es ist jedoch bevorzugt, daß bei der Erstellung des Ablationsprogramms der Wassergehalt in Abhängigkeit vom Ort auf der Oberfläche oder in einem zu ablatierenden Bereich berücksichtigt wird. Dieses Vorgehen hat den Vorteil, daß zum einen lokale Unterschiede im Wassergehalt in dem zu ablatierenden Bereich, der durch das Soll-Ablationsprofil definiert ist, oder Volumen und zum anderen durch die Ablation hervorgerufene Unterschiede, beispielsweise zwischen den Rändern des, ablatierenden Bereichs und dessen Zentrum, berücksichtigt werden können, was zu einer höheren Präzision der Ablation führt.

Der Wassergehalt kann vorzugsweise dadurch bei der Erstellung des zur Ablation zu verwendenden Ablationsprogramms berücksichtigt werden, daß ein Modell verwendet wird, das die Abhängigkeit der durch wenigstens einen auf einen Zielort auf der Oberfläche abgegebenen Puls erzielten Ablationstiefe oder des durch wenigstens einen auf einen Zielort auf der Oberfläche abgegebenen Puls erzielten Ablationsvolumens von dem Wassergehalt des mit dem Puls zu ablatierenden Materials wiedergibt. Das Modell kann dabei insbesondere durch eine Funktion gegeben sein, die von einer von dem Wassergehalt abhängenden oder diesen wiedergebenden Größe abhängt und die Ablationstiefe für einen Puls des gepulsten Laserstrahls mit vorgegebenen Pulseigenschaften, z.B. gegebener Pulsenergie oder Fluence, wenigstens näherungsweise wiedergibt. Solche Modelle können aus theoretischen Überlegungen oder aus experimentellen Untersuchungen der Ablationstiefe oder des Ablationsvolumens bei Ablation mit einem Einzelpuls in Abhängigkeit von wenigstens dem Wassergehalt und nachfolgende Darstellung der Ergebnisse durch geeignete Funktionen gewonnen werden. Das Modell kann insbesondere in einem Verfahrensschritt oder in einem Computerprogramm, mittels dessen das Erstellungsverfahren durchgeführt wird, in der Weise berücksichtig werden, daß aus dem Modell hergeleitete Formeln mit Parametern des Modells bei Programmausführung ausgewertet werden.

Vorzugsweise enthält das Modell als Parameter eine Ablationsrate, die von dem Wassergehalt abhängig ist. Hierdurch kann in vorteilhafter Weise berücksichtigt werden, daß der Wassergehalt die Ablationstiefe beeinflußt.

Typischerweise führt nicht jeder Laserpuls zu einer Ablation. Vorzugsweise wird daher bei dem Verfahren ein Fluence-Schwellwert für die Ablation von Material von der Oberfläche des Körpers verwendet und der Schwellwert in Abhängigkeit von dem Wassergehalt ermittelt. Dieses Vorgehen hat den Vorteil, daß auf einfache und kontrollierte Weise eine Wirkung des in dem Material enthaltenen Wassers darauf, ob überhaupt eine Ablation eintritt, berücksichtigt werden kann.

Angaben zu dem Wassergehalt in dem Material können auf unterschiedlich Art und Weise in das Verfahren einfließen. Dabei brauchen grundsätzlich nur entsprechende Daten verwendet zu werden. So kann ein fest vorgegebenes weiteres Modell für den Wassergehalt für einen vorgegebenen Materialtyp verwendet werden, wozu die Datenverarbeitungseinrichtung der Erstellungsvorrichtung ein entsprechendes in einem Speicher in der Erstellungsvorrichtung gespeichertes Programmmodul aufweisen kann, in dem das weitere Modell einschließlich entsprechender Parameterwerte fest codiert ist. Alternativ kann ein Parameterwert auch in einer Parameterdatei fest gespeichert sein.

Bereits auf einen Bereich des Körpers abgegebene Pulse können bereits einen Einfluß auf den Wassergehalt des Bereichs oder benachbarter Bereiche haben. Es ist daher bevorzugt, daß bei dem Erstellungsverfahren ein weiteres Modell verwendet wird, das für einen vorgegebenen Bereich des Materials den Einfluß von auf diesen Bereich oder benachbarte Bereiche aufgetroffenen Pulsen des gepulsten Laserstrahls auf den Wassergehalt wiedergibt. Das Modell braucht dabei den Einfluß nur näherungsweise wiederzugeben. Insbesondere kann das Modell den Einfluß dadurch wiedergeben, daß es von der Soll-Ablationstiefe in dem Bereich abhängt, die in guter Näherung der Anzahl der auf den Bereich abzugebenden Pulse, gegebenenfalls in nichtlinearer Weise, entspricht. Unter Bereich kann dabei insbesondere eine im wesentlichen orthogonal zu der Oberfläche verlaufende Säule von Material verstanden werden, die sukzessive durch auftreffende Pulse ablatiert wird, wobei sich der Wassergehalt in dem verbleibenden Teil der Säule durch die Ablation verändert. Dieses Vorgehen hat den Vorteil, daß während der Ablation eine Messung des Wassergehalts nicht unbedingt notwendig ist. Das Ablationsprogramm berücksichtigt den Effekt bereits bei seiner Erstellung. Vorzugsweise sind das Modell für die Ablationstiefe oder das Ablationsvolumen und das weitere Modell so gewählt, daß mit zunehmender Soll-Ablationstiefe bzw. zunehmender Anzahl auf denselben Bereich abzugebender Pulse, die Ablationswirkung eines Pulses zunimmt, d.h. die durch einen Puls erzeugte Ablationstiefe bzw. das durch den Puls erzeugte Ablationsvolumen zunimmt. Hierdurch können insbesondere die Probleme bei der Korrektur von Kurz- oder Weitsichtigkeit mit konventionellen Ablationsverfahren reduziert werden, bei denen in Bereichen geringer Soll-Ablationstiefe die tatsächlich erreichte Ablation zu gering verglichen mit der Soll-Ablationstiefe ausfällt.

Wenn jedoch Variationen im Wassergehalt des ansonsten im wesentlichen gleichen Materials bei verschiedenen Körpern auftreten können und/oder die Möglichkeit bestehen soll, Ablationsprogramme für die Ablation verschiedener Materialien erstellen zu können, werden bei dem Erstellungsverfahren vorzugsweise Daten erfaßt, die den Wassergehalt, insbesondere auf der Oberfläche oder in dem zu ablatierenden Bereich ortsaufgelöst, wiedergeben oder aus denen der Wassergehalt, insbesondere auf der Oberfläche oder in dem zu ablatierenden Bereich ortsaufgelöst, ermittelbar ist. Es ist dann bevorzugt, daß die Erstellungsvorrichtung eine Schnittstelle zum Erfassen von Daten umfaßt, die den Wassergehalt des Materials wiedergeben oder aus denen der Wassergehalt ermittelbar ist. Vorzugsweise ist die Datenverarbeitungseinrichtung zur Ermittlung des Wassergehalts aus den Daten, aus den der Wassergehalt des Materials ermittelbar ist, ausgebildet. Wird der Wassergehalt als im zu ablatierenden Bereich als konstant angenommen, kann es genügen nur einen entsprechenden Wert über die Schnittstelle einzulesen. Wird der Wassergehalt ortsaufgelöst berücksichtigt, können entsprechende Daten in Form eines Feldes von den Wassergehalt wiedergebenden Werten eines Parameters an vorgegebenen Stützstellen in dem zu ablatierenden Volumen oder auf einer Referenzfläche zur Beschreibung des Volumens gegeben sein. Alternativ ist möglich, den Wassergehalt durch eine parametrisierte, ortsabhängige Funktion wiederzugeben, indem die Werte der Parameter der Funktion vorgegeben werden.

Die Angaben zum Wassergehalt können auf unterschiedliche Art und Weise ermittelt werden. So ist es bei einer Ausführungsform bevorzugt, daß der Wassergehalt aus empirischen Untersuchungen verschiedener Proben des Materials ermittelt wird. Insbesondere können im Fall von biologischem Material Reihenuntersuchungen an Material bzw. Gewebe des gleichen Typs, aber von verschiedenen Individuen einer Spezies durchgeführt werden, bei denen ein mittlerer Wassergehalt ermittelt wird, der dann in die Erstellung des Ablationsprogramms einfließt. Insbesondere kann in diesem Fall angenommen werden, daß der Wassergehalt in dem gesamten zu behandelnden Gebiet näherungsweise konstant ist. Dieses Vorgehen hat den Vorteil, daß es keine individuellen Daten über jeweils zu behandelnden Körper voraussetzt und daher einfach und schnell anwendbar ist. Insbesondere können entsprechende Werte fest in der Datenverarbeitungseinrichtung gespeichert sein, wobei jedoch auch eine Eingabe über eine zuvor erwähnte Schnittstelle möglich ist.

Im Verlaufe der Ablation kann sich der Wassergehalt des Materials unter Umständen durch die Ablation ändern. Es ist daher bei dem Erstellungsverfahren bevorzugt, daß zur Berücksichtigung des Wassergehalts bei der Erstellung des Ablationsprogramms ein Modell für den Wassergehalt oder die Änderung des Wassergehalts des Materials in Abhängigkeit von wenigstens der Anzahl und/oder Lage vorher auf den gleichen Ort und/oder benachbarte Orte abgegebener Pulse verwendet wird. In vorteilhafter Weise kann so eine Berücksichtigung der Änderung des Wassergehalts durch die Ablation erfolgen, ohne daß eine Messung des Wassergehalts während der Ablation notwendig ist. Werte gegebenenfalls in dem Modell auftretender Parameter können wie zuvor beispielsweise empirisch bestimmt werden.

Um eine hohe Genauigkeit der Ablation, d.h. eine geringe Abweichung zwischen Soll-Ablationsprofil und durch die Ablation gemäß dem erstellten und zu verwendenden Ablationsprogramm erzieltem Ist-Ablationsprofil zu erhalten, ist es jedoch bevorzugt, daß bei dem Erstellungsverfahren der Wassergehalt aus an dem Körper gemessenen Daten ermittelt wird. Dies hat den Vorteil, daß keine Annahmen über den Wassergehalt verwendet zu werden brauchen. Der Wassergehalt braucht dabei nicht explizit berechnet zu werden, vielmehr kann es genügen, daß eine diesen eindeutig bestimmende Größe ermittelt wird, die dann in eine Formel eingesetzt wird, in der die Abhängigkeit von dem Wassergehalt selbst durch die Abhängigkeit von der Größe ersetzt ist. Die Datenverarbeitungseinrichtung der Erstellungsvorrichtung ist vorzugsweise dazu ausgebildet, den Wassergehalt aus an dem Körper gemessenen Daten zu ermitteln. Dazu kann ein in der Datenverarbeitungseinrichtung ablaufendes Computerprogramm entsprechenden Programmcode enthalten. Die Messung der Daten kann dabei vor der Erstellung des Ablationsprogramms und insbesondere der Ablation oder während der Erstellung des Ablationsprogramms und der Bildung der Steuersignale erfolgen.

Die Daten können insbesondere, beispielsweise über eine geeignete oben erwähnte Schnittstelle, von einem Datenträger, einer Benutzerschnittstelle oder einer Meßeinrichtung eingelesen werden. Vorzugsweise werden die Daten an dem Körper automatisch gemessen, wozu die Erstellungsvorrichtung eine Steuerschnittstelle für eine Meßeinrichtung zur Messung von Daten an dem Körper, die den Wassergehalt wiedergeben oder aus denen der Wassergehalt ermittelt werden kann, aufweisen kann, über die Steuerbefehle zur Erfassung von Meßdaten an die Meßeinrichtung abgebbar sind. Auf diese Weise kann die gesamte Bearbeitungszeit wesentlich reduziert werden.

Vorzugsweise werden als an dem Körper gemessene Daten Daten verwendet, die eine ortsaufgelöste Ermittlung des Wassergehalts in dem Material an der Oberfläche oder in einem zu ablatierenden Bereich ermöglichen. Werden die Daten automatisch gemessen, erfolgt die Messung dementsprechend vorzugsweise ortsaufgelöst in dem Material an der Oberfläche oder in einem zu ablatierenden Bereich. Auf diese Weise kann in vorteilhafter Weise der Wassergehalt ortsabhängig berücksichtigt werden, was zu einer erhöhten Genauigkeit der Ablation führt.

Die Daten können vor der eigentlichen Ablation erfaßt werden. Bei einer besonders bevorzugten Ausführungsform der Erfindung werden entsprechende Daten jedoch auch während der Bildung von Steuersignalen gemäß einem ersten Teil des Ablationsprogramms bzw. während der Ablation erfaßt, so daß eine sehr genaue Steuerung der Ablation möglich ist.

Der Wassergehalt kann auf unterschiedliche Art und Weise ermittelt werden. Insbesondere kann eine der im folgenden genannten Möglichkeiten oder eine Kombination dieser Möglichkeiten verwendet werden. Bei einer ersten Alternative des Erstellungsverfahrens werden zur Ermittlung des Wassergehalts Daten verwendet, die die Temperatur der Oberfläche wiedergeben. Die Erstellungsvorrichtung kann hierzu vorzugsweise eine Einrichtung zur Erfassung einer Temperatur der Oberfläche aufweisen, die zur Übermittlung von erfaßten Temperaturdaten an die Datenverarbeitungseinrichtung über eine Datenverbindung mit der Datenverarbeitungseinrichtung verbunden ist, und bei der die Datenverarbeitungseinrichtung zur Ermittlung des Wassergehalts des Materials in Abhängigkeit von den Temperaturdaten ausgebildet ist. Diese Ausführungsform hat den Vorteil, daß eine Temperaturmessung einfach auch ortsaufgelöst berührungslos durchführbar ist, wenn beispielsweise entsprechende Infrarotkameras als Meßeinrichtung verwendet werden.

Als weitere Möglichkeit ist es bevorzugt, daß zur Ermittlung des Wassergehalts Daten verwendet werden, die Eigenschaften von optischer Strahlung wiedergeben, die von dem Material im zu ablatierenden Bereich des Körpers ausgeht. Vorzugsweise werden die entsprechenden Daten automatisch erfaßt. Die Erstellungsvorrichtung kann hierzu vorzugsweise ein mit der Datenverarbeitungseinrichtung über eine Datenverbindung verbundenes Spektrometer zur Analyse von von dem zu ablatierenden Bereich des Körpers ausgehender Strahlung aufweisen. Die Datenverarbeitungseinrichtung kann dann zur Ermittlung des Wassergehalts in Abhängigkeit von von dem Spektrometer erfaßten Daten ausgebildet sein. Hierzu kann ein in der Datenverarbeitungseinrichtung gespeichertes Computerprogramm Programmcode zur Ermittlung des Wassergehalts in Abhängigkeit von von dem Spektrometer erfaßten Daten enthalten. Diese Alternative hat den Vorteil, daß zum einen eine berührungslose Erfassung der Daten möglich ist und zum anderen ein genaue Erfassung des Wassergehalts möglich ist. Darüber hinaus ist es auch möglich, die Strahlung ortsaufgelöst zu erfassen und damit eine ortsabhängige Bestimmung des Wassergehalts zu ermöglichen. Bei einer Laserablationsvorrichtung mit einem Laser zur Abgabe des Laserstrahls und einer Erstellungsvorrichtung ist die Blick- bzw. Detektionsrichtung des Spektrometers vorzugsweise gegenüber dem Laserstrahl an der Oberfläche des Körpers geneigt. Dies ermöglicht eine einfache Integration des Spektrometers in die Laserablationsvorrichtung.

Besonders bevorzugt werden zur Ermittlung des Wassergehalts Daten verwendet, die durch konfokale Raman-Spektroskopie von optischer Strahlung von der Oberfläche erhältlich sind. Die Erstellungsvorrichtung weist dazu vorzugsweise eine Einrichtung zur Durchführung von konfokaler Raman-Spektroskopie auf, die zur Übermittlung von erfaßten Spektroskopiedaten an die Datenverarbeitungseinrichtung über eine Datenverbindung mit der Datenverarbeitungseinrichtung verbunden ist, wobei die Datenverarbeitungseinrichtung vorzugsweise zur Ermittlung des Wassergehalts des Materials in Abhängigkeit von den Spektroskopiedaten ausgebildet ist. Dieses Verfahren erlaubt eine besonders gute Spezifität der Messung für Wasser.

Als weitere Möglichkeit können zur Ermittlung des Wassergehalts Daten verwendet werden, die Eigenschaften von Fluoreszenzstrahlung wiedergeben, die von dem zu ablatierenden Bereich des Körpers ausgeht. Die Erstellungsvorrichtung kann hierzu vorzugsweise eine zur Übermittlung von detektierten Fluoreszenzstrahlungsdaten über eine Datenverbindung mit der Datenverarbeitungseinrichtung verbundene Einrichtung zur Detektion von Fluoreszenzstrahlung aufweisen, die bei Bestrahlung der zu ablatierenden Oberfläche des Körpers von Material an der Oberfläche des Körpers abgegeben wird, und bei der die Datenverarbeitungseinrichtung vorzugsweise zur Ermittlung des Wassergehalts des Materials in Abhängigkeit von den Fluoreszenzstrahlungsdaten ausgebildet ist. Die Fluoreszenzstrahlung kann dabei entweder durch Bestrahlen des zu untersuchenden Bereichs des Körpers mit einer nicht zur Ablation verwendeten Strahlungsquelle, oder soweit möglich, durch Bestrahlung mit Laserstrahlung des zur Ablation verwendeten Lasers angeregt werden. Diese Alternative zeichnet sich dadurch aus, daß die instrumentellen Anforderungen zur Durchführung der Messung relativ gering gehalten werden können.

Noch eine weitere Möglichkeit besteht darin, zur Ermittlung des Wassergehalts Daten zu verwenden, die den Brechungsindex in dem Material wiedergeben. Vorzugsweise kann die Erstellungsvorrichtung zur Ermittlung des Brechungsindex des Materials einen optischen Kohärenztomographen aufweisen, der zur Übermittlung von Meßdaten über eine Datenverbindung mit der Datenverarbeitungseinrichtung verbunden ist. Hierbei wird ausgenutzt, daß der Brechungsindex des Materials von dem Wassergehalt abhängen kann. Ein Vorteil dieser Variante kann darin liegen, daß der optische Kohärenztomograph auch für Topographiemessungen an der Oberfläche des Körpers verwendet werden kann. Der Kohärenztomograph kann weiter dazu verwendet werden, die Dicke der Cornea vor und/oder während der Ablation zu ermitteln, d.h. eine pachymetrische Messung durchzuführen. Damit kann insbesondere vermieden werden, daß bei der Ablation die Restdicke der Cornea einen vorgegebenen Mindestwert unterschreitet. Die Berücksichtigung des Wassergehalts kann auf unterschiedliche Art und Weise erfolgen. So können beispielsweise bekannte Erstellungsverfahren dahingehend modifiziert werden, daß in diesen Modelle für die Ablation verwendet werden, die den Wassergehalt berücksichtigen. Dies kann jedoch einen Neuentwurf dieser Verfahren erfordern.

Bei einer weiteren Möglichkeit ist es daher zur Erstellung des zu verwendenden Ablationsprogramms bevorzugt, daß zur Berücksichtigung des Wassergehalts aus dem vorgegebenen Soll-Ablationsprofil ein in Abhängigkeit von dem Wassergehalt vorkompensiertes Ablationsprofil ermittelt wird und aus dem vorkompensierten Ablationsprofil das Ablationsprogramm erstellt wird. Das Soll-Ablationsprofil wird dabei vorzugsweise so modifiziert, daß die Ablation gemäß dem auf der Basis des modifizierten Soll-Ablationsprofil erstellten Ablationsprogramm wenigstens näherungsweise zu dem gewünschten Soll-Ablationsprofil führt. Insbesondere kann zur Erstellung des Ablationsprogramms aus dem modifizierten bzw. vorkompensierten Soll-Ablationsprofil ein einfaches Erstellungsverfahren verwendet werden. Unter einem einfachen Erstellungsverfahren wird im Rahmen des zweiten Aspekts der vorliegenden Erfindung ein Erstellungsverfahren verstanden, das bei der Erstellung des Ablationsprogramms den Wassergehalt des Materials nicht berücksichtigt. Die Fehler, die bei Verwendung eines einfachen Erstellungsverfahrens zur Erstellung eines Ablationsprogramms durch die Nichtberücksichtigung des Wassergehalts auftreten, können durch eine entsprechende Modifikation des Soll-Ablationsprofils, das heißt der Ausgangsdaten zur Erstellung des Ablationsprogramms, so kompensiert werden, daß in der Folge ein einfaches Erstellungsverfahren verwendet werden kann. Dabei können die Art und der Umfang der Vorkompensation bzw. Modifikation von den Eigenschaften des verwendeten einfachen Erstellungsverfahrens bzw. von den diesem zugrundeliegenden Modellannahmen abhängen. Die Modifikation verändert also im Wege einer Vorkorrektur oder Vorkompensation das Soll-Ablationsprofil in der Art, daß Abweichungen zwischen der Soll-Ablationstiefe und der Ist-Ablationstiefe, die durch Nichtberücksichtigung des Wassergehalts des Materials bei der Erstellung eines Ablationsprogramms mit einem einfachen vorgegebenen Erstellungsverfahren, bei dem der Wassergehalt nicht berücksichtigt wird, entstehen können, durch Änderung des vorgegebenen Soll-Ablationsprofils in entgegengesetzter Richtung bzw. Größe vorkompensiert werden. Diese Ausführungsform hat den Vorteil, daß zur Erstellung des zu verwendenden Ablationsprogramms einfache Erstellungsverfahren verwendet werden können, so daß bereits erprobte Verfahren verwendet werden können. Beispiele für solche einfachen Erstellungsverfahren sind in DE 197 27 573 C1 oder EP 1060710 A2 beschrieben, deren Inhalt hiermit insoweit durch Bezugnahme in die Beschreibung aufgenommen wird.

Dazu ist es bevorzugt, daß zur Ermittlung des vorkompensierten Ablationsprofils eine Modifikationsfunktion verwendet wird, die explizit oder implizit von dem Wassergehalt des zu ablatierenden Materials abhängt. Insbesondere kann für wenigstens zwei zu ablatierende Bereiche der Oberfläche jeweils ein Wert einer Modifikationsfunktion in Abhängigkeit von wenigstens dem Wassergehalt des Materials an der Oberfläche in dem jeweiligen Bereich ermittelt und das modifizierte bzw. vorkompensierte Soll-Ablationsprofil unter Verwendung des Soll-Ablationsprofils und der Werte der Modifikationsfunktion ermittelt werden. Die Erstellung des Ablationsprogramms kann dabei insbesondere durch ein einfaches Erstellungsverfahren erfolgen. Die Werte der Modifikationsfunktion werden vorzugsweise für jeden Puls bzw. für jeden Ort auf der Oberfläche, auf den ein Puls gelenkt werden soll, berechnet. Die Werte können für jeden Bereich bzw. Zielort jeweils neu berechnet werden. Es ist jedoch auch möglich, die Werte nur einmal zu berechnen und dann in einer Tabelle, beispielsweise in Abhängigkeit von dem Wassergehalt oder einer von diesem abhängigen Größe, abzulegen, aus der sie dann jeweils bei Bedarf ausgelesen werden können. Dieses Vorgehen erlaubt eine besonders einfache Ermittlung des vorkompensierten Soll-Ablationsprofils. Die Modifikationsfunktion kann dabei explizit von dem Wassergehalt des Materials an dem Zielort oder implizit über wenigstens einen anderen Parameter der Modifikationsfunktion abhängen.

Die Modifikationsfunktion kann insbesondere unter Verwendung des zuvor erwähnten Modells für die durch einen Einzelpuls in Abhängigkeit von dem Wassergehalt erzielte Ablationstiefe bzw. das durch einen Einzelpuls in Abhängigkeit von dem Wassergehalt erzielte Ablationsvolumen und gegebenenfalls das weitere Modell für die Änderung des Wassergehalts durch Ablation bestimmt bzw. abgeleitet sein und dieses insofern enthalten.

Vorzugsweise hängt die Modifikationsfunktion von der Intensität, der Energie oder der Fluence der bei der Ablation zu verwendenden Pulse ab. Dabei kann die Modifikationsfunktion auch von einem Schwellwert für die Energie oder wirksame Fluence abhängen, der angibt, ab welcher Energie bzw. wirksamen Fluence eine Ablation des Materials auftritt und der vorzugsweise von dem Wassergehalt in dem Material abhängig ist. Insbesondere kann die Modifikationsfunktion von dem dimensionslosen Verhältnis der Fluence und des Fluence-Schwellwerts abhängen.

Während der Ablation kann sich der Wassergehalt des Materials durch die bereits auf den Körper aufgetroffenen Pulse verändern. Da auf einen Zielort unter Umständen zur Erzielung einer gewünschten Ablationstiefe mehrere Pulse abgegeben werden müssen, ist es bei dem Erstellungsverfahren bevorzugt, daß der Wert der Modifikationsfunktion an einem jeweils vorgegebenen Ort von einer durch das Soll-Ablationsprofil gegebenen Soll-Ablationstiefe an dem Ort abhängt. Dies hat den Vorteil, daß auf einfache Weise die Abhängigkeit vom Wassergehalt des Materials auch für große Ablationstiefen einfach vor der eigentlichen Ablation berücksichtigt werden kann. Die Modifikationsfunktion kann dabei noch von wenigstens einem weiteren Parameter abhängen, der insbesondere selbst von dem in dem Material vorhandenen Wassergehalt oder der Änderung des Wassergehalts durch Auftreffen eines Pulses abhängt.

Weiterhin kann es sich als günstig erweisen, daß aus dem Soll-Ablationsprofil ein vorläufiges Ablationsprogramm erstellt wird und zur Bildung des zu erstellenden Ablationsprogramms in Abhängigkeit von dem Wassergehalt wenigstens ein durch das vorläufige Ablationsprogramm implizit oder explizit vorgegebener Fluence-Wert oder eine durch das vorläufige Ablationsprogramm implizit oder explizit vorgegebene Pulsenergie eines auf einen durch das vorläufige Ablationsprogramm gegebenen Zielort abzugebenden Pulses in Abhängigkeit von dem Wassergehalt an dem Zielort geändert und als Angabe dem Zielort zugeordnet wird. Das vorläufige Ablationsprogramm kann dabei mit beliebigen Erstellungsverfahren erstellt sein, insbesondere kann ein einfaches Erstellungsverfahren benutzt werden, das aus dem vorgegebenen Soll-Ablationsprofil ohne die Berücksichtigung des Wassergehalts des Materials das vorläufige Ablationsprogramm erstellt. Es ist jedoch auch möglich, ein vorläufiges Ablationsprogramm beispielsweise unter einer nur näherungsweisen oder vereinfachten Berücksichtigung des Wassergehalts zu erstellen und dieses dann durch Änderung der Pulsenergie bzw. des Fluencewertes nachzukorrigieren. Bei der Ermittlung der Änderung des Fluencewertes oder der Pulsenergie kann daher insbesondere berücksichtigt werden, welche Annahmen oder Modelle das zur Erstellung des vorläufigen Ablationsprogramms verwendet wurden.

Die Änderung des Fluence-Wertes kann insbesondere unter Verwendung des zuvor erwähnten Modells für die durch einen Einzelpuls in Abhängigkeit von dem Wassergehalt erzielte Ablationstiefe bzw. das durch einen Einzelpuls in Abhängigkeit von dem Wassergehalt erzielte Ablationsvolumen bestimmt bzw. abgeleitet sein und dieses insofern enthalten.

Die Einstellung der Energie oder Fluence eines Pulses kann dabei auf wenigstens drei Weisen erfolgen, die alternativ oder in Kombination angewendet werden können. Bei einer ersten Variante wird zur Einstellung der Energie oder Fluence eines Pulses der Laserstrahl abgeschwächt. Hierzu wird vorzugsweise ein Steuersignal gebildet, mittels dessen ein einen zur Ablation verwendeten Laserstrahl schwächender Modulator in seiner Transmission steuerbar ist. Die Steuereinrichtung ist dazu vorzugsweise zur Bildung und Abgabe von Steuersignalen zur Ansteuerung eines den Laserstrahls schwächenden Modulators gemäß einem erstellten Ablationsprogramm ausgebildet. Die Laserablationsvorrichtung weist vorzugsweise neben der Steuereinrichtung einen mit der Steuereinrichtung verbundenen Modulator auf, der den Laserstrahl auf Steuersignale der Steuereinrichtung abschwächt. Als Modulator kann dabei jegliche Einrichtung dienen, mit der die Intensität des von dem Laser abgegebenen Laserstrahls reduziert werden kann. Beispielsweise können Pockels-Zellen, gesteuert durch einen Antrieb drehbare Glasscheiben, mit radialen Sektoren unterschiedlichen Transmissionsvermögens oder Flüssigkristallelemente mit steuerbarem Transmissionsvermögen verwendet werden. Der Modulator kann dabei beispielsweise polarisationsabhängig oder auch wellenlängenabhängig wirken. Die Verwendung von Modulatoren erlaubt eine einfache Einstellung der Pulsenergie bei beliebigen Lasertypen.

Bei der zweiten Variante kann zur Einstellung der Energie oder Fluence eines Pulses der zur Abgabe des gepulsten Laserstrahls verwendete Laser gesteuert werden. Hierzu können Steuersignale zur Ansteuerung des Lasers der Laserablationsvorrichtung gebildet werden, mittels derer die Fluence oder Pulsenergie von dem Laser abgegebener Puls gesteuert wird. Die Steuereinrichtung kann entsprechend zur Bildung und Abgabe von Steuersignalen zur Ansteuerung des Lasers gemäß einem erstellten Ablationsprogramm ausgebildet sein. Bei der Laserablationsvorrichtung ist dann vorzugsweise der Laser mittels der Steuereinrichtung steuerbar. Bei Verwendung von Excimerlasern kann die Steuereinrichtung insbesondere die Hochspannung zur Aufladung eines Kondensators bzw. mehrerer Kondensatoren zur Speicherung von Energie zur Erzeugung eines Laserpulses steuern. Die Steuerung der Pulsenergie oder Fluence des Lasers hat den Vorteil, daß weniger Energie benötigt wird.

Als dritte Variante kann zur Einstellung der Fluence eines Pulses der Strahlquerschnitt des Laserstrahls an der Oberfläche geändert werden. Hierzu können Steuersignale zur Steuerung einer strahlformenden optischen Einrichtung im Strahlengang des Laserstrahls, die zur Einstellung des Strahlquerschnitts dient, gemäß einem erstellten Ablationsprogramm gebildet und an die strahlformende optische Einrichtung abgegeben werden. Die Steuereinrichtung kann entsprechend zur Bildung und Abgabe von Steuersignalen zur Ansteuerung einer strahlformenden optischen Einrichtung im Strahlengang des Laserstrahls, die zur Einstellung des Strahlquerschnitt dient, gemäß einem erstellten Ablationsprogramm ausgebildet sein. Eine Laserablationsvorrichtung kann hierzu eine entsprechende im Strahlengang des Laserstrahls angeordnete strahlformende optische Einrichtung besitzen, die von der Steuereinrichtung zur Änderung des Strahlquerschnitts angesteuert wird.

Ein Ablationsprogramm, insbesondere ein Ablationsprogramm, bei dem die Fluence oder Energie der verwendeten Pulse geändert wird, kann vor der eigentlichen Ablation erstellt werden. Es ist jedoch bei dem Steuersignalbildungsverfahrens bevorzugt, daß nach der Erstellung eines ersten Teils des Ablationsprogramms und Abgabe entsprechender Steuersignale wenigstens ein weiterer noch abzuarbeitender Teil des Ablationsprogramms erstellt und entsprechende Steuersignale abgegeben werden. Dies ermöglicht es in vorteilhafter Weise, schon mit der Ablation zu beginnen, während das vollständige Programm noch erstellt wird, so daß die Zeit zwischen Bereitstellung des Soll-Ablationsprofils und Ende der Ablation verkürzt wird.

Darüber hinaus ermöglicht es eine besonders bevorzugte Ausführungsform des Steuersignalbildungs- und abgabeverfahrens, bei der ausgehend von dem Soll-Ablationsprofil ein vorläufiges Ablationsprogramm erstellt wird und zur Erstellung des wenigstens einen weiteren Teils des Ablationsprogramms für wenigstens einen durch das vorläufige Ablationsprogramm gegebenen Zielort auf der Oberfläche der Wassergehalt ermittelt wird, und das vorläufige Ablationsprogramm unter Bildung des Ablationsprogramms in Abhängigkeit von dem ermittelten Wassergehalt verändert wird. Dies hat den Vorteil, daß einer Änderung des Wassergehalts während der Ablation, die beispielsweise durch Umgebungseinflüsse oder durch vorangegangene Ablation an demselben oder einem benachbarten Ort verursacht sein kann, noch während der Ablation Rechnung getragen werden kann. Dabei kann insbesondere das vorläufige Ablationsprogramm bereits unter Berücksichtigung eines beispielsweise als statistischen Mittelwert entsprechenden Wassergehalts erstellt sein, wobei individuelle Variationen des Wassergehalts während der Ablation berücksichtigt werden. Vorzugsweise wird das vorläufige Ablationsprogramm durch Änderung der Fluence oder Pulsenergie abzugebender Pulse wenigstens in expliziter oder impliziert Abhängigkeit vom Wassergehalt in das abzuarbeitende bzw. zu verwendende Ablationsprogramm überführt.

Insbesondere bei der zuletzt geschilderten Ausführungsform ist es bevorzugt, daß während der Abgabe von Steuersignalen durch Messungen an dem Körper Daten erfaßt werden, die den Wassergehalt des Materials wiedergeben oder aus denen der Wassergehalt des Materials ermittelbar ist. Hierzu kann die Erstellungsvorrichtung über eine Steuerschnittstelle die Einrichtung zur Messung der Daten automatisch ansteuern.

Bei der Erstellung des Ablationsprogramms können noch weitere Einflüsse berücksichtigt werden, die insbesondere bei bekannten Erstellungsverfahren, wie sie in DE 197 27 573 C1 oder EP 1060710 A2 beschrieben sind, nicht berücksichtigt sind. So ist es bevorzugt, daß das Ablationsprogramm auch in Abhängigkeit von der Luftfeuchte der Luft an der Oberfläche und/oder der Dicke eines Flüssigkeitsfilms auf der Oberfläche erstellt wird. Diese Weiterbildung hat den Vorteil, daß auch Einflüsse berücksichtigt werden, die indirekt den Wassergehalt in dem Material beeinflussen. Darüber hinaus kann die Dicke des Flüssigkeitsfilms auf der Oberfläche die Effektivität der Ablation beeinflussen, wenn der Flüssigkeitsfilm vor der Ablation von Material erst durch die Pulse verdampft werden muß. Sowohl die Luftfeuchte als auch die Dicke des Flüssigkeitsfilms können vor oder vorzugsweise während der Ablation gemessen und bei der Erstellung des Ablationsprogramms berücksichtigt werden. Die Erstellungsvorrichtung verfügt hierzu vorzugsweise über eine Schnittstelle zum Einlesen von Daten, die die Luftfeuchte bzw. die Dicke des Flüssigkeitsfilms wiedergeben, und besonders bevorzugt eine Einrichtung zur Messung der die Luftfeuchte bzw. die Dicke des Flüssigkeitsfilms wiedergebenden Daten. Hierdurch wird in vorteilhafter Weise ein besonders kompakter Aufbau erzielt und eine automatische Messung ermöglicht.

Weiter ist es bevorzugt, daß das Ablationsprogramm auch in Abhängigkeit von einer Form eines Strahlprofils des Laserstrahls und/oder einer Neigung der Oberfläche erstellt wird. Die Neigung der Oberfläche ist dabei vorzugsweise wenigstens näherungsweise relativ zu dem Laserstrahl gegeben. Wird der Laserstrahl nur um kleine Winkel, beispielsweise kleiner als 5°, geschwenkt, kann beispielsweise eine mittlere Lage des Laserstrahls als Bezugsrichtung gewählt werden, relativ zu der die Neigung ermittelt wird. Diese Weiterbildung beruht unter anderem auf dem Gedanken, daß die tatsächlich für die Ablation wirksame Energie pro Fläche, das heißt die tatsächlich wirksame Fluence, eines auf die Oberfläche des Körpers auftreffenden Pulses des gepulsten Laserstrahls nicht allein von der Neigung der Oberfläche relativ zu der Richtung des Laserstrahls an der Oberfläche abhängt, sondern auch von dem Strahlprofil des Laserstrahls, insbesondere an der Oberfläche des Körpers. Unter dem Strahlprofil wird dabei der Verlauf der Intensität bzw. der auf die Fläche bezogenen Energie oder Fluence des Pulses über den Strahlquerschnitt nahe der Oberfläche verstanden. Die Form des Strahlprofils beinhaltet dabei keine absoluten Werte der Intensität oder der Energie bzw. der auf eine zum Laserstrahl senkrechte Querschnittsfläche bezogenen Fluence, sondern nur den Verlauf dieser Größen. Insbesondere eignet sich das Verfahren daher für die Ablation mit Laserstrahlen mit einem nicht-konstanten Strahlprofil. Diese Verfahrensvariante hat den Vorteil, daß auch stark gewölbte Oberflächen mittels eines Laserstrahls, der insbesondere kein konstantes Strahlprofil aufzuweisen braucht, mit hoher Genauigkeit ablatiert werden können. Die zur Erstellung des Ablationsprogramms notwendigen Oberflächendaten, aus denen die Neigung ermittelbar ist, können unmittelbar die Neigung der Oberfläche in Abhängigkeit von dem Ort, die Gradienten der Höhe der Oberfläche über einer vorgegebenen Bezugsebene, in der die Orte angegeben sind, oder auch die Höhen der Oberfläche über der Bezugsebene als Funktion des Ortes angeben, wobei insbesondere auch die in Bezug auf das Soll-Ablationsprofil erwähnten Darstellungsmöglichkeiten verwendet werden können. Falls diese Daten die Neigung, beispielsweise gegeben durch entsprechende Winkel, nicht explizit enthalten, kann diese Information durch Gradientenbildung und/oder die Verwendung trigonometrischer Beziehungen einfach ermittelt werden.

Je nach Anwendung können in der Datenverarbeitungseinrichtung das Soll-Ablationsprofil und/oder die Daten in Bezug auf die Oberflächenneigung gespeichert sein, was sich insbesondere anbietet, wenn mehrere gleiche Körper nacheinander ablatiert werden sollen. Können sich auch die Oberflächendaten in Bezug auf die Neigung der Oberfläche ändern, kann die erfindungsgemäße Erstellungsvorrichtung weiterhin über eine Schnittstelle zur Erfassung von Oberflächendaten verfügen, die die Neigung der Oberfläche direkt oder indirekt wiedergeben. Die Schnittstelle kann wie die Schnittstelle zur Erfassung des Soll-Ablationsprofils ausgebildet sein. Vorzugsweise werden die Oberflächendaten automatisch erfaßt, wozu die Datenverarbeitungseinrichtung eine entsprechende Steuerschnittstelle zur Ausgabe von Steuerbefehlen an eine Einrichtung zur Erfassung von Oberflächendaten aufweisen kann.

Die Erstellungsvorrichtung kann insbesondere Einrichtung zur Erfassung von Oberflächendaten ein Topographiemeßsystem zur Erfassung der Topographie der Oberfläche und/oder ein Aberrometer zur Erfassung von optischen Eigenschaften des Körpers umfassen. Diese Ausbildung hat den Vorzug, daß eine einfache und genaue Erfassung der Oberflächentopographie der zu ablatierenden Oberfläche ohne eine zusätzliche Ausrichtung der Koordinatensysteme in denen die Neigungen oder Oberflächentopographiedaten und das Soll-Ablationsprofil sowie gegebenenfalls die Wassergehaltsdaten angegeben sind, notwendig wird, da das Topographiemeßsystem in der Vorrichtung gehalten ist.

Die Berücksichtigung der Neigung der Oberfläche und/oder der Form des Strahlprofils kann insbesondere dadurch erzielt werden, daß zur Berücksichtigung der Form des Strahlprofils und/oder der Neigung der Oberfläche aus dem Soll-Ablationsprofil unter Verwendung einer Modifikationsfunktion, die von der Form des Strahlprofils und/oder der Neigung der Oberfläche abhängt, ein in Bezug auf Einflüsse der Form des Strahlprofils und/oder der Neigung der Oberfläche vorkompensiertes Soll-Ablationsprofil ermittelt wird, und daß aus dem ermittelten in Bezug auf Einflüsse der Form des Strahlprofils und/oder der Neigung der Oberfläche vorkompensiertes Soll-Ablationsprofil das Ablationsprogramm erstellt wird. Zur Erstellung des Ablationsprogramms aus dem in Bezug auf Einflüsse der Form des Strahlprofils und/oder der Neigung der Oberfläche vorkompensierten Soll-Ablationsprofil können wiederum Erstellungsverfahren verwendet werden, die eine Abhängigkeit von der Form des Strahlprofils und/oder der Neigung der Oberfläche nicht berücksichtigen. Auf diese Weise kann in vorteilhafter Weise eine Kompensation von Fehlern im Wege einer Vorkorrektur oder Vorkompensation erfolgen, bei der das Soll-Ablationsprofil derart modifiziert wird, daß Fehler, die durch Verwendung eines Erstellungsverfahrens, das eine Abhängigkeit von der Form des Strahlprofils und/oder der Neigung der Oberfläche nicht berücksichtigt, auftreten, bei Ablation gemäß dem Ablationsprogramm wenigstens in guter Näherung unterdrückt werden. Vorzugsweise enthält die Modifikationsfunktion die Einflüsse sowohl des Wassergehalts als auch der Oberflächenneigung und der Form des Strahlprofils. Dadurch kann die Erstellung des Ablationsprofils in vorteilhafter Weise vereinfacht werden.

Besonders bevorzugt wird bei dem Verfahren für wenigstens zwei zu ablatierende Bereiche der Oberfläche jeweils ein Wert der strahl- und/oder neigungsabhängigen Modifikationsfunktion in Abhängigkeit von wenigstens der Form des Strahlprofils und/oder der Neigung der Oberfläche in dem jeweiligen Bereich und ein Wert der wassergehaltabhängigen Modifikationsfunktion ermittelt werden und das vorkompensierte Soll-Ablationsprofil unter Verwendung des Soll-Ablationsprofils und der Werte der Modifikationsfunktionen, insbesondere des Produkts der Modifikationsfunktionen, ermittelt wird. Insbesondere kann das unkompensierte Soll-Ablationsprofil zur Erstellung des vorkompensierten Ablationsprofils für jeweils vorgegebene Zielorte mit dem Produkt der Modifikationsfunktionen für diese Orte multipliziert werden. Eine solche Vorkompensation ist besonders einfach und schnell durchzuführen.

Die Gegenstände der den wassergehalt berücksichtigenden Weiterbildung der Erfindung können vorteilhaft zur Ablation eines Soll-Ablationsprofils von biologischem Material, insbesondere der Cornea bei der laserchirurgischen Behandlung einer Fehlsichtigkeit des menschlichen Auges, beispielsweise mit PRK, LASIK oder LASEK, eingesetzt werden.

Die Erfindung insgesamt kann auch vorteilhaft zur Ablation eines Soll-Ablationsprofils von Werkstücken, insbesondere Brillengläsern, Kontaktlinsen und Linsenimplantaten, und vorzugsweise zur laserchirurgischen Behandlung einer Fehlsichtigkeit des menschlichen Auges eingesetzt werden.

Die Erfindung wird im folgenden noch näher beispielhaft anhand der Zeichnungen erläutert. Es zeigen:

Fig. 1    eine schematische perspektivische Darstellung eines Patienten während einer Behandlung mit einem laserchirurgischen Instrument mit einer Ablationsvorrich- tung nach einer bevorzugten Ausführungsform nach einem ersten Aspekt der Erfindung,

Fig. 2    eine schematische Blockdarstellung der Ablationsvorrichtung in Fig. 1,

Fig. 3    eine schematische Darstellung eines sphärischen, zu bearbeitenden Körpers,

Fig. 4    ein Ablaufdiagramm eines Verfahrens zur Erstellung eines Ablationsprogramms für das Instrument in Fig. 2 nach einer ersten bevorzugten Ausführungsform des ersten Aspekts der Erfindung,

Fig. 5    eine Darstellung einer Modifikationsfunktion in Abhängigkeit vom Ort im rotati- onssymmetrischen Strahlprofil eines Laserstrahls für drei verschiedene Erstel- lungsverfahren,

Fig. 6    eine Blockdarstellung einer Ablationsvorrichtung nach einer zweiten bevorzug- ten Ausführungsform des ersten Aspekts der Erfindung für ein laserchirurgi- sches Instruments,

Fig. 7.    eine schematische Darstellung einer Einrichtung zur Erfassung einer Form ei- nes Strahlprofils eines von dem Laser der Ablationsvorrichtung in Fig. 6 abge- gebenen Laserstrahls,

Fig. 8    ein Ablaufdiagramm eines Verfahrens zur Erstellung eines Ablationsprogramms nach einer dritten bevorzugten Ausführungsform des ersten Aspekts der Erfin- dung,

Fig. 9    eine Blockdarstellung eines laserchirurgischen Instruments mit einer Ablations- vorrichtung, die eine Vorrichtung zur Erstellung eines Ablationsprogramms nach einer weiteren bevorzugten Ausführungsform des ersten Aspekts der Erfindung enthält,

Fig. 10    ein Ablaufdiagramm eines Verfahrens zur Erstellung eines Ablationsprogramms nach einer weiteren bevorzugten Ausführungsform des ersten Aspekts der Er- findung,

Fig. 11a und b    Diagramme zum Vergleich von Ablationstiefen, die bei Ablation mit dem Verfah- ren nach Fig. 10 und mit einem bekannten Verfahren erhalten werden, und

Fig. 12    ein Diagramm, in dem ein Verhältnis aus einer Fluence und einem Schwellwert für die Fluence in Abhängigkeit vom Radius in dem Laserstrahl für ein Gauß- sches Strahlprofil und ein abgeschnittenes Gaußsches Strahlprofil gezeigt sind.

Fig. 13    eine schematische Blockdarstellung der Laserablationsvorrichtung mit einer Signalbildungsvorrichtung nach einer ersten bevorzugten Ausführungsform ei- nes zweiten Aspekts der Erfindung,

Fig. 14    eine schematische Darstellung eines Schnitts durch ein Soll-Ablationgsprofil entlang eines Durchmessers durch den Körper in Fig. 3 und von Einzelpuls- Ablationsvolumina,

Fig. 15    ein Ablaufdiagramm eines Verfahrens zur Bildung von Steuersignalen gemäß einem bei dem Verfah- ren erstellten Ablationsprogramm für das Instrument in Fig. 13 nach einer ersten bevorzugten Ausführungsform des zweiten Aspekts der Erfindung,

Fig. 16    ein Ablaufdiagramm eines Verfahrens zur Bildung und Abgabe von Steuersi- gnalen gemäß einem bei dem Verfahren erstellten Ablationsprogramm nach ei- ner weiteren bevorzugten Ausführungsform des zweiten Aspekts der Erfindung,

Fig. 17    ein Ablaufdiagramm mit Teilschritten zu Schritt S128 aus dem Ablaufdiagramm in Fig. 16,

Fig. 18    eine Blockdarstellung einer Ablationsvorrichtung mit einer Signalbildungsvor- richtung nach einer dritten bevorzugten Ausführungsform des zweiten Aspekts der Erfindung für ein laserchirurgisches Instrument,

Fig. 19    Blockdarstellung einer Ablationsvorrichtung mit einer Signalbildungsvorrichtung nach einer weiteren bevorzugten Ausführungsform des zweiten Aspekts der Er- findung, und

Fig. 20    ein Ablaufdiagramm eines Verfahrens zur Bildung und Abgabe von Steuersi- gnalen gemäß einem bei dem Verfahren erstellten Ablationsprogramm nach der weiteren bevorzugten Ausführungsform des zweiten Aspekts der Erfindung in der zweiten Variante.

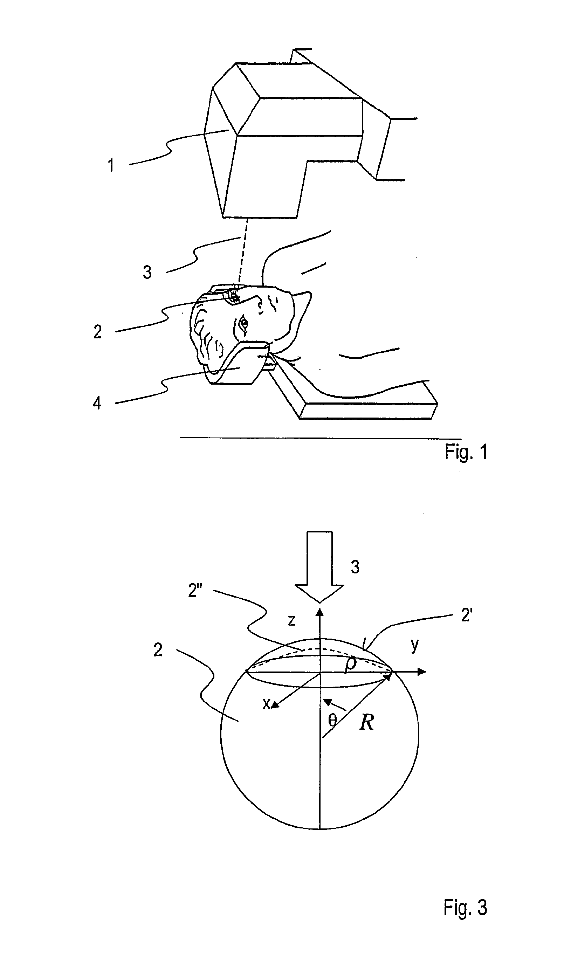

In Fig. 1 ist ein laserchirurgisches Instrument 1 zur Behandlung eines Auges 2 eines Patienten gezeigt, das zur Ausführung einer refraktiven Korrektur des Auges dient. Das Instrument 1 gibt dazu einen gepulsten Laserstrahl 3 auf das Auge 1 des Patienten ab, dessen Kopf in einem Kopfhalter 4, der fest mit dem Instrument 1 verbunden ist, fixiert ist.

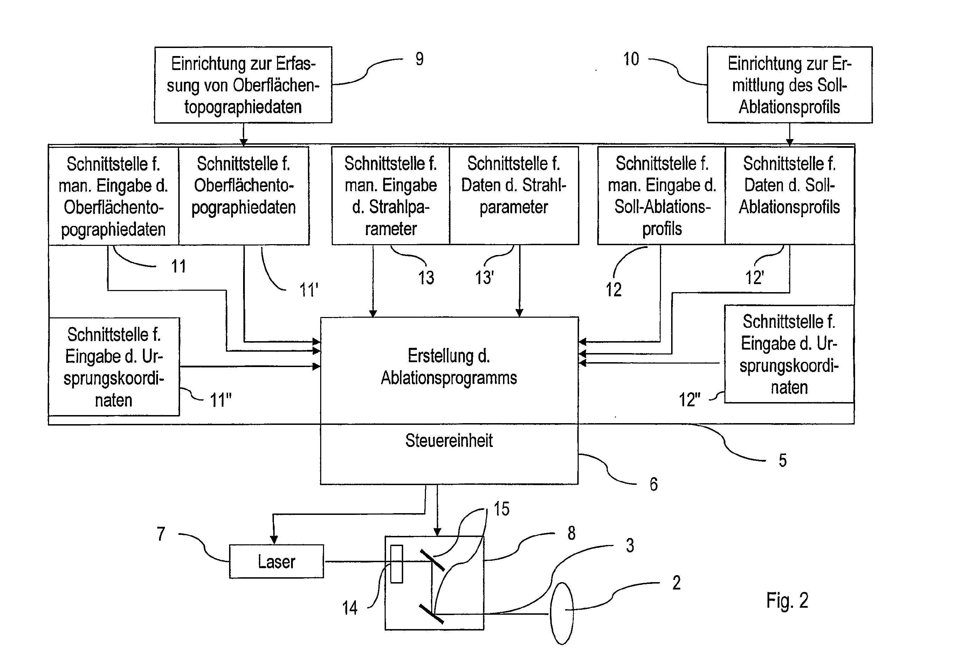

Fig. 2 zeigt genauer den Aufbau des Instruments, das eine Ablationsvorrichtung nach einer ersten bevorzugten Ausführungsform des ersten Aspekts der Erfindung darstellt. Das Instrument umfaßt eine Datenverarbeitungseinrichtung 5 mit einer integrierten Steuereinrichtung 6, einen von der Steuereinrichtung 6 angesteuerten Laser 7 und eine ebenfalls von der Steuereinrichtung angesteuerte Ablenkeinrichtung 8, mittels derer der von dem Laser 7 abgegebene gepulste Laserstrahl 3 entsprechend einem vorgegebenen Ablationsprogramm auf Zielorte auf der Hornhaut des Auges 2 gelenkt und fokussiert werden kann.

Das Instrument verfügt weiter über eine Einrichtung 9 zur Erfassung von Topographiedaten der Oberfläche eines zu behandelnden Bereichs des Auges 2 und eine Einrichtung 10 zur Ermittlung eines Soll-Ablationsprofils. Beide Einrichtungen sind mit der Datenverarbeitungseinrichtung 5 verbunden. Die Datenverarbeitungseinrichtung 5, die Steuereinrichtung 6, die Einrichtung 9 zur Erfassung der Oberflächentopographie und die Einrichtung 10 zur Ermittlung des Soll-Ablationsprofils bilden eine Vorrichtung zur Erstellung eines Ablationsprogramms nach einer ersten bevorzugten Ausführungsform des ersten Aspekts der Erfindung.

Die Datenverarbeitungseinrichtung 5 mit der integrierten Steuereinrichtung 6 dient zur Erstellung eines Ablationsprogramms unter Verwendung von Daten, die die Oberflächentopographie des zu behandelnden Bereichs wiedergeben, Daten in Bezug auf Eigenschaften des Laserstrahls 3 und Daten in Bezug auf das zu ablatierende Soll-Ablationsprofil. Die Datenverarbeitungseinrichtung 5 verfügt hierzu über einen Prozessor und einen Speicher zur Abspeicherung von Daten, in dem insbesondere auch ein Computerprogramm mit Programmcode abgelegt ist, mittels dessen bei Ausführung des Programms auf dem Prozessor das Ablationsprogramm erstellt und unter Verwendung der integrierten Steuereinrichtung 6 die eigentliche Ablation durchgeführt wird. Der Speicher und der Prozessor sind in der Blockdarstellung teilweise durch den Block "Erstellung des Ablationsprogramms" dargestellt.

Die Datenverarbeitungseinrichtung 5 besitzt hierzu Schnittstellen zur Eingabe von Daten, nämlich eine Schnittstelle 11 für manuelle Eingabe der Oberflächentopographie, eine Schnittstelle 11' für das Einlesen von Oberflächentopographiedaten von der Einrichtung zur Erfassung von Oberflächentopographiedaten und eine Eingabeschnittstelle 11" zur Eingabe der Koordinaten eines Bezugspunkts des Koordinatensystems, in dem die Oberflächentopographiedaten angegeben werden, im Beispiel die Koordinaten des Koordinatensystemursprungs, eine Schnittstelle 12 zur manuellen Eingabe des Soll-Ablationsprofils, eine Schnittstelle 12' zum Einlesen von Daten in Bezug auf das Soll-Ablationsprofil von der Einrichtung 10 zur Ermittlung des Soll-Ablationsprofil und eine Schnittstelle 12" zur Eingabe der Koordinaten eines Bezugspunkts des Koordinatensystem, in dem das Soll-Ablationsprofil gegeben ist, im Beispiel des Koordinatensystemursprungs. Darüber hinaus sind eine Schnittstelle 13 zur manuellen Eingabe von Strahlparametern und eine Schnittstelle 13' zum Einlesen von Daten in Bezug auf Strahlparameter für den Laserstrahl 3 von einer Datenquelle vorgesehen. Wenigstens einer der Strahlparameter kann zur Beschreibung der Form des Strahlprofils dienen.

Bei den Schnittstellen für eine manuelle Eingabe und insbesondere auch den Schnittstellen zur Eingabe der Koordinaten der Bezugspunkte kann es sich in physischer Hinsicht um eine einzige Schnittstelle handeln, an die, in den Figuren nicht gezeigt, eine Tastatur und ein Monitor, auf dem ein Eingabeprompt darstellbar ist, wenn entsprechende Daten eingelesen werden sollen, angeschlossen sind. Die Schnittstellen umfassen weiter entsprechende Module des Computerprogramms zum Einlesen der Daten von der Tastatur.

Die anderen Schnittstellen 11', 12' und 13' sind konventionelle Schnittstellen für Datenströme, die neben entsprechenden elektronischen Modulen auch Softwaremodule umfassen.

Die Steuereinrichtung 6 ist in die Datenverarbeitungseinrichtung 5 integriert und verfügt weiter über in den Figuren nicht gezeigte Schnittstellen zur Ansteuerung des Lasers 7 und der Ablenkeinrichtung 8. Solche Steuereinrichtungen sind grundsätzlich bekannt und brauchen daher nicht näher erläutert zu werden.

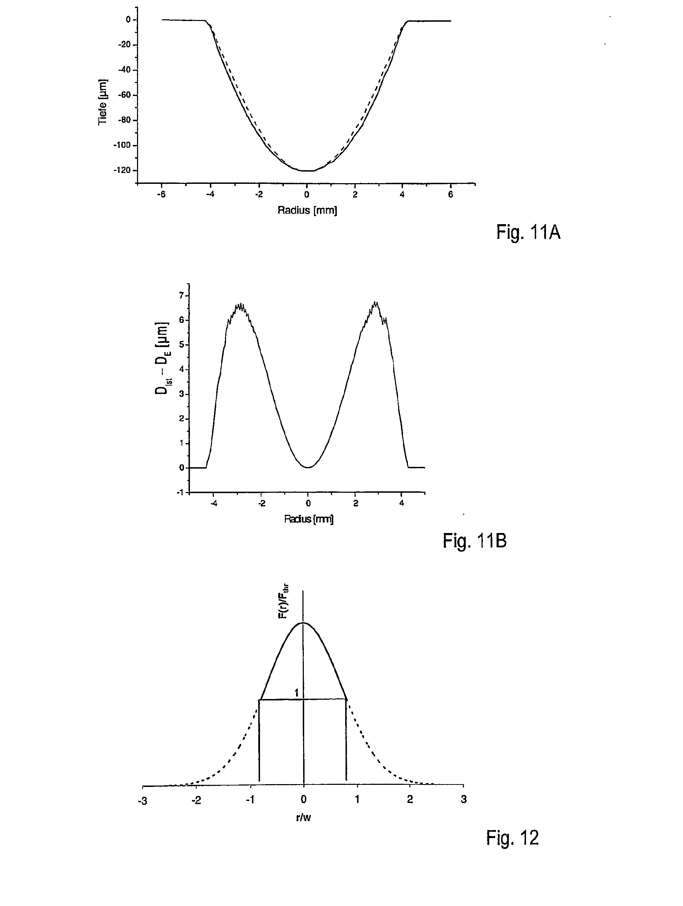

Der Laser 7 ist mit der Steuereinrichtung 6 verbunden und gibt in Abhängigkeit von dem Ablationsprogramm einen gepulsten Laserstrahl mit vorgegebenen Pulsenergien ab. Beispielsweise kann ein Excimerlaser mit einer Wellenlänge im Wellenlängenbereich von 193 nm verwendet werden. Der von dem Laser 7 abgegebene Laserstrahl 3 hat ein in Fig. 12 durch eine gestrichelte Linie dargestelltes Strahlprofil mit Gaußform.

Die Ablenkeinrichtung 8 ist ebenfalls über eine Datenverbindung mit der Steuereinrichtung 6 verbunden und lenkt entsprechend Steuersignalen von der Steuereinrichtung 6 gemäß dem abzuarbeitenden Ablationsprogramm den von dem Laser 7 abgegebenen, gepulsten Laserstrahl 3 auf vorgegebene Zielorte auf der Oberfläche des Auges 2. Hierzu verfügt die Ablenkeinrichtung 8 über eine Fokussierungseinrichtung 14 zur Fokussierung des Laserstrahls entlang seiner Ausbreitungsrichtung und zur Ablenkung quer zu dem Laserstrahl über zwei um jeweils orthogonal zueinander verlaufende Achsen dreh- bzw. schwenkbare Spiegel 15, die im Strahlengang nach der Fokussiereinrichtung 14 angeordnet sind.

Sowohl bei dem Laser 7 als auch bei der Ablenkeinrichtung 8 kann es sich um konventionelle, bekannte Einrichtungen eines laserchirurgischen Instruments handeln.

Zur Darstellung der Oberflächentopographie werden zwei parallel zueinander ausgerichtete kartesische Koordinatensysteme verwendet, deren x-y-Ebenen zusammenfallen. Die z-Achse ist dabei möglichst in guter Näherung parallel zu der optischen Achse des Auges ausgerichtet. In Fig. 3 ist dies für einen sphärischen Körper bzw. eine Kugel mit einer Oberfläche 2' als vereinfachtes Modell für das Auge 2 dargestellt, wobei der Übersicht halber die z-Achse zur Darstellung des Soll-Ablationsprofils nicht gezeigt ist. Die Festlegung der z-Achsen erfolgt bei der Ausrichtung des Auges 2 relativ zu dem Instrument 1.

Die Einrichtung 9 zur Erfassung von Oberflächentopographiedaten umfaßt im Beispiel einen optischen Kohärenztomographen, der in dem Instrument 1 so angeordnet ist, daß mit diesem die Oberflächentopographie des Auges 2 im zu behandelnden Bereich erfaßt werden kann. Von dem optischen Kohärenztomographen werden Oberflächentopographiedaten in Form von Höhen in z-Richtung an Gitterpunkten in der in Bezug auf das Instrument 1 festen näherungsweise orthogonal zu dem Laserstrahl 3 verlaufenden x-y-Ebene erfaßt. Die Daten werden in die Datenverarbeitungseinrichtung 5 über die Schnittstelle 11' eingelesen.

Die Einrichtung 10 zur Ermittlung des Soll-Ablationsprofils umfaßt im vorliegenden Beispiel einen Wellenfrontanalysator vom Hartmann-Shack-Typ sowie gegebenenfalls Einrichtungen zur Ermittlung der Brechkraft des Auges 2, aus dem bzw. denen nach bekannten Verfahren ein Soll-Ablationsprofil $D_{soll}$ für den zu behandelnden Bereich des Auges 2 ermittelt werden kann. Das Soll-Ablationsprofil wird dabei so bestimmt, daß eine möglichst weitgehende Korrektur von Abbildungsfehlern durch das Auge 2 durch die noch durchzuführende Ablation erreicht werden kann. Ein Beispiel für das Soll-Ablationsprofil ist Fig. 3 entnehmbar. Es ist durch die Abstände in z-Richtung zwischen der anfänglichen Oberfläche, der Kugelkappe 2', und einer gewünschten Oberfläche 2", die durch eine gestrichelte Linie angedeutet ist, als Funktion des Ortes in der x-y-Ebene gegeben.

Zur Ermittlung des Soll-Ablationsprofils kann die Einrichtung 10 über einen entsprechenden Prozessor verfügen, der die Daten in Bezug auf die Brechkraft des Auges 2 und die Wellenfrontdaten auswertet, um daraus im vorliegenden Beispiel wiederum in Form von Soll-Ablationstiefen für Punkte eines Punktegitters in der x-y Ebene des entsprechenden Koordinatensystems, die mit der x-y Ebene des Koordinatensystems zur Angabe der zusammenfällt, das Soll-Ablationsprofil zu ermitteln. Soweit die Koordinatenursprünge nicht übereinstimmen, kann die Lage eines der Bezugspunkte bzw. Koordinatenursprünge in dem jeweils anderen Koordinatensystem angegeben werden, so daß im späteren Verfahrensverlauf die Daten durch eine einfache Verschiebung in ein gleiches Koordinatensystem transformiert werden können. Dies kann sich dann als günstig erweisen, wenn das Zentrum des Soll-Ablationsprofils, das heißt ein Punkt, relativ zu dem das Soll-Ablationsprofil näherungsweise symmetrisch ist, von dem Zentrum der Oberfläche des Auges 2, das heißt einem Punkt, relativ zu dem die Oberfläche des Auges näherungsweise symmetrisch ist, abweicht. Ein Verfahren zur Erstellung eines Soll-Ablationsprofils ist beispielsweise in der WO 01/08075 A1 beschrieben, deren Inhalt insoweit hiermit durch Bezugnahme in die Beschreibung aufgenommen wird.

Das Verfahren zur Erstellung eines Ablationsprogramms des ersten Ausführungsbeispiels beruht auf folgenden Überlegungen.

Um das Soll-Ablationsprofil $D_{soll}$ von dem Auge 2 zu ablatieren, werden entsprechend einem erstellten Ablationsprogramm auf vorgegebene Zielorte auf dem Auge 2 Laserpulse einer vorgegeben Pulsenergie abgegeben, die jeweils einzeln zu einem Abtrag von Material führen. Die Tiefe des Abtrags kann durch verschiedene Modelle beschrieben werden. Im vorliegenden Beispiel wird das sogenannte "Blow-Off"-Modell verwendet, das beispielsweise in "Refraktive Chirurgie der Hornhaut", Herausgeber Theo Seiler, 1. Auflage, ENKE im Georg Thieme Verlag, Stuttgart / New York, 2000 (ISBN 3-13-118071-4), Kapitel 6.1, S.150 oder in R. Srinivasan: "Ablation of Polymers and Biological Tissue by Ultraviolet Lasers", Science vol. 234, p.559-565, 31. Oct. 1986 beschrieben ist. Danach wird an einem Ort mit den Koordinaten (x, y) durch eine an diesem Ort auftreffende Laserstrahlung mit einer wirksamen Fluence F(x,y), das heißt Energie pro Fläche, Material bis in eine Tiefe $D_{Ist}$ gemäß folgender Formel abgetragen:

$$D_{Ist}(x,y) = \begin{cases} \mu \cdot \ln \dfrac{F(x,y)}{F_{thr}} & ,falls\ F(x,y) > F_{thr} \\ 0 & ,sonst \end{cases}.$$

Dabei bezeichnet $\mu$ einen materialabhängigen Ablationskoeffizienten und $F_{thr}$ einen ebenfalls materialabhängigen Ablationsschwellwert für die Fluence, unterhalb dessen Laserstrahlung keinen Abtrag von Material von dem Auge 2 mehr bewirkt.

Bei dem im Folgenden verwendeten einfachen Erstellungsverfahren für ein Ablationsprogramm wird davon ausgegangen, daß das Soll-Ablationsprofil in Einzelpuls-Ablationsvolumina, die bei Auftreffen eines Pulses entstehen, zerlegt werden kann. Die Erstellung des Ablationsprogramms beinhaltet dann die Bestimmung der Zielorte (x,y), auf die Pulse einer vorgegebenen Pulsenergie gelenkt werden müssen.

Zur Erstellung eines Ablationsprogramms wird davon ausgegangen, daß ein Laserpuls ein Einzelpuls- bzw. Spot-Ablationsvolumen $V_{Puls}$ abträgt, das sich als Integral der Ablationstiefe $D_{Ist}$ über die gesamte Pulsfläche ergibt:

$$V_{Puls} = \iint\limits_{Spot} D_{Ist}(x,y)\,dxdy = \mu \iint\limits_{Spot} \ln \frac{F(x,y)}{F_{thr}}\,dxdy\,.$$

Das Integral erstreckt sich dabei über die mit "Spot" bezeichnete Fläche, in der $D_{Ist} > 0$ ist.

Während bei konventionellen Verfahren die wirksame Fluence F (x, y) als konstant über die Fläche des Pulses bzw. Spots angenommen wird, werden erfindungsgemäß zwei Einflüsse gleichzeitig berücksichtigt. Zum einen wird berücksichtigt, daß durch die Neigung der zu bearbeitenden Oberfläche die wirksame Fluence entsprechend der Neigung gegenüber der Fluence des Laserstrahls 3 herabgesetzt wird. Wird die zu bearbeitende Oberfläche durch eine Höhenfunktion f(x, y), die die Höhe der Oberfläche über der x-y-Ebene angibt, beschrieben, kann der Neigungswinkel θ der Oberfläche relativ zu der z-Achse und damit näherungsweise zu dem auf die Oberfläche 2' einfallenden Laserstrahl 3 gemäß der Formel

$$\theta(x,y) = \arctan\left(\left|\operatorname{grad} f(x,y)\right|\right)$$

ermittelt werden. Die bei der Ablation wirksame Fluence F(x, y) auf der Oberfläche ergibt sich damit zu

$$F(x,y) = F_P(x,y) \cdot \cos\theta(x,y)$$

wobei Fp(x,y) die Fluence bei senkrechtem Einfall des Laserstrahls 3 auf die Oberfläche, d.h. bei einem Winkel θ=0 bezeichnet. $F_P$ entspricht daher der Fluence bzw. dem Strahlprofil des Laserstrahls 3.

Als zweiter Effekt wird berücksichtigt, daß die wirksame Fluence entsprechend dem Intensitäts- bzw. Energieprofil des Pulses in einer Ebene orthogonal zur Richtung des Laserstrahls 3 variiert und dabei insbesondere auch den Schwellwert $F_{thr}$ unterschreiten kann.

Damit ergibt sich das tatsächliche Einzelpuls-Ablationsvolumen aus der folgenden Formel:

$$V_{Puls} = \iint\limits_{Spot} D_{Ist}(x,y)dxdy = \mu \iint\limits_{Spot} \ln\frac{F(x,y)}{F_{thr}}dxdy = \iint\limits_{Spot} \ln\frac{F_P(x,y)\cos\theta(x,y)}{F_{thr}}dxdy.$$

Das Einzelpuls-Ablationsvolumen hängt daher nichtlinear vom Neigungswinkel der Oberfläche und dem Fluenceprofil $F_P(x,y)$ ab.

Bei bekannten Einzelpuls-Ablationsvolumina kann mit bekannten einfachen Erstellungsverfahren wie sie beispielsweise in DE 197 27 573 C1 oder EP 1060710 A2 beschrieben sind, ein Ablationsprogramm erstellt werden, gemäß dem sich die mittlere Ablationstiefe $D_M$ gleichmäßig oder langsam variierend unter einem Abstand a auf die Oberfläche gelenkter Laserstrahlpulse gemäß

$$D_M = c\frac{V_{Puls}}{a^2}$$

ergibt. c ist hierbei ein Proportionalitätsfaktor der sich aus der Anordnung der Auftreffpunkte der Pulse des Laserstrahls ergibt. Beispielsweise gilt bei einem näherungsweise quadratischen Muster c=1, während bei einem näherungsweise hexagonalen Muster c=2/√3 gilt.

In folgenden wird davon ausgegangen, daß sich das Ablationsprofil räumlich langsam gegenüber der Länge des Abstands a oder die Neigung langsam gegenüber dem Durchmesser des Laserstrahls 3 an der Oberfläche verändert. Zur Beschreibung der räumlichen Abhängigkeit dieser räumlich langsam veränderlichen Größen werden im folgenden Koordinaten u und v verwendet, die in einem u-v-Koordinatensystem gegeben sind, das mit dem x-y-Koordinatensystem zusammenfällt. Das Einzelpuls-Ablationsvolumen ist damit ebenso wie die sich aus den nebeneinandergesetzten Pulsen ergebende mittlere Tiefe $D_M$ als Funktion von u und v aufzufassen.

Es kann dann eine von u und v abhängige Modifikationsfunktion K berechnet werden, die sich zu

$$K(u,v) = \frac{V_{Puls,Ist}(u,v)\cos\theta(u,v)}{V_{Puls,E}(u,v)}$$

ergibt. Dabei bezeichnet der Index "E" Größen, die bei Verwendung eines einfachen Erstellungsverfahrens, d.h. ohne Berücksichtigung der Neigung und der Form des Strahlprofils, ermittelt werden. Der Faktor $\cos(\theta(u,v))$ kommt dadurch zustande, daß sich der Abstand a in der x-y-Ebene aufgrund der Neigung der Oberfläche in einer Richtung um $1/\cos(\theta(u,v))$ vergrößert. $V_{Puls,Ist}(u,v)$ kann selbst von $\cos(\theta(u,v))$ abhängen. K hängt über $V_{Puls,Ist}$ von der Form des Strahlprofils ab.

Würde mit einem der geschilderten einfachen Erstellungsverfahren ausgehend von dem Soll-Ablationsprofil $D_{Soll}$ ein Ablationsprogramm ermittelt, ergäbe sich ein Ist-Ablationsprofil, das entsprechend der Modifikationsfunktion K gegenüber dem Soll-Ablationsprofil reduziert ist:

$$D_{Ist}(u,v) = K(u,v) \times D_{Soll}.$$

Daher wird, um diesen Fehler vorweg zu kompensieren, das zu ablatierende Soll-Ablationsprofil $D_{Soll}$ durch die Modifikationsfunktion K dividiert und damit ein modifiziertes oder vorkompensiertes Soll-Ablationsprofil $D_{Mod}$ erhalten:

$$D_{Mod}(u,v) = \frac{D_{Soll}(u,v)}{K(u,v)}.$$

Dieses führt mit dem einfachen Erstellungsverfahren zu einem Ablationsprogramm, das bei Ausführung das Soll-Ablationsprofil $D_{Soll}$ ergibt. Wird aus dem vorkompensierten bzw. modifizierten Soll-Ablationsprofil ein Ablationsprogramm mit den konventionellen einfachen Erstellungsverfahren ermittelt, ergibt sich auch bei zu dem Laserstrahl geneigten Oberflächen und einem nicht konstant verlaufenden Strahlprofil in sehr guter Näherung bei Ablation das Soll-Ablationsprofil.

Zu Ermittlung der Orte, auf die Laserpulse abzugeben sind, können verschiedene bekannte Verfahren verwendet werden, beispielsweise die in DE 19727573 C1 und EP 1060710 A2 beschriebenen Verfahren. In DE 19727573 C1 erfolgt die Ablation schichtweise, das heißt es werden Auftrefforte für Pulse schichtweise ermittelt, wobei sich das gewünschte Profil dann durch Überlagerung dieser Schichten ergibt. Bei dem Verfahren in EP 1060710 A2 wird der Spotabstand quasi- kontinuierlich variiert, um die gewünschte Ablationstiefe zu erreichen.

Die Berechnung der Modifikationsfunktion sei am Beispiel der Ablation von einer sphärischen Oberfläche mit einem Laserstrahl mit einem Strahlprofil mit Gauß-Form erläutert (vgl. Fig. 3).

Das Strahlprofil bzw. die Fluence ist durch die Formel

$$F_P(r) = F_0 e^{-\frac{r^2}{w^2}}$$

beschrieben, wobei $F_0$ der Spitzenwert der Fluence, r der radiale Abstand zum Zentrum des Strahlprofils und w der Abstand ist, nach dem das Profil relativ zu dem Wert im Zentrum auf 1/e abgefallen ist.

Damit ergibt sich für das Ist-Ablationsprofil eines Laserpulses, der orthogonal auf eine ebene Fläche auftrifft:

$$D_{Ist}(r) = \mu \ln \frac{F_0}{F_{thr}} - \mu \frac{r^2}{w^2},$$

so daß sich das Einzelpuls-Ablationsvolumen für diesen Puls zu

$$V_{Puls,E} = \frac{\pi}{2}\mu w^2 \left[ \ln \frac{F_0}{F_{thr}} \right]^2$$

berechnet. Als mittlere Tiefe des gewünschten Profils bei einem quadratischen Muster der Auftreffpunkte der Pulse bzw. Spot-Muster mit Kantenlänge a ist eine mittlere Tiefe der Ablation gemäß

$$D_E(r) = \frac{V_{s,E}}{a^2} = \frac{\pi}{2}\mu \frac{w^2}{a^2} \left[ \ln \frac{F_0}{F_{thr}} \right]^2$$

zu erwarten. Setzte man nun $D_E$ gleich $D_{Soll}$ so könnte a als Funktion des Ortes ermittelt werden. Tatsächlich ist die sphärische Oberfläche jedoch außer im Zentrum gegenüber dem als in hinreichend guter Näherung parallel zur z-Achse einfallend angenommen Laserstrahl 3 geneigt. Wie sich aus Abbildung 3 ergibt, kann der Neigungswinkel in Abhängigkeit von dem Abstand $\rho$ eines Ortes auf der Oberfläche des Auges 2 folgendermaßen berechnet werden:

$$\theta(\rho) = \arcsin\left(\frac{-\rho}{R}\right) = \arctan\left(\frac{-\rho}{\sqrt{R^2 - \rho^2}}\right).$$

Die Neigung der Oberfläche am Ort $\rho$ führt nun dazu, daß sowohl der Spotabstand in der Neigungsrichtung als auch die Spotbreite w in Neigungsrichtung um den Faktor $1/\cos(\theta(\rho))$ vergrößert werden. Daher ändert sich das Verhältnis w/a in der Gleichung für D nicht mit der Neigung. Es ändert sich jedoch die Fluence in dieser Gleichung. Man erhält für das zu erwartende Tiefenprofil auf der sphärischen Fläche daher folgendes Ergebnis:

$$D_{Ist}(\rho) = \frac{V_{Puls,Ist}(\rho)\cos\theta(\rho)}{a^2} = \frac{\pi}{2}\mu\frac{w^2}{a^2}\left[\ln\frac{F_0 \cdot \cos\theta(\rho)}{F_{thr}}\right]^2$$

Die Modifikationsfunktion K(p) berechnet sich dann zu

$$K(\rho) = \frac{V_{Puls,Ist}(\rho)\cos\theta(\rho)}{V_{Puls,E}} = \left(1 + \frac{\ln\cos\theta(\rho)}{\ln\frac{F_0}{F_{thr}}}\right)^2$$

Systematisch kann dieses Resultat auch dadurch erhalten werden, daß in der Formel für das Einzelpuls-Ablationsvolumen $\cos(\theta)$ als über die Spotfläche konstant angesehen wird.

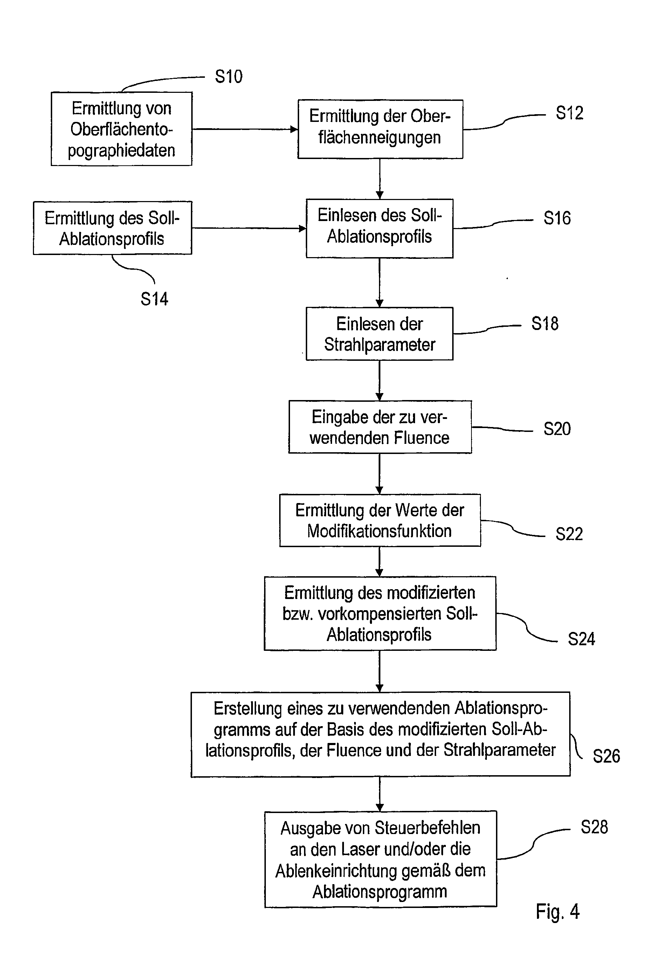

Zur Ermittlung des Ablationsprogramms werden die in dem Ablaufdiagramm in Fig. 4 skizzierten Verfahrensschritte durchgeführt.

Zunächst wird in Schritt S10 die Oberflächentopographie des zu behandelnden Bereichs mittels der Einrichtung 9 zu Erfassung der Oberflächentopographiedaten ermittelt. Die entsprechenden Daten können beispielsweise über einem Punktraster in der x-y-Ebene erfaßte Höhen der Oberfläche gegenüber der x-y-Ebene umfassen.

In Schritt S12 werden diesen Daten dann über die Schnittstelle 11' für die Oberflächentopographiedaten in die Datenverarbeitungseinrichtung 5 eingelesen. Aus den Höhendaten werden dann Oberflächenneigungen durch numerische Ermittlungen von Gradienten und die oben angegebene Formel für den Neigungswinkel ermittelt. Die entsprechenden Daten werden dann in dem Speicher der Datenverarbeitungseinrichtung 5 gespeichert.

Im Beispiel nach den Schritten S10 und S12, in anderen Ausführungsbeispielen aber auch vor diesen Schritten, wird in Schritt S14 das Soll-Ablationsprofil $D_{Soll}$ unter Verwendung der Einrichtung 10 zur Ermittlung des Soll-Ablationsprofils ermittelt. Hierzu werden aus Wellenfrontdaten Abbildungsfehler ermittelt. Mit bekannten Verfahren wird errechnet, an welchen Stellen der Oberfläche des Auges Material bis zu welcher Tiefe abzutragen ist. Im vorliegenden Beispiel ist das Soll-Ablationsprofil $D_{Soll}$ durch die Angabe der Soll-Ablationstiefe als Funktion des Ortes auf einem Gitter in der x-y-Ebene gegeben.

Diese Daten werden dann in Schritt S16 über die Schnittstelle 12' in die Datenverarbeitungseinrichtung 5 eingelesen und dort in deren Speicher abgespeichert.

In Schritt S18 werden dann über die Schnittstelle 13 Strahlparameter des zu verwendenden Laserstrahls 3 eingelesen. In diesem Ausführungsbeispiel wird dazu nur der Durchmesser oder die Halbwertsbreite eines Gaußschen Strahlprofils des Laserstrahls 3 eingegeben. Die Gauß-Form des Laserstrahls 3 wird als fest vorgegeben angenommen und ist in Form entsprechender Formeln in dem auf dem Prozessor der Datenverarbeitungseinrichtung 5 ablaufenden Programm berücksichtigt.

In Schritt S20 wird dann noch die zu verwendende Pulsenergie pro Fläche bzw. die zu verwendende Fluence eingelesen und gespeichert. Genauer wird der Spitzenwert $F_0$ der Fluence für das Gauß-Profil eingelesen.

In dem Schritt S22 werden dann Werte der Modifikationsfunktion K für das gesamte Soll-Ablationsprofil ermittelt, wobei gegebenenfalls Werte des Neigungswinkels auf dem Raster, das zur Angabe des Soll-Ablationsprofil verwendet wird, durch Interpolation bestimmt werden. Hierzu wird die oben angegebene Formel für die Funktion K verwendet. Die entsprechenden Werte werden dann wiederum abgespeichert.

In dem Schritt S24 wird daraufhin ein modifiziertes bzw. vorkompensiertes Soll-Ablationsprofil $D_{Mod}$ ermittelt, indem die durch das eingelesene Soll-Ablationsprofil gegebenen Soll-Ablationstiefen für jeweilige Rasterpunkte durch den für den jeweiligen Rasterpunkt ermittelten Wert der Modifikationsfunktion K dividiert werden. Das modifizierte Soll-Ablationsprofil wird dann abgespeichert.

In dem folgenden Schritt S26 wird auf der Basis des modifizierten Soll-Ablationsprofils $D_{Mod}$, der eingelesenen Fluence und des Strahlparameters ein Ablationsprogramm erstellt, wozu ein Verfahren verwendet wird, das nicht gleichzeitig die Oberflächenneigung und die Form des Strahlprofils berücksichtigt. Beispielsweise kann das in DE 19727573 C1 beschriebene Verfahren verwendet werden. Das so erstellte Ablationsprogramm umfaßt dabei eine Folge von Zielorten in der x-y-Ebene, das heißt entsprechende Koordinaten, auf die Laserpulse mit der durch die eingelesene Fluence bestimmten Pulsenergie gelenkt werden müssen, um das gewünschte Soll-Ablationsprofil zu erreichen. Das Ablationsprogramm wird in der Datenverarbeitungseinrichtung 5 gespeichert. Mit diesem Schritt ist die eigentliche Erstellung des Ablationsprogramms beendet.

In dem folgenden Schritt S28 werden dann von der Datenverarbeitungseinrichtung 5 mit der integrierten Steuereinrichtung 6 Steuerbefehle an den Laser 7 und die Ablenkeinrichtung 8 abgegeben, um entsprechend dem erstellten Ablationsprogramm Material von dem Auge 2 abzutragen.

Das Ablationsverfahren eignet sich sowohl für die photorefraktive Keratektomie als auch LASIK. Da bei diesen Verfahren Material in unterschiedlichen Schichten des Auges abgetragen wird, müssen entsprechend gegebenenfalls unterschiedliche Soll-Ablationsprofile verwendet werden.

Bei einer anderen Variante des soeben beschriebenen Verfahrens kann der benötigte Speicherplatz reduziert werden, indem die Schritte S22 und S24 zusammen gefaßt werden und die Modifikationsfunktion für jede Stützstelle des Soll-Ablationsprofils berechnet wird, ohne nach der Ermittlung des entsprechenden modifizierten Soll-Ablationsprofilwertes gespeichert zu werden.

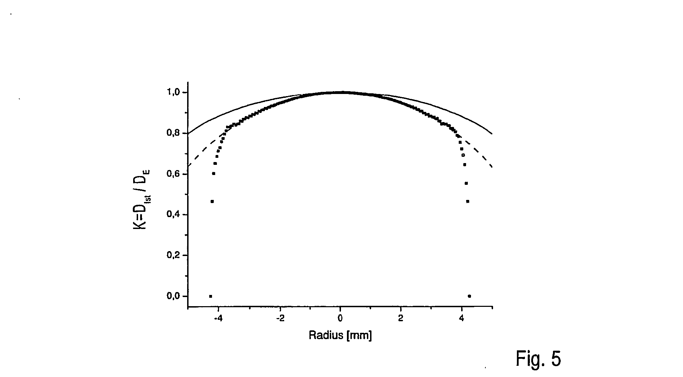

Figur 5 zeigt den Modifikationsfaktor als Funktion des Radius $\rho$ anhand eines konvexsphärischen Ablationsprofils auf einem kreisförmigen Gebiet mit einem Durchmesser von 8 mm, das auf einer Oberfläche mit 7,86 mm Krümmungsradius durch Ablation gebildet wurde. Die durchgezogene Linie zeigt dabei einen Korrekturfaktor, der mit dem in WO 01/85075 A1 angegebenen Verfahren, das heißt insbesondere ohne Berücksichtigung der Form des Strahlprofils, erhalten wird. Die gestrichelte Linie stellt das mit dem soeben beschriebenen Verfahren erzielte Ergebnis dar. Man erkennt leicht, daß mit zunehmendem radialem Abstand von dem Zentrum der Kugelkappe und damit zunehmender Neigung die Korrekturen mit dem vorliegenden Verfahren an Bedeutung gewinnen und sich erheblich von denen nach dem Stand der Technik unterscheiden.

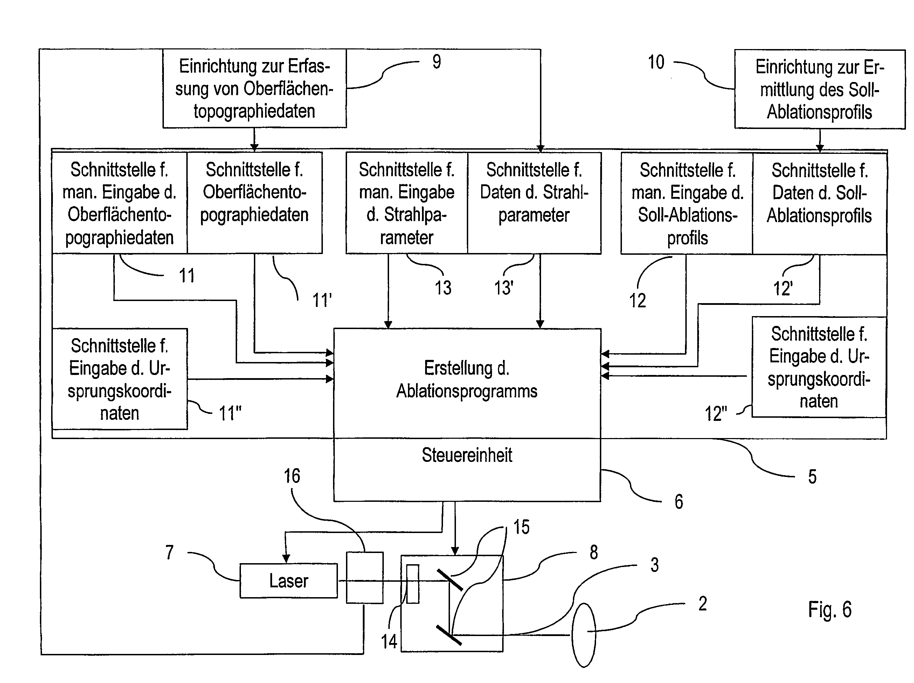

Figur 6 zeigt ein Instrument nach einer weiteren bevorzugten Ausführungsform des ersten Aspekts der Erfindung, das sich von dem Instrument des ersten Ausführungsbeispiels dadurch unterscheidet, daß nun eine Einrichtung 16 zur Ermittlung des Strahlprofils des Laserstrahls 3 in dem Strahlgang des Laserstrahls 3 zwischen dem Laser 7 und der Ablenkeinrichtung 8 angeordnet ist, mittels derer das Strahlprofil des Laserstrahls 3 ermittelbar und über eine entsprechende Datenverbindung über die Schnittstelle 13' für die Strahlparameter der Datenverarbeitungseinrichtung 5 zuführbar ist, die zur Durchführung der im folgenden geschilderten Variante des Erstellungsverfahrens programmiert ist.

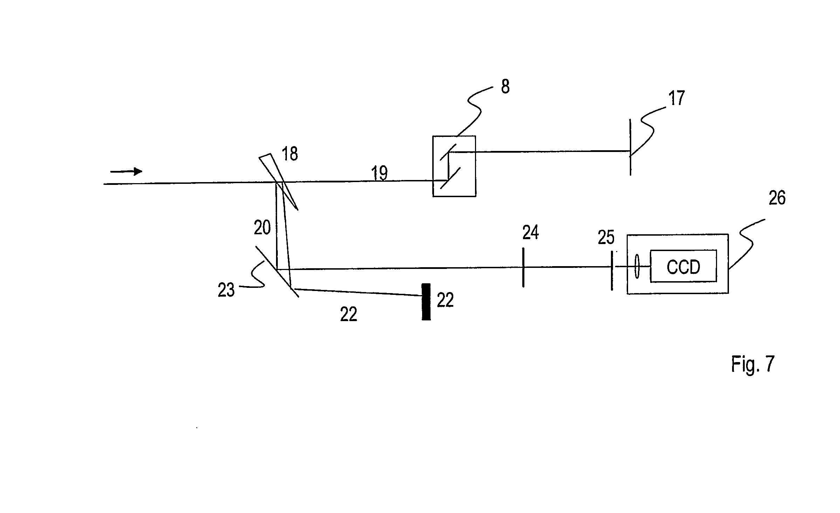

Die Einrichtung 16 zur Ermittlung des Strahlprofils ist in Figur 7 genauer dargestellt. Der Laserstrahl 3 wird, bevor er die Arbeitsebene 17, das heißt die Ebene, in der die Ablation stattfindet, erreicht, mit Hilfe eines Strahlteilers 18 in Form einer Keilplatte in einen Bearbeitungsstrahl 19 und einen Meßstrahl 20 aufgeteilt. Die Keilplatte 18 erzeugt durch Reflexion an Ihrer zweiten Oberfläche noch einen weiteren Strahl 21, der durch eine Blende 22 aufgefangen wird.

Der Meßstrahl 20 wird von einem Spiegel 23 umgelenkt, trifft auf einen optionalen Abschwächer 24 und fällt dann auf eine Mattscheibe oder, bei Verwendung von UV-Licht, eine Fluoreszenzscheibe 25. Der Spiegel 23 und die Mattscheibe 25 sind dabei so angeordnet, daß der Meßstrahl 20 näherungsweise die gleiche Weglänge durchläuft wie der Bearbeitungsstrahl 19 auf seinem Weg von dem Strahlteiler 18 zu der Arbeitsebene 17.

Mit einer Video-Kamera 26 als ortsauflösendem Detektor, beispielsweise einer CCD-Kamera, wird das Bild des Streulichts der Mattscheibe bzw. des Fluoreszenzlichts ortsaufgelöst aufgenommen und elektronisch ausgewertet. Die Video-Kamera 26 ist dabei so gewählt, daß deren Aufnahmerate größer ist als die verwendete Repetitionsrate des Lasers 7, so daß eine Vermessung des Strahlprofils auch während des Ablationsvorgangs möglich ist. Bei langsamen Veränderungen des Strahlprofils kann auch eine langsame Analyse des Strahlprofils vor Durchführung des Ablationsvorgangs erfolgen.

Ein entsprechendes Verfahren zur Erstellung eines Ablationsprogramms unterscheidet sich von dem entsprechenden Verfahren des ersten Ausführungsbeispiels darin, daß in dem Schritt S18 nicht die Breite des Gaußschen Strahls eingelesen wird, sondern das mit der Einrichtung 16 ermittelte Strahlprofil.

Darüber hinaus werden in Schritt S22 die Werte der Modifikationsfunktion durch numerische Integration ermittelt gemäß folgender Formel

$$K(u,v) = \frac{V_{Puls,Ist}(u,v)\cos\theta(u,v)}{V_{Puls,E}(u,v)} = \frac{\int\limits_{Spot} r \ln\dfrac{F_P(r)\cos\theta(u,v)}{F_{thr}}\,dr}{\int\limits_{Spot} r \ln\dfrac{F_0}{F_{thr}}\,dr}\cos\theta(u,v),$$

wobei angenommen wird, daß das Strahlprofil rotationssymmetrisch ist, und r den Radius quer zur Ausbreitungsrichtung des Laserstrahls bezeichnet.

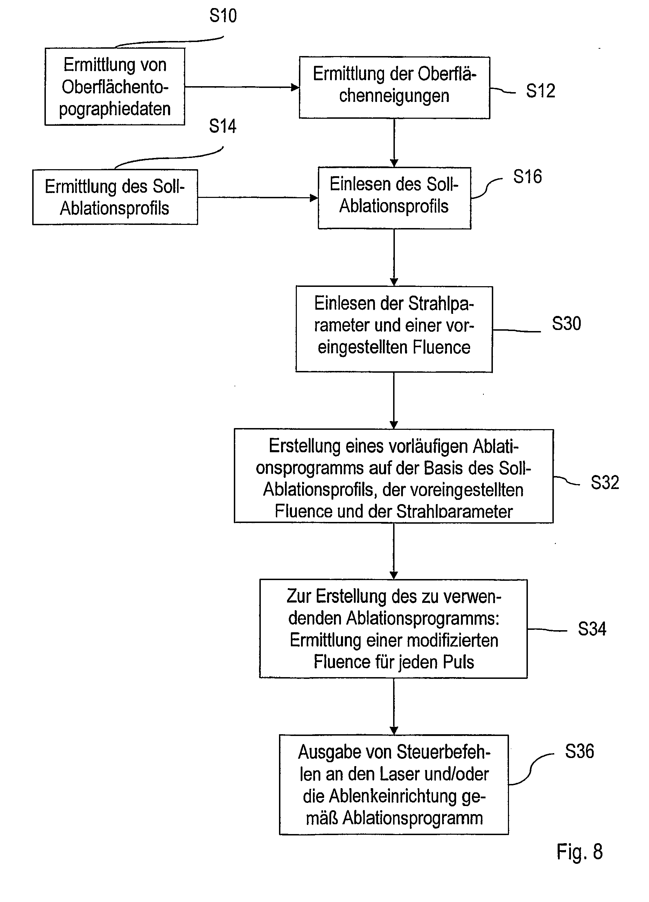

Ein drittes Ausführungsbeispiel für ein Verfahren zur Erstellung eines Ablationsprogramms ist in Fig. 8 dargestellt. Ein entsprechendes Instrument unterscheidet sich von dem Instrument des ersten Ausführungsbeispiels in der Programmierung der Datenverarbeitungseinrichtung 5 derart, daß diese das im folgenden geschilderte Verfahren zur Ablation durchführen kann.

Das Ablationsverfahren, das das Verfahren zur Erstellung eines Ablationsprogramms einschließt, weist einige Schritte auf, die denen des in Figur 4 beschriebenen Verfahrens entsprechen. Diese werden daher im folgenden mit den gleichen Bezugszeichen bezeichnet und nicht nochmals näher beschrieben.

In den Schritten S10 und S12 werden also Daten zur Oberflächenneigung und in den Schritten S14 und S16 zu dem Soll-Ablationsprofil ermittelt und gespeichert.

In dem dann folgenden Schritt S30 werden unter der Vorgabe eines Gaußschen Strahlprofils dessen Breite und ein voreingestellter Anfangsfluencewert, d.h. ein voreingestellter Anfangsspitzenwert, eingelesen.

In dem folgenden Schritt S32 wird dann ein vorläufiges Ablationsprogramm auf der Basis des eingelesenen Soll-Ablationsprofils, der eingelesenen voreingestellten Fluence und der Strahlparameter erstellt, wobei das hierzu verwendete einfache Erstellungsverfahren die Form des Strahlprofils zusammen mit der Neigung nicht berücksichtigt. Insbesondere kann das oben erwähnte Verfahren aus DE 197 27 573 C1 verwendet werden. Das vorläufige Ablationsprogramm, das wiederum aus einer Folge von Koordinaten für Orte auf der Oberfläche des Auges 2, auf die Pulse zu lenken sind, umfaßt, wird dann abgespeichert.

Daraufhin werden in Schritt S34 zur Erstellung des schließlich zu verwendenden Ablationsprogramms gegenüber dem voreingestellten Fluence-Spitzenwert modifizierte Fluence-Werte bzw. Pulsenergien für jeden Puls ermittelt. Geht das verwendete einfache Verfahren zur Erstellung des vorläufigen Ablationsprogramms von einem Einzelpuls-Ablationsvolumen $V_{S,E}$ aus, so ergibt sich, wie oben bereits ausgeführt, folgende Beziehung zwischen dem tatsächlichen Einzelpuls-Ablationsvolumen $V_{S,Ist}$ für einen Puls und dem zur Erstellung des vorläufigen Ablationsprogramm verwendeten Einzelpuls-Ablationsvolumens $V_{S,E}$:

$$K(u,v) = \frac{V_{Puls,Ist}(u,v)\cos(\theta(u,v))}{V_{Puls,E}(u,v)}.$$

$V_{Puls,Ist}$ und $V_{Puls,E}$ sind dabei Funktionen des dimensionslosen Verhältnisses $F_0/F_{THR}$. $V_{Puls,Ist}$ hängt darüber hinaus noch unmittelbar von der Neigung bzw. dem Winkel $\theta$ an dem entsprechenden Ort ab.

Für jeden Puls und damit einen entsprechenden Ort auf der Oberfläche ist die Neigung bekannt, so daß diese Beziehung als Gleichung zur Bestimmung einer für den Puls zu verwendenden Fluence bzw. eines Spitzenwerts $F_0$ oder einer entsprechenden Pulsenergie aufgefaßt werden kann, von denen die Einzelpuls-Ablationsvolumina bzw. die Modifikationsfunktion K abhängt. Für jeden Puls wird nun diese Gleichung, beispielsweise unter Verwendung eines dem Fachmann bekannten Newton-Verfahrens, numerisch für das Verhältnis $F_0/F_{THR}$ gelöst und abgespeichert. Damit ergibt sich ein zu verwendendes Ablationsprogramm, das eine Folge von Zielorten, auf die Pulse des Laserstrahls 3 zu lenken sind, gemäß dem vorläufigen Ablationsprogramm und für jeden der Zielorte einen entsprechenden orts- bzw. koordinatenabhängigen Fluence-Spitzenwert $F_0$ bzw. eine entsprechende Pulsenergie enthält.

Durch entsprechende Ansteuerungen einer Hochspannungsversorgung des Eximerlasers 7 und damit der Aufladung von Kondensatoren, in denen jeweils die Energie für einen Puls gespeichert wird, entsprechend dem Ablationsprogramm und gleichzeitige Ansteuerung der Ablenkeinrichtung 8 kann nun in Schritt S36 die Ablation mit Laserpulsen mit einer orts- bzw. koordinatenabhängigen Pulsenergie und damit Fluence erfolgen.

Bei einem anderen Ausführungsbeispiel kann dabei die Ermittlung des Spitzenwertes der Fluence gegebenenfalls auch während der eigentlichen Ablation erfolgen.

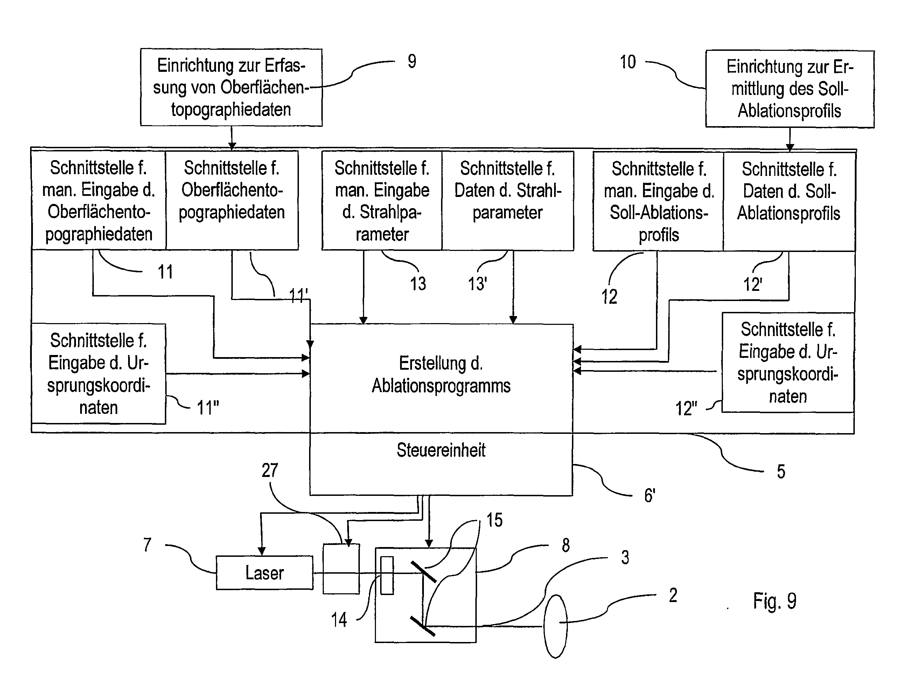

Eine Variante dieses Ausführungsbeispiels ist in Figur 9 gezeigt. Das hier schematisch dargestellte Instrument unterscheidet sich von dem Instrument des vorhergehenden Ausführungsbeispiels dadurch, daß im Strahlengang des Laserstrahls 3 zwischen dem Laser 7 und der Ablenkeinrichtung 8 ein Modulator 27, im Beispiel ein von der Steuereinrichtung 6' in seiner Transmission steuerbares Flüssigkristall-Element, aufweist. Darüber hinaus weist die Steuereinrichtung 6' gegenüber der Steuereinrichtung 6 einen Ausgang zur Ansteuerung des Modulators 27 auf und das in der Datenverarbeitungseinrichtung 5 abgearbeitete Computerprogramm ist zur Steuerung der Fluence nicht durch Ansteuerung des Lasers 7, sondern durch Änderung der Transmission des Modulators 27 ausgebildet.

Die Fluence des Lasers 7 wird nun so eingestellt, daß bei maximaler Transmission des Modulators 27 die maximal erforderliche Fluence zur Ablation gemäß dem Ablationsprogramm erreicht werden kann. Der Modulator 27 wird bei der Ablation entsprechend dem Ablationsprogramm so gesteuert, daß die auf die Oberfläche auftreffenden Pulse die gewünschte Energie bzw. Fluence besitzen.

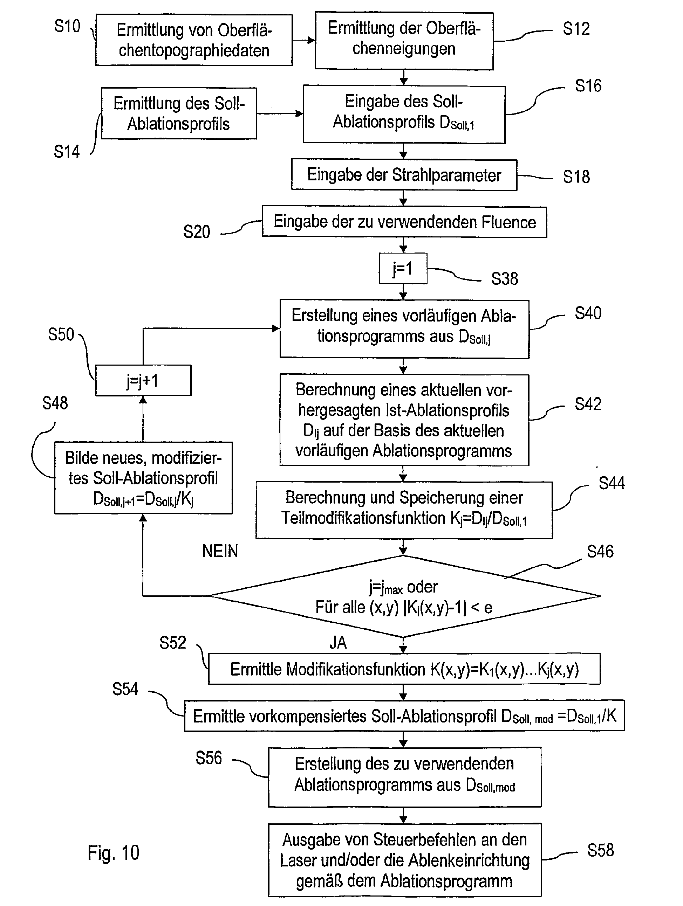

In Fig. 10 ist ein weiteres Ausführungsbeispiel für ein Verfahren zur Erstellung eines Ablationsprogramms dargestellt, daß mit einem Instrument ausgeführt werden kann, das sich von dem Instrument 1 nur in der Programmierung der Datenverarbeitungseinrichtung 5 unterscheidet.

Das Verfahren ist in den Schritten S10 bis S20 identisch mit dem Verfahren des ersten Ausführungsbeispiels, sodaß für diese Schritte die gleichen Bezugszeichen verwendet werden und die Erläuterungen entsprechend gelten. Die Erstellung des Ablationsprogramms erfolgt hier jedoch iterativ und unter Berücksichtigung der Form des Strahlprofils mit einer Ortsauflösung, die größer ist als der Strahlquerschnitt.

Ausgehend von einem auf der Basis des Soll-Ablationsprofils erstellten vorläufigen Ablationsprogramm wird mit hoher Ortsauflösung ein vorhergesagtes Ist-Ablationsprofil $D_I$ vorhergesagt. Die $N$ Einzelpulskoordinaten seien beschrieben durch die Koordinaten $(x_{Pi}, y_{Pi})$, i=1..N. Auf einem feinen Punktraster von $M \times M$ Punkten $(x_m, y_m)$, m=1, .., M, für das $x_m\text{-}x_{m\text{-}1} \ll w$ und ebenso $y_m\text{-}y_{m\text{-}1} \ll w$ gilt, wird der Beitrag zur Ablationstiefe von jedem der N Einzelpulsen mit einer Ortsauflösung besser als der Strahlprofildurchmesser berechnet:

$$D_I(x_m, y_m) = \sum_{\substack{i=1 \\ D_j > 0}}^{N} D_i(x_m, y_m) = \sum_{\substack{i=1 \\ D_j > 0}}^{N} \left( \mu \ln \frac{F_0 \cos\theta(x_m, y_m)}{F_{thr}} - \mu \frac{(x_{Pi} - x_m)^2 + (y_{Pi} - y_m)^2}{w^2} \right)$$

Es wird also an jeder Stelle des Abtragsprofils der korrekte lokale Neigungswinkel $\theta$ berücksichtigt und die Fluence um cos $\theta$ reduziert. Gleichzeitig wird die Form des Strahlprofils berücksichtigt. Dieses vorhergesagte Ablationsprofil wird nach weiteren Schritten zur Erstellung eines weiteren vorläufigen Ablationsprogramms verwendet. Genauer werden folgende Schritte durchgeführt.

In Schritt S38 wird zunächst ein Schleifenzähler j auf den Wert 1 für die erste Iterationsschleife gesetzt.

In Schritt S40 wird dann in der ersten Iterationsschleife j=1 aus dem Soll-Ablationsprofil $D_{Soll,1}$ und in folgenden Iterationsschleifen j= 2, 3, ... aus modifizierten Soll-Ablationsprofilen $D_{Soll,j}$ aus der vorhergehenden Iterationsschleife ein vorläufiges Ablationsprogramm in der aktuellen Iterationsschleife j mittels eines einfachen Erstellungsverfahrens erstellt, wobei bei der Erstellung des Ablationsprogramms nicht zugleich die Form des Strahlprofils und die Neigung der Oberfläche berücksichtigt werden. Beispielsweise kann dazu das oben genannte Verfahren DE 197 27 573 C1 verwendet werden.

In Schritt S42 wird dann unter Verwendung des "Blow-Off"-Modells ein Ist-Ablationsprofil $D_{Ij}$ auf der Basis des vorläufigen Ablationsprogramms wie zuvor beschrieben vorhergesagt. Dazu wird ein Gitter von Stützpunkten in der x-y-Ebene verwendet, das wesentlich feiner ist, als die Ausdehnung des Laserstrahls, so daß dessen Form aufgelöst werden kann. Für jeden Puls wird dann an einem entsprechendem Stützpunkt entsprechend dem "Blow-Off"-Model und entsprechend der dort tatsächlich wirksamen Fluence, das heißt der Fluence des Laserstrahls korrigiert um die Oberflächenneigung, eine Ablationstiefe berechnet, wobei in dem Fall, daß Teile mehrerer Pulse auf den gleichen Ort fallen, diese Ablationstiefen summiert werden.

In dem folgenden Schritt S44 werden dann an den Stützstellen des vorgegebenen Soll-Ablationsprofils Werte einer Teilmodifikationsfunktion $K_j = D_{Ij}/D_{Soll,1}$ ermittelt, die wiedergibt, wie weit das vorhergesagte Ist-Ablationsprofil $D_{Ij}$ von dem vorgegebenen Soll-Ablationsprofil $D_{Soll,1}$ abweicht. Die Werte werden dann zwischengespeichert. Die durch die Werte gegebene Funktion wird nach der ersten Berechnung nochmals rechnerisch unter Verwendung eines Tiefpaßfilters geglättet.

In dem folgendem Schritt S46 wird dann überprüft, ob die in Schritt S44 ermittelten Werte der Modifikationsfunktion und damit das Verhältnis der Ablationsprofile für alle betrachteten Orte bzw. Stützstellen (x,y) um weniger als ein vorgegebener Fehler e, beispielsweise 0,05, von 1 abweicht.

Tritt eine solche Abweichung auf, wird eine weitere Iterationsschleife durchlaufen, indem zunächst in einem Schritt S48 ein neues, modifiziertes Soll-Ablationsprofil $D_{Soll,j+1} = D_{Soll,j}/K_j$ gebildet wird. Dieses dient dann in dem folgendem Schritt S40 als Grundlage zur Erstellung eines neuen vorläufigen Ablationsprogramms.

In Schritt S50 wird der Schleifenzähler j um 1 erhöht. Danach wird die nächste Iterationsschleife mit Schritt S40 begonnen.

Ist die Teilmodifikationsfunktion $K_j$ hinreichend genau konstant 1, so wird dagegen nach Schritt S46 das Verfahren mit Schritt S52 fortgesetzt, in dem für jeden Ort bzw. jede Stützstelle (x,y) aus allen in den Iterationsschleifen ermittelten Werten der Teilmodifikationsfunktionen $K_j$ durch Produktbildung der Wert einer Modifikationsfunktion K(x,y) $=K_1(x,y) ... K_j(x,y)$ ermittelt wird.

In Schritt S54 wird aus dem vorgegebenen Soll-Ablationsprofil $D_{Soll,1}$ durch Division der durch $D_{Soll,1}$ vorgegebenen Soll-Ablationstiefe an allen Stützstellen (x,y) durch den entsprechenden Wert der Modifikationsfunktion K für den Ort ein vorkompensiertes Soll-Ablationsprofil $D_{Soll, mod}$ ermittelt.

In Schritt S56 wird aus dem vorkompensierten Soll-Ablationsprofil $D_{Soll, mod}$ wie in Schritt S26 in dem ersten Ausführungsbeispiel das zu verwendende Ablationsprogramm mit einem einfachen Erstellungsverfahren erstellt. Durch die Vorkompensation, d.h. die Modifikation mit der Modifikationsfunktion K, ist das vorgegebene Soll-Ablationsprofil derart verändert, daß Fehler, die bei der Verwendung des einfachen Erstellungsverfahrens zur Erstellung eines Ablationsprogramms unmittelbar aus dem vorgegebenen Soll-Ablationsprofil durch Vernachlässigung der Einflüsse der Form des Strahlprofils und der Oberflächenneigung entstehen würden, schon vor Erstellung kompensiert werden, d.h. das erstellte, zu verwendende Ablationsprogramm führt bei Ablation entsprechend dem Programm in sehr guter Näherung zu dem gewünschten Soll-Ablationsprofil.

In Schritt S58 können dann entsprechende Steuerbefehle an den Laser 7 und/oder die Ablenkeinrichtung 8 gemäß dem Ablationsprogramm ausgegeben werden, um das gewünschte Soll-Ablationsprofil von der Oberfläche abzutragen.

In Figur 11a ist eine horizontale Schnittkurve durch das Zentrum des sphärischen Ablationsprofils auf einem Gebiet mit 8 mm Durchmesser dargestellt, das von einer Kugel mit Radius 7,86 mm ablatiert werden soll. Die durchgezogene Linie gibt dabei das vorhergesagte Ist-Profil $D_E$ wieder, das sich bei einer gegenüber dem Laserstrahl 3 nicht geneigten Oberfläche ergeben würde, die gestrichelte Linie dagegen das Ist-Ablationsprofil $D_{I,1}$ bei Berücksichtigung der Neigung und der Form des Strahlprofils. Figur 11b zeigt die Differenz zwischen diesen beiden Kurven.

Das Ergebnis des Verfahrens des letzten Ausführungsbeispiels ist in Figur 5 den Ergebnissen der beiden zuvor beschrieben Verfahren gegenübergestellt. Dabei wurde die Modifikationsfunktion nach vollständigem Durchlaufen einer ersten Iteration durch schwarze Quadrate dargestellt. Man erkennt, daß in größerer Entfernung von dem Zentrum der Kugelkappe, das heißt bei größeren Neigungswinkeln, die Abweichungen von dem Modifikationsfaktor, der die Form des Strahlprofils nicht berücksichtigt, nochmals deutlich größer sind, so daß für dieses Verfahren bessere Ablationsergebnisse zu erwarten sind.

Es zeigt sich, daß eine hinreichend gute Vorkompensation von Fehlern bereits erreicht wird, wenn nur eine Iteration durchgeführt wird, d.h. j=1 gesetzt wird. Bei einer modifizierten Ausführungsform werden daher die Schritte S38 und S46 bis S52 fortgelassen, wobei in Schritt S44 unmittelbar die Werte der Modifikationsfunktion K ermittelt werden, die dann der ersten Teilmodifikationsfunktion entspricht. Die Erstellung des Ablationsprogramms erfolgt dann besonders schnell. Die Datenverarbeitungseinrichtung 5 und damit die Erstellungsvorrichtung ist entsprechend zur Durchführung des Verfahrens programmiert.

Bei den vorhergehenden Ausführungsbeispielen wurde davon ausgegangen, daß die Neigungsdaten und die Daten für das Soll-Ablationsprofil in dem gleichen Koordinatensystem gegeben sind. Dies ist jedoch nicht notwendig, vielmehr können bei einem modifizierten Verfahren zu Beginn Daten eingegeben werden, die die Relativlage der Ursprünge der entsprechenden Koordinatensysteme wiedergeben, so daß nach dem Einlesen der entsprechenden Daten durch eine einfache Verschiebung die Neigungsdaten und das Soll-Ablationsprofil in ein gemeinsames Koordinatensystem überführt werden können.

Bei einer weiteren Ausführungsform des erfindungsgemäßen Verfahrens wird ein Strahl mit einer besonders günstigen Strahlprofilform an der zu ablatierenden Oberfläche verwendet. Das Strahlprofil ist so geformt, daß alle Bereiche der zweidimensionalen Fluence- bzw. Energieverteilung oberhalb des Schwellwertes $F_{thr}$ für die Ablation liegen. Ein Beispiel für ein solches Strahlprofil ist in Fig. 12 gezeigt. Das Strahlprofil geht aus einem Gaußprofil hervor, bei dem die Flanken entsprechend dem genannten Kriterium abgeschnitten sind.

Bei Verwendung eines solchen Strahlprofils wirkt der gesamte Strahlquerschnitt an der Oberfläche ablatierend, so daß eine thermische Belastung benachbarter Bereiche minimiert werden kann.

Die Formung eines solchen Strahlprofils kann durch eine Blende bewirkt werden, die die gewünschten Teile eines Ausgangsstrahlprofils abschneidet. In einem solchen Fall wird der auf die Blende fallende Teil der Laserenergie nicht genutzt, was in energiekritischen Anwendungen nachteilig ist. Der Effekt der geringeren thermischen Belastung der zu bearbeitenden Oberfläche ist hier allerdings auch gegeben.

Vorzugsweise wird zur Strahlformung ein mikrooptisches Element verwendet, das auf diffraktive oder refraktive Weise direkt die gewünschte Intensitätsverteilung erzeugt. In WO99/20429 A1 und DE 196 23 749 A1 sind entsprechende mikrooptische Elemente zur Erzeugung einer gaußförmigen Intensitätsverteilung beschrieben. Beispielsweise kann ein statisches refraktives Mikrolinsenarray verwendet werden, bei dem durch die Größenverteilung der Mikrolinsen eine vorgegebene Winkelverteilung der Strahlung festgelegt wird. Das mikrooptische Element ordnet den einfallenden Laserstrahl mit hoher Effektivität so um, daß das resultierende, umgeformte Strahlprofil der gewünschten Intensitätsverteilung entspricht, jedoch dabei keine Energie- bzw. Leistungsanteile verloren gehen.

Damit kann nicht nur die thermische Belastung der zu bearbeitenden Oberfläche reduziert, sondern auch erheblich Energie gespart werden, was besonders bei Anwendungen, bei denen die Strahlenergie des Lasers kritisch ist, vorteilhaft ist. Bei einem abgeschnittenen ("truncated") Gaußprofil, dessen Spitzenfluence das vierfache der Schwellfluence beträgt und dessen Fluence-Anteile unterhalb der Schwellfluence wegfallen, können gegenüber dem vollständigen Gaußprofil etwa 25 % der Energie bei gleicher Ablationswirkung eingespart werden. Dies bedeutet gleichzeitig, daß die thermische Erwärmung der zu bearbeitenden Oberfläche bzw. im Fall einer Behandlung des Auges, des Gewebes, nicht in Wärme umgesetzt wird, was besonders im Bereich der Hornhautchirurgie ein großer Vorteil sein kann. Solche Strahlprofile können für beliebige Spot-Scanning-Ablationsverfahren und insbesondere auch die erfindungsgemäßen Ablationsverfahren eingesetzt werden.

Ein laserchirurgisches Instrument 101 zur Behandlung eines Auges 2 eines Patienten nach einer ersten bevorzugten Ausführungsform des zweiten Aspekts der Erfindung wird ähnlich wie das Instrument 1 zur Ausführung einer refraktiven Korrektur des Auges angewendet und ersetzt insofern das Instrument 1 in Fig. 1. Das Instrument 101 gibt also, wie in Fig. 1, einen gepulsten Laserstrahl 3 auf das Auge 1 des Patienten ab, dessen Kopf in einem Kopfhalter 4, der fest mit dem Instrument 101 verbunden ist, fixiert ist.

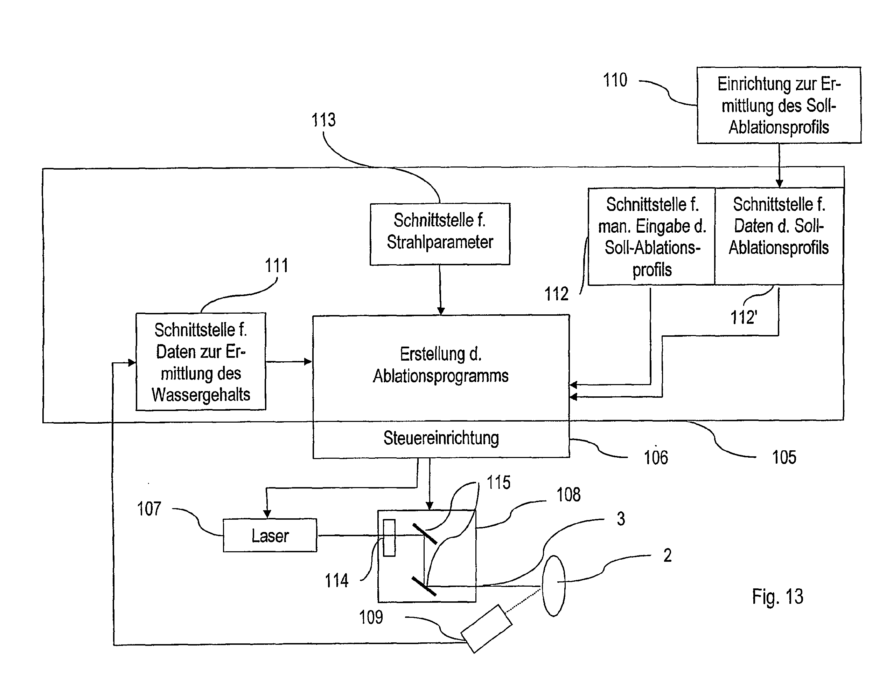

Fig. 13 zeigt schematisch genauer den Aufbau des Instruments, das eine Laserablationsvorrichtung darstellt. Das Instrument umfaßt eine Datenverarbeitungseinrichtung 105 mit einer integrierten Steuereinrichtung 106, einen von der Steuereinrichtung 106 angesteuerten Laser 107 und eine ebenfalls von der Steuereinrichtung angesteuerte Ablenkeinrichtung 108, mittels derer der von dem Laser 107 abgegebene gepulste Laserstrahl 103 entsprechend einem vorgegebenen Ablationsprogramm auf Zielorte auf der Hornhaut des Auges 2 gelenkt und fokussiert werden kann.

Das Instrument verfügt weiter über eine Einrichtung 109 zur Erfassung von Wassergehaltsmeßdaten, aus denen der Wassergehalt eines zu behandelnden Bereichs des Auges 2, genauer der Hornhaut bzw. Cornea, ermittelbar ist, und eine Einrichtung 110 zur Ermittlung eines Soll-Ablationsprofils. Beide Einrichtungen sind mit der Datenverarbeitungseinrichtung 105 verbunden. Die Datenverarbeitungseinrichtung 105, die Steuereinrichtung 106, die Einrichtung 109 zur Erfassung von Wassergehaltsmeßdaten und die Einrichtung 110 zur Ermittlung des Soll-Ablationsprofils bilden eine Vorrichtung zur Erstellung eines Ablationsprogramms nach einer ersten bevorzugten Ausführungsform des zweites Aspekts der Erfindung.

Die Datenverarbeitungseinrichtung 105 mit der integrierten Steuereinrichtung 106 dient zur Erstellung eines Ablationsprogramms unter Verwendung von Daten, aus denen der Wassergehalt in dem Material des zu behandelnden Bereichs ermittelbar ist, von Daten in Bezug auf Eigenschaften des Laserstrahls 3 und von Daten in Bezug auf das zu ablatierende Soll-Ablationsprofil. Die Datenverarbeitungseinrichtung 105 verfügt hierzu über einen Prozessor und einen Speicher zur Abspeicherung von Daten, in dem insbesondere auch ein Computerprogramm mit Programmcode abgelegt ist, mittels dessen bei Ausführung des Programms auf dem Prozessor das Ablationsprogramm erstellt und unter Verwendung der integrierten Steuereinrichtung 106 Steuersignale zur Ansteuerung des Lasers 107 und/oder der Ablenkeinrichtung 108 gebildet und an den Laser 107 bzw. die Ablenkeinrichtung 108 abgegeben werden, um die eigentliche Ablation durchzuführen. Der Speicher und der Prozessor sind in der Blockdarstellung teilweise durch den Block "Erstellung des Ablationsprogramms" dargestellt.

Die Datenverarbeitungseinrichtung 105 besitzt hierzu Schnittstellen zur Eingabe von Daten, nämlich eine Schnittstelle 111 für das Einlesen von Wassergehaltsmeßdaten von der Einrichtung 109 zur Erfassung von Wassergehaltsmeßdaten, eine Schnittstelle 112 zur manuellen Eingabe des Soll-Ablationsprofils und eine Schnittstelle 112' zum Einlesen von Daten in Bezug auf das Soll-Ablationsprofil von der Einrichtung 110 zur Ermittlung des Soll-Ablationsprofils. Darüber hinaus ist eine Schnittstelle 113 zur Eingabe von Strahlparametern für den Laserstrahl 3 vorgesehen. In diesem Ausführungsbeispiel ist die Schnittstelle 113 als Schnittstelle zur manuellen Eingabe ausgebildet.

Bei den Schnittstellen für eine manuelle Eingabe kann es sich in physischer Hinsicht um eine einzige Schnittstelle handeln, an die, in den Figuren nicht gezeigt, eine Tastatur und ein Monitor, auf dem ein Eingabeprompt darstellbar ist, wenn entsprechende Daten eingelesen werden sollen, angeschlossen sind. Die Schnittstellen umfassen weiter entsprechende Module des Computerprogramms zum Einlesen der Daten von der Tastatur.

Die anderen Schnittstellen 111' und 112' sind konventionelle Schnittstellen für Datenströme, die neben entsprechenden elektronischen Modulen auch Softwaremodule umfassen.

Die Steuereinrichtung 106 ist in die Datenverarbeitungseinrichtung 105 integriert und verfügt weiter über in den Figuren nicht gezeigte Schnittstellen zur Abgabe von Steuersignalen zur Ansteuerung des Lasers 107 und der Ablenkeinrichtung 108. Solche Steuereinrichtungen sind grundsätzlich bekannt und brauchen daher nicht näher erläutert zu werden.

Der Laser 107 ist mit der Steuereinrichtung 106 verbunden und gibt in Abhängigkeit von dem Ablationsprogramm einen gepulsten Laserstrahl mit vorgegebenen Pulsenergien ab. Beispielsweise kann ein Excimerlaser mit einer Wellenlänge im Wellenlängenbereich von 193 nm verwendet werden.

Die Ablenkeinrichtung 108 ist ebenfalls über eine Datenverbindung mit der Steuereinrichtung 106 verbunden und lenkt entsprechend Steuersignalen von der Steuereinrichtung 106 gemäß dem abzuarbeitenden Ablationsprogramm den von dem Laser 107 abgegebenen, gepulsten Laserstrahl 3 auf vorgegebene Zielorte auf der Oberfläche des Auges 2. Hierzu verfügt die Ablenkeinrichtung 108 über eine Fokussierungseinrichtung 114 zur Fokussierung des Laserstrahls entlang seiner Ausbreitungsrichtung und zur Ablenkung quer zu dem Laserstrahl über zwei um jeweils orthogonal zueinander verlaufende Achsen dreh- bzw. schwenkbare Spiegel 115, die im Strahlengang nach der Fokussiereinrichtung 114 angeordnet sind.

Sowohl bei dem Laser 107 als auch bei der Ablenkeinrichtung 108 kann es sich um konventionelle, bekannte Einrichtungen eines laserchirurgischen Instruments handeln.

Zur Angabe des Wassergehalts und des Soll-Ablationsprofils werden zwei parallel zueinander ausgerichtete kartesische Koordinatensysteme verwendet, deren x-y-Ebenen zusammenfallen. Die z-Achse ist dabei möglichst in guter Näherung parallel zu der optischen Achse des Auges ausgerichtet. Im Beispiel wird zur einfacheren Darstellung angenommen, daß die Ursprünge der Koordinatensysteme zusammenfallen, so daß die Koordinatensysteme zusammenfallen und im folgenden nicht mehr unterschieden werden. Fallen die Koordinatenursprünge nicht zusammen, kann die Relativlage nach dem Einlesen der zum Wassergehalt oder zum Soll-Ablationsprofil manuell eingegeben werden, woraufhin die Daten in ein gemeinsames Koordinatensystem überführt werden können. Zur Eingabe der Relativlage kann dann eine entsprechende Schnittstelle vorgesehen sein. In Fig. 3 ist die Lage des Koordinatensystems für einen sphärischen Körper bzw. eine Kugel mit einer Oberfläche 2' als vereinfachtes Modell für das Auge 2 dargestellt. Die Festlegung der z-Achsen erfolgt bei der Ausrichtung des Auges 2 relativ zu dem Instrument 101, wozu beispielsweise ein in den Figuren nicht gezeigtes Fixierlicht benutzt werden kann.

Die Einrichtung 109 zur Erfassung von Wassergehaltsmeßdaten umfaßt im Beispiel eine Einrichtung zur konfokalen Raman-Spektroskopie der Hornhaut des Auges, wie sie beispielsweise in "Assessment of Transient Changes in Corneal Hydration Using Confocal Raman Spectroscopy", Brian T. Fischer et al, Cornea 22 (4), Seiten 363-370, 2003, beschrieben ist. Der zur Behandlung vorgesehene Bereich der Cornea des Auges 2 wird dabei mit einem geeigneten Laserstrahl abgetastet, der sowohl in der Tiefe als auch lateral zum Laserstrahl fokussiert ist. Von dem Auge 2 ausgehende Raman-Strahlung wird dann konfokal detektiert, so daß für verschiedene Orte in dem zu ablatierenden Volumen die Intensität der Raman-Strahlung erfaßt werden kann. Die die Intensität wiedergebenden Daten, d.h. die Wassergehaltsmeßdaten, werden für jeden gemessenen Ort über die Schnittstelle 111 in die Datenverarbeitungseinrichtung 105 eingelesen. Das Computerprogramm in der Datenverarbeitungseinrichtung 105 verfügt über Programmcode, mittels dessen die Daten in den Wassergehalt an dem jeweiligen Ort beschreibende Daten umgerechnet werden können.

Die Einrichtung 110 zur Ermittlung des Soll-Ablationsprofils umfaßt im vorliegenden Beispiel einen Wellenfrontanalysator vom Hartmann-Shack-Typ sowie gegebenenfalls Einrichtungen zur Ermittlung der Brechkraft des Auges 2, aus dem bzw. denen nach bekannten Verfahren ein Soll-Ablationsprofil $D_{Soll}$ für den zu behandelnden Bereich des Auges 2 ermittelt werden kann. Das Soll-Ablationsprofil wird dabei so bestimmt, daß eine möglichst weitgehende Korrektur von Abbildungsfehlern durch das Auge 2 durch die noch durchzuführende Ablation erreicht werden kann. Ein Beispiel für das Soll-Ablationsprofil ist Fig. 3 entnehmbar. Es ist durch die Abstände in z-Richtung zwischen der anfänglichen Oberfläche, der Kugelkappe 2', und einer gewünschten Oberfläche 2", die durch eine gestrichelte Linie angedeutet ist, als Funktion des Ortes in der x-y-Ebene gegeben. Ein zu ablatierender Bereich wird durch das Volumen zwischen der Oberfläche 2' vor der Ablation und der gewünschten Oberfläche 2" gebildet, die durch das Soll-Ablationsprofil gebildet wird.

Zur Ermittlung des Soll-Ablationsprofils kann die Einrichtung 110 über einen entsprechenden Prozessor verfügen, der die Daten in Bezug auf die Brechkraft des Auges 2 und die Wellenfrontdaten auswertet, um daraus im vorliegenden Beispiel wiederum in Form von Soll-Ablationstiefen für Stützstellen in Form von Punkten eines Punktegitters in der x-y Ebene des entsprechenden Koordinatensystems, die mit der x-y Ebene des Koordinatensystems zur Angabe der zusammenfällt, das Soll-Ablationsprofil zu ermitteln. Soweit die Koordinatenursprünge nicht übereinstimmen, kann die Lage eines der Bezugspunkte bzw. Koordinatenursprünge in dem jeweils anderen Koordinatensystem angegeben werden, so daß im späteren Verfahrensverlauf die Daten durch eine einfache Verschiebung in ein gleiches Koordinatensystem transformiert werden können. Dies kann sich dann als günstig erweisen, wenn das Zentrum des Soll-Ablationsprofils, das heißt ein Punkt, relativ zu dem das Soll-Ablationsprofil näherungsweise symmetrisch ist, von dem Zentrum der Oberfläche des Auges 2, das heißt einem Punkt, relativ zu dem die Oberfläche des Auges näherungsweise symmetrisch ist, abweicht. Ein Verfahren zur Erstellung eines Soll-Ablationsprofils ist beispielsweise in der WO 01/08075 A1 beschrieben, deren Inhalt insoweit hiermit durch Bezugnahme in die Beschreibung aufgenommen wird.

Das Verfahren zur Erstellung eines Ablationsprogramms des ersten Ausführungsbeispiels für den zweiten Aspekt der Erfindung beruht auf folgenden Überlegungen.

Um das Soll-Ablationsprofil $D_{Soll}$ von dem Auge 2 zu ablatieren, werden entsprechend einem erstellten Ablationsprogramm auf vorgegebene Zielorte auf dem Auge 2 Laserpulse einer vorgegeben Pulsenergie abgegeben, die jeweils einzeln zu einem Abtrag von Material führen. Die Tiefe des Abtrags durch einen Puls kann durch verschiedene Modelle beschrieben werden. Im vorliegenden Beispiel wird das sogenannte "Blow-Off"-Modell verwendet, das beispielsweise in "Refraktive Chirurgie der Hornhaut", Herausgeber Theo Seiler, 1. Auflage, ENKE im Georg Thieme Verlag, Stuttgart / New York, 2000 (ISBN 3-13-118071-4), Kapitel 6.1, S.150 oder in R. Srinivasan: "Ablation of Polymers and Biological Tissue by Ultraviolet Lasers", Science vol. 234, p.559-565, 31. Oct. 1986 beschrieben ist.

Danach wird an einem Ort mit den Koordinaten (x, y) durch eine an diesem Ort auftreffende Laserstrahlung mit einer wirksamen Fluence F(x,y), das heißt Energie pro Fläche, Material bis in eine Tiefe $D_P$ gemäß folgender Formel abgetragen:

$$D_P(x,y) = \begin{cases} \mu \cdot \ln \dfrac{F(x,y)}{F_{thr}} & ,falls\ F(x,y) > F_{thr} \\ 0 & ,sonst \end{cases} \qquad (1).$$

Dabei bezeichnen $\mu$ einen materialabhängigen Ablationskoeffizienten und $F_{thr}$ einen ebenfalls materialabhängigen Ablationsschwellwert für die Fluence, unterhalb dessen Laserstrahlung keinen Abtrag von Material von dem Auge 2 mehr bewirkt.

Bei dem im Folgenden verwendeten einfachen Erstellungsverfahren für ein Ablationsprogramm wird davon ausgegangen, daß das Soll-Ablationsprofil in Einzelpuls-Ablationsvolumina, die bei Auftreffen eines Pulses entstehen, zerlegt werden kann. Die Erstellung des Ablationsprogramms beinhaltet dann die Bestimmung der Zielorte (x,y), auf die Pulse einer vorgegebenen Pulsenergie gelenkt werden müssen.

Zur Erstellung eines Ablationsprogramms wird davon ausgegangen, daß ein Laserpuls ein Einzelpuls- bzw. Spot-Ablationsvolumen $V_{Puls}$ abträgt, das sich als Integral der Ablationstiefe $D_P$ über die gesamte Pulsfläche ergibt:

$$V_{Puls} = \iint\limits_{Spot} D_P(x,y)dxdy = \mu \iint\limits_{Spot} \ln \frac{F(x,y)}{F_{thr}} dxdy \qquad (2).$$

Das Integral erstreckt sich dabei über die mit "Spot" bezeichnete Fläche, in der $D_P > 0$ ist.

Im folgenden wird davon ausgegangen, daß die Fläche, in der $D_P > 0$ ist, konstant ist und einen Flächeninhalt $d^2$ hat. Es ist dann $V_{Puls} = d^2 D_P$.

Bei einfachen Erstellungsverfahren, bei denen der Wassergehalt des zu ablatierenden Materials und insbesondere Variationen in dem Wassergehalt des zu ablatierenden Materials nicht berücksichtigt werden, ergibt sich für die Ablationstiefe $D_{P,0}$ für einen Puls ohne Berücksichtigung des Wassergehalts:

$$D_{P,0} = \mu_0 \cdot \ln\left(\frac{F_0}{F_{thr}}\right). \qquad (3)$$

Dabei bezeichnet $\mu_0$ eine empirisch bestimmte, als konstant angenommene Ablationsrate. Die empirische Bestimmung kann dabei durch Untersuchung der Ablation an einer größeren Anzahl von verschiedenen Augen, bzw. deren Cornea, ermittelt werden.

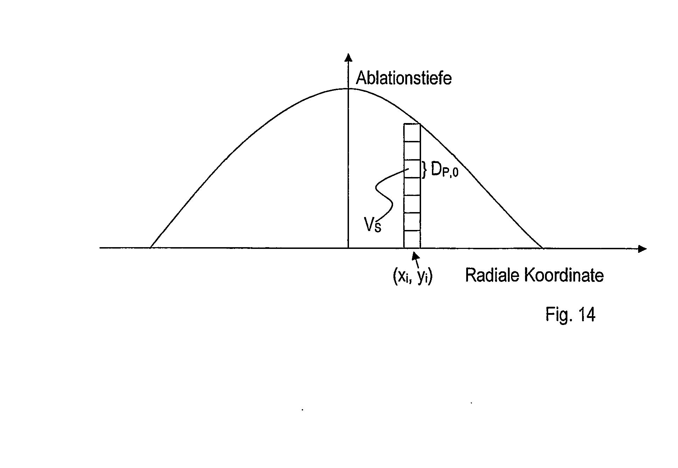

Bei Verwendung eines einfachen Verfahrens zur Erstellung eines Ablationsprogramms wird davon ausgegangen, daß jeder auf denselben Ort abgegebene Puls das gleiche Einzelpulsvolumen bzw. die gleiche Einzelpulsablationstiefe $D_{P,0}$ ablatiert, wobei sich die Volumina addieren. Dies ist in Fig. 14a veranschaulicht. In dieser Figur ist schematisch beispielhaft ein Soll-Ablationsprofil entlang eines Durchmessers durch das Auge 2, etwa entlang der x-Achse dargestellt. Die Einzelpuls-Ablationsvolumina $V_S$ werden sukzessive ablatiert und ergeben die gewünschte Ablationstiefe, wobei jedes Einzelpuls-Ablationsvolumen $V_S$ einer Einzelpuls-Ablationstiefe $D_{P,0}$ entspricht. Werden die Koordinaten der betrachteten Zielorte mit $(x_j,\ y_j)$, i=1, ..., G, G ein natürliche Zahl, bezeichnet, ergibt sich die Anzahl $N_{i,0}$ der auf einen Zielort $(x_i,y_i)$ abzugebenden Pulse:

$$N_{I,0} = \frac{D_{Soll}(x_i, y_i)}{D_{P,0}}.$$ (4)

Tatsächlich hängt die Ablationsrate $\mu$ jedoch von dem Wassergehalt H in dem zu ablatierenden Material ab. In einem einfachen, auf dem Blow-off-Modell basierenden Modell ergibt sich im Beispiel die Einzelpuls-Ablationstiefe $D_{P,W}$ für einen Puls unter Berücksichtigung des Wassergehalts des Materials zu

$$D_{P,W} = \mu(H) \cdot \ln\left(\frac{F_0}{F_{thr}}\right),$$ (5)

wobei die vom Wassergehalt H abhängige Ablationsrate $\mu(H)$ beispielsweise als proportional zu dem Wassergehalt H mit einer Proportionalitätskonstanten k angenommen werden kann:

$$\mu(H) = k \cdot H.$$ (6)

Der Wert der Proportionalitätskonstante k ist dabei so gewählt, daß für einen bei den oben erwähnten Reihen-untersuchungen ermittelten mittleren Wassergehalt $H_m$ der Cornea $k \cdot H_m = \mu_0$ gilt.

Tatsächlich kann sich jedoch der Wassergehalt des zu ablatierenden Materials bzw. Gewebes der Cornea ändern in Abhängigkeit von den Umgebungsbedingungen wie Luftfeuchte, Luftströmung über dem Auge, Lufttemperatur und von den Ablationsbedingungen, beispielsweise der Ablationsrate, der Anzahl der auf einen Zielort $(x_i, y_i)$ oder in dessen Nachbarschaft bereits abgegebenen Pulse und der Fluence der Pulse. In diesem Ausführungsbeispiel wird von folgendem Modell für den Wassergehalt H von zu ablatierendem Material über einem Zielort $(x_i, y_i$, auf den bereits $n(x_i, y_i)$ Pulse abgegeben wurden, ausgegangen:

$$H(x_i, y_i; n(x_i, y_i)) = a(x_i, y_i) + b \cdot \log(n(x_i, y_i)).$$ (7)

Der Parameter a beschreibt dabei den Wassergehalt an dem Zielort $(x_i, y_i)$ vor Beginn der Ablation. Dieser Wassergehalt kann unter anderem von der Art der Behandlung, beispielsweise photorefraktive Keratektomie, LASIK, LASIK mit photodisruptiver Bildung eines abklappbaren Corneadeckels oder LASEK, den Eigenschaften des individuellen Auges und der Umgebungsbedingungen vor der Ablation abhängen. Der in diesem Ausführungsbeispiel als konstant angenommene Parameter b beschreibt die Änderung des Wassergehalts durch Abgabe von Pulsen auf den Ort $(x_i, y_i)$. Insbesondere der Parameter b kann empirisch durch Reihenuntersuchungen gewonnen werden.

Bei Abgabe von $N_{W,i}$ Pulsen auf den gleichen Zielort $(x_i, y_i)$ summieren sich die Einzelpuls-Ablationstiefen, die nun durch Abhängigkeit von dem Wassergehalt H nicht mehr konstant sind, zu einer Gesamttiefe. Die Anzahl der $N_{W,I}$ kann so bestimmt werden, daß gerade die Soll-Ablationstiefe $D_{Soll}(x_i, y_i)$ an dem Ort $(x_i, y_i)$ erreicht wird:

$$D_{Soll}(x_i, y_i) = \sum_{1}^{N_{W,I}} D_{P,W} \approx \int_{0}^{N_{W,I}} D_{P,W}(u)du.$$ (8)

Wie in Gleichung (8) dargestellt, kann bei Abgabe einer großen Anzahl von Pulsen auf den gleichen Ort die Summe näherungsweise durch ein Integral ersetzt werden, um die Summe näherungsweise, aber schneller berechnen zu können.

Da die Ablationsrate mit zunehmender Anzahl von Pulsen zunimmt, ändert sich die tatsächlich erzielte Ablationstiefe bei Abgabe von $N_{W,i}$ Pulsen auf den Ort $(x_i, y_I)$ mit jedem Puls und unterscheidet sich von dem $N_{i,0}$-fachen der Einzelpuls-Ablationstiefe $D_{P,0}$ in dem einfachen Modell, bei dem der Wassergehalt nicht berücksichtigt wird.

Um zur Erstellung des Ablationsprogramms ein einfaches, beispielsweise bekanntes Erstellungsverfahren, bei dem der Wassergehalt nicht berücksichtigt wird, einsetzen zu können, wird daher eine Modifikationsfunktion M definiert, die zur Berechung eines vorkompensiertes bzw. modifizierten Soll-Ablationsprofils $D_{Mod}$ aus dem vorgegebenen Soll-Ablationsprofil $D_{Soll}$ dient:

$$D_{Mod}(x_i, y_i) = M(D_{Soll}(x_i, y_i))D_{Soll}(x_i, y_i) \qquad (9)$$

mit

$$M(D_{Soll}(x_i, y_i)) = \frac{N_{W,i}}{N_{i,0}} = \frac{N_{W,i}D_{P,0}}{D_{Soll}(x_i, y_i)} . \qquad (10)$$

Wie in den Formeln (9) und (10) ausgedrückt, hängt die Modifikationsfunktion von der Soll-Ablationstiefe $D_{Soll}$ $(x_i, y_i)$, dem Parameter a und damit dem Wassergehalt des Materials vor der Ablation sowie dem Parameter b und damit der Änderung des Wassergehalts durch die Ablation ab. Darüber hinaus besteht eine Abhängigkeit von dem bei dem einfachen Erstellungsverfahren verwendeten Modell für $D_{P,0}$.

Insbesondere kann die Ablationswirkung eines Einzelpulses mit zunehmender Anzahl der auf den gleichen Ort abgegebenen Pulse zunehmen, so daß bei einer großen Soll-Ablationstiefe, die eine größere Anzahl von Einzelpulsen auf den gleichen Ort erfordert, weniger Einzelpulse zu verwenden sind, als gemäß der Formel (4) zu erwarten ist.

Wird nun ein einfaches Erstellungsverfahren zur Erstellung des Ablationsprogramms aus dem vorkompensierten Soll-Ablationsprofil $D_{Mod}$ verwendet, kompensiert der in $D_{Mod}$ enthaltene Faktor M gerade die Fehler, die dadurch entstehen, daß der Wassergehalt in Formel (3) nicht berücksichtigt wird, so daß bei Ablation mit einem mittels des einfachen Erstellungsverfahrens aus dem vorkompensierten Soll-Ablationsprofil erstellten Ablationsprogramm in guter Näherung das vorgegebene Soll-Ablationsprofil erzielt wird.

Zur Ermittlung der Orte, auf die Laserpulse abzugeben sind, können verschiedene bekannte einfache Erstellungsverfahren verwendet werden, beispielsweise die in DE 19727573 C1 und EP 1060710 A2 beschriebenen Verfahren. In DE 19727573 C1 erfolgt die Ablation schichtweise, das heißt es werden Auftrefforte für Pulse schichtweise ermittelt, wobei sich das gewünschte Profil dann durch Überlagerung dieser Schichten ergibt. Bei dem Verfahren in EP 1060710 A2 wird der Spotabstand quasi- kontinuierlich variiert, um die gewünschte Ablationstiefe zu erreichen.

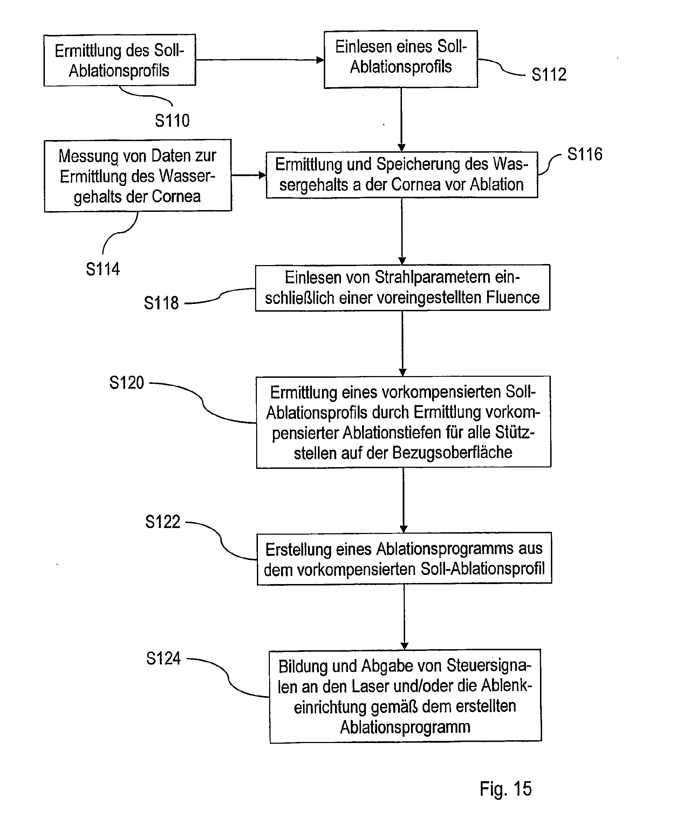

Zur Ermittlung des Ablationsprogramms werden die in dem Ablaufdiagramm in Fig. 15 skizzierten Verfahrensschritte durchgeführt, wozu das in der Datenverarbeitungseinrichtung ausgeführte Computerprogramm entsprechenden Programmcode aufweist.

Zunächst wird in Schritt S110 das Soll-Ablationsprofil $D_{Soll}$ unter Verwendung der Einrichtung 110 zur Ermittlung des Soll-Ablationsprofils ermittelt. Diese Ermittlung wird hier von einem Benutzer ausgelöst. In einem anderen Ausführungsbeispiel gibt die Datenverarbeitungseinrichtung 105 über eine in den Figuren nicht gezeigte Steuerschnittstelle ein entsprechendes Steuersignal an die Einrichtung 110 zur Ermittlung des Soll-Ablationsprofils aus, um die Ermittlung automatisch zu starten. Zur Ermittlung werden aus Wellenfrontdaten Abbildungsfehler ermittelt. Mit bekannten Verfahren wird errechnet, an welchen Stellen der Oberfläche des Auges Material bis zu welcher Tiefe abzutragen ist. Im vorliegenden Beispiel ist das Soll-Ablationsprofil $D_{Soll}$ durch die Angabe der Soll-Ablationstiefe als Funktion des Ortes auf einem Gitter in der x-y-Ebene gegeben.

Diese Daten werden dann in Schritt S112 über die Schnittstelle 112' in die Datenverarbeitungseinrichtung 105 eingelesen und dort in deren Speicher abgespeichert.

Im Beispiel nach den Schritten S110 und S112, in anderen Ausführungsbeispielen aber auch vor diesen Schritten, werden in Schritt S114 mittels konfokaler Raman-Spektroskopie ortsaufgelöst Intensitäten von Raman-Strahlung als Wassergehaltsmeßdaten erfaßt und diese Intensitäten wiedergebende Daten zusammen mit entsprechenden Ortskoordinaten an die Datenverarbeitungseinrichtung 105 gesendet. Diese Ermittlung wird hier von einem Benutzer ausgelöst. In einem anderen Ausführungsbeispiel ist die Schnittstelle 110 auch als Steuerschnittstelle ausgebildet und die Datenverarbeitungseinrichtung 105 gibt über diese Steuerschnittstelle 110 ein entsprechendes Steuersignal an die Einrichtung 109 aus, um die Ermittlung automatisch zu starten.

In Schritt S116 werden die Wassergehaltsmeßdaten über die Schnittstelle 110 eingelesen und in dem Speicher der Datenverarbeitungseinrichtung 105 zwischengespeichert. Es werden dann für alle Orte, für die Wassergehaltsmeßdaten erfaßt wurden, aus den Intensitäten Wassergehaltswerte a ermittelt und den Orten zugeordnet gespeichert.

In Schritt S118 werden dann über die Schnittstelle 113 Strahlparameter des zu verwendenden Laserstrahls 3 eingelesen. In diesem Ausführungsbeispiel wird dazu der Durchmesser Strahlprofils des Laserstrahls 3 eingegeben. Die Form des Laserstrahls 3 wird als konstant über den Strahlquerschnitt und fest vorgegeben angenommen und ist in Form entsprechender Formeln in dem auf dem Prozessor der Datenverarbeitungseinrichtung 105 ablaufenden Programm berücksichtigt. Weiter wird die zu verwendende Pulsenergie pro Fläche bzw. die zu verwendende Fluence $F_0$ eingelesen und gespeichert.

In dem Schritt S120 wird dann aus dem vorgegebenen Soll-Ablationsprofil $D_{Soll}$ ein vorkompensiertes Soll-Ablationsprofil $D_{Mod}$ ermittelt. Dazu werden für alle Stützstellen, an denen das Soll-Ablationsprofil gegeben ist, die folgenden beiden Teilschritte ausgeführt: In dem Programmcode sind Werte für die Proportionalitätskonstante k und den Schwellwert für die Fluence $F_{thr}$ gespeichert. Bezeichnet ($x_i$ $y_i$) den Ort, wird die durch die Formel (8) gegebene Gleichung unter Verwendung der durch das Soll-Ablationsprofil vorgegebenen Soll-Ablationstiefe $D_{Soll}$ ($x_i,y_i$) und der Werte a($x_i$, $y_i$),b, k und $F_0/F_{thr}$ beispielsweise mittels eines Newton-Verfahrens zur Lösung nichtlinearer Gleichungen für die Pulsanzahl $N_{W,i}$ gelöst. Sollte a nicht an dem Ort ($x_i$, $y_i$) vorliegen, kann ein entsprechender Wert durch Interpolation erhalten werden. Dann wird der Wert M($D_{Soll}(x_i, y_i)$) der Modifikationsfunktion M gemäß Formel (10) ermittelt.

In dem folgenden Teilschritt wird zur Bildung einer vorkompensierten Soll-Ablationstiefe $D_{Mod}$ an dem Ort ($x_i$, $y_i$) die vorgegebene Soll-Ablationstiefe $D_{Soll}(x_i, y_i)$ mit dem Wert M($D_{Soll}(x_i, y_i)$) der Modifikationsfunktion multipliziert und als $D_{Mod}(x_i, y_i)$ dem Ort ($x_i$, $y_i$) zugeordnet gespeichert.

Das vorkompensierte Soll-Ablationsprofil $D_{Mod}$ ist dann durch die Orte ($x_i$, $y_i$) und die diesen zugeordneten vorkompensierten Soll-Ablationstiefen $D_{Mod}(x_i, y_i)$ gegeben.

In dem folgenden Schritt S122 wird auf der Basis des modifizierten Soll-Ablationsprofils $D_{Mod}$, der eingelesenen Fluence und des anderen Strahlparameters und der Formel (3) ein Ablationsprogramm erstellt, wozu ein Verfahren verwendet wird, das daher nicht den Wassergehalt des Gewebes oder die Änderung des Wassergehalts des Gewebes bei der Ablation berücksichtigt. Beispielsweise kann das in DE 19727573 C1 beschriebene Verfahren verwendet werden. Das so erstellte Ablationsprogramm umfaßt dabei eine Folge von Zielorten in der x-y-Ebene, das heißt entsprechende Koordinaten, auf die Laserpulse mit der durch die eingelesene Fluence bestimmten Pulsenergie gelenkt werden müssen, um das gewünschte Soll-Ablationsprofil zu erreichen. Das Ablationsprogramm wird in der Datenverarbeitungseinrichtung 105 gespeichert. Mit diesem Schritt ist die eigentliche Erstellung des Ablationsprogramms beendet.

In dem folgenden Schritt S126 werden dann von der Datenverarbeitungseinrichtung 105 mit der integrierten Steuereinrichtung 106 Steuerbefehle an den Laser 107 und die Ablenkeinrichtung 108 abgegeben, um entsprechend dem erstellten Ablationsprogramm Material von dem Auge 2 abzutragen.

Das Ablationsverfahren eignet sich sowohl für die photorefraktive Keratektomie als auch LASIK. Da bei diesen Verfahren Material in unterschiedlichen Schichten des Auges abgetragen wird, müssen dementsprechend gegebenenfalls unterschiedliche Soll-Ablationsprofile verwendet werden.

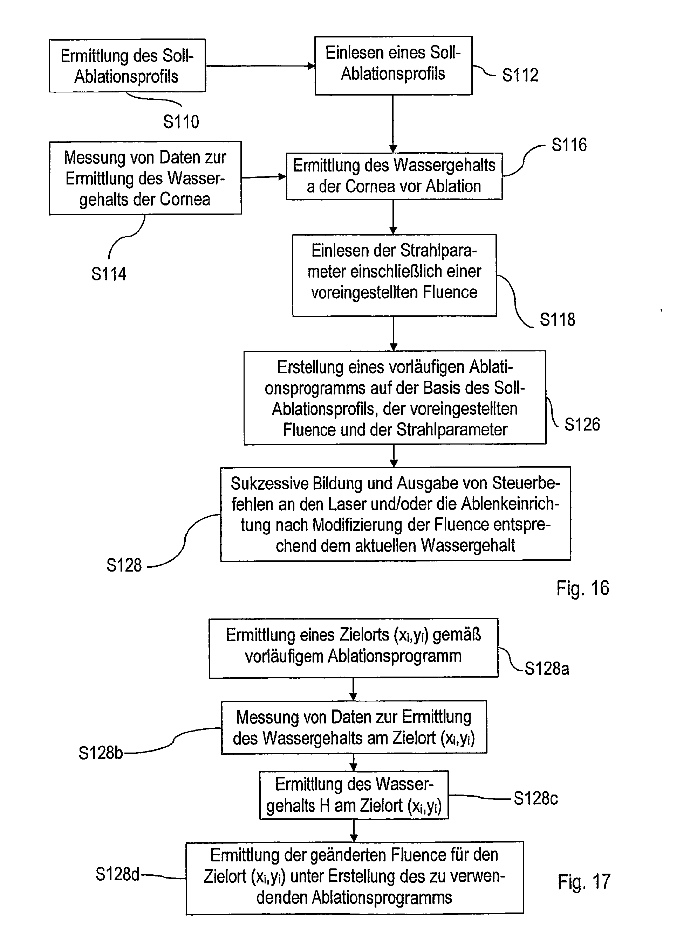

Ein zweites Ausführungsbeispiel für ein Verfahren zur Bildung und Abgabe von Steuersignalen zur Ablation gemäß einem mit dem Verfahren erstellten Ablationsprogramm ist in den Figuren 16 und 17 dargestellt. Eine entsprechende Signalbildungsvorrichtung unterscheidet sich von der Signalbildungsvorrichtung des ersten Ausführungsbeispiels für den zweiten Aspekt der Erfindung nur in der Programmierung der Datenverarbeitungseinrichtung 105 und dabei nur darin, daß diese das im folgenden geschilderte Verfahren zur Bildung von Steuersignalen bzw. zur Erstellung eines Ablationsprogramms durchführen kann. Das Verfahren unterscheidet sich von dem ersten Verfahren im wesentlichen dadurch, daß nun die Fluence von Pulsen des gepulsten Laserstrahls entsprechend dem Wassergehalt geändert wird, was darüber hinaus während der Abgabe der Steuersignale und damit der Ablation erfolgt.

Das Verfahren zur Bildung und Abgabe von Steuersignalen, das das Verfahren zur Erstellung eines Ablationsprogramms einschließt, weist einige Schritte auf, die denen des in Fig. 15 dargestellten Signalbildungs- bzw. Erstellungsverfahrens entsprechen. Diese werden daher im folgenden mit den gleichen Bezugszeichen bezeichnet und nicht nochmals näher beschrieben.

In den Schritten S110 und S112 bzw. S114 und S116 werden also Daten zu dem Soll-Ablationsprofil bzw. dem Wassergehalt des Cornea-Gewebes vor der Ablation ermittelt und gespeichert.

In dem dann folgenden Schritt S118 werden ein Strahlquerschnitt und ein voreingestellter Anfangsfluencewert $F_0$ eingelesen.

In dem folgenden Schritt S126 wird dann ein vorläufiges Ablationsprogramm auf der Basis des eingelesenen Soll-Ablationsprofils, der eingelesenen voreingestellten Fluence und des Strahlparameters erstellt, wobei das hierzu verwendete einfache Erstellungsverfahren den Wassergehalt der Cornea bzw. Variationen des Wassergehalts nicht berücksichtigt. Insbesondere kann das oben erwähnte Verfahren aus DE 197 27 573 C1 verwendet werden. Das vorläufige Ablationsprogramm, das wiederum aus einer Folge von Koordinaten für Orte auf der Oberfläche des Auges 2, auf die Pulse zu lenken sind, umfaßt, wird dann abgespeichert.

Daraufhin werden in Schritt S128 aus der Folge der Zielorte entsprechend dem vorläufigen Ablationsprogramm für die entsprechenden Pulse modifizierte Fluence-Werte ermittelt und den Zielorten unter Bildung des zu verwendenden Ablationsprogramms zugeordnet. Nach Bildung eines modifizierten Fluence-Wertes wird unmittelbar ein Steuersignal gebildet und an den Laser 107 bzw. die Ablenkeinrichtung 108 abgegeben. Durch entsprechende Ansteuerungen einer Hochspannungsversorgung des Eximerlasers 107 und damit der Aufladung von Kondensatoren, in denen jeweils die Energie für einen Puls gespeichert wird, entsprechend dem Ablationsprogramm und gleichzeitige Ansteuerung der Ablenkeinrichtung 108 kann die Ablation mit einem Laserpuls mit einer orts- bzw. koordinatenabhängigen Pulsenergie und damit Fluence erfolgen.

Dabei wird die Folge der Zielorte sukzessive abgearbeitet. Die entsprechenden Teilschritte zur Ermittlung einer geänderten Fluence einen auf einen Zielort $(x_j, y_j)$ abzugebenden Puls sind in Fig. 17 gezeigt.

In Teilschritt S128a wird zunächst bei dem ersten Durchlauf der erste Zielort, bei späteren Durchläufen der nächste Zielort gemäß dem vorläufigen Ablationsprogramm ausgelesen. Dieser hat die Koordinaten $(x_j, y_j)$, wobei j die Ordinalzahl des Zielortes in der Folge ist.

In Teilschritt S128b wird dann durch Ansteuerung der Einrichtung 109 zur Erfassung von Wassergehaltsmeßdaten durch die Datenverarbeitungseinrichtung 105 über eine nicht gezeigte Steuerschnittstelle eine Erfassung entsprechender Daten für den Zielort $(_{x_j}, y_j)$ durchgeführt.

Die Daten werden über die Schnittstelle 111 eingelesen und in Teilschritt S128c mittels der Datenverarbeitungseinrichtung in einen Wassergehalt $H(x_j, y_j)$ an dem Zielort $(x_j, y_j)$ umgerechnet.

In Teilschritt S128d wird dann ein geänderter Fluence-Wert für den auf den Zielort $(x_j, y_j)$ abzugebenden Puls ermittelt, der dem Zielort zugeordnet ist. Der geänderte Fluence-Wert zusammen mit dem Zielort $(x_j, y_j)$ bildet ein Element des schließlich zu verwendenden Ablationsprogramms.

Der Fluence-Wert wird nun so geändert, daß bei Ablation mit der geänderten Fluence die Einzelpuls-Ablationstiefe erreicht wird, die bei Erstellung des vorläufigen Ablationsprogramms ohne die Berücksichtigung des Wassergehalts als Ablationstiefe für einen Einzelpuls angenommen wurde. Ausgehend von den oben angegebenen Gleichungen (1), (3) und (4) muß also die Gleichung $D_{P,W}(F) = D_{P,0}(F_0)$ nach F aufgelöst werden, wobei $D_{P,W}$ über $\mu(H)$ von dem Wassergehalt H an dem Zielort abhängt. Verwendet man die Modelle gemäß den Gleichungen (1), (3) und (4) erhält man die folgende Gleichung für die geänderte Fluence:

$$\frac{F}{F_{thr}} = \frac{F_0}{F_{thr}} e^{\frac{\mu_0}{\mu(H)}} \qquad\qquad (11).$$

Diese Berechnung kann sehr schnell durchgeführt werden, so daß der Teilschritt S28d sehr schnell durchlaufen werden kann.

Es können entsprechende Steuersignale für den Laser 107 und die Ablenkeinrichtung 108 gebildet und an den Laser 107, d.h. die Hochspannungsversorgung des Lasers 107, bzw. die Ablenkeinrichtung 108 abgegeben werden. Danach wird das Verfahren mit dem Teilschritt S128a für das nächste Element des vorläufigen Ablationsprogramms fortgesetzt. Die Schritte S110 bis S128d ohne die Bildung und Abgabe der Steuersignale bilden wie zuvor die Schritte S110 bis S122 ein Ausführungsbeispiel für ein erfindungsgemäßes Erstellungsverfahren des zweiten Aspekts der Erfindung.

Damit brauchen, bis auf das Modell für die Ablationstiefe, keinerlei Annahmen über den Wassergehalt der Cornea getroffen zu werden. Insbesondere können auch unvorhergesehene Änderungen des Wassergehalts während der Bildung und Abgabe der Steuersignale und damit der Ablation voll berücksichtigt werden.

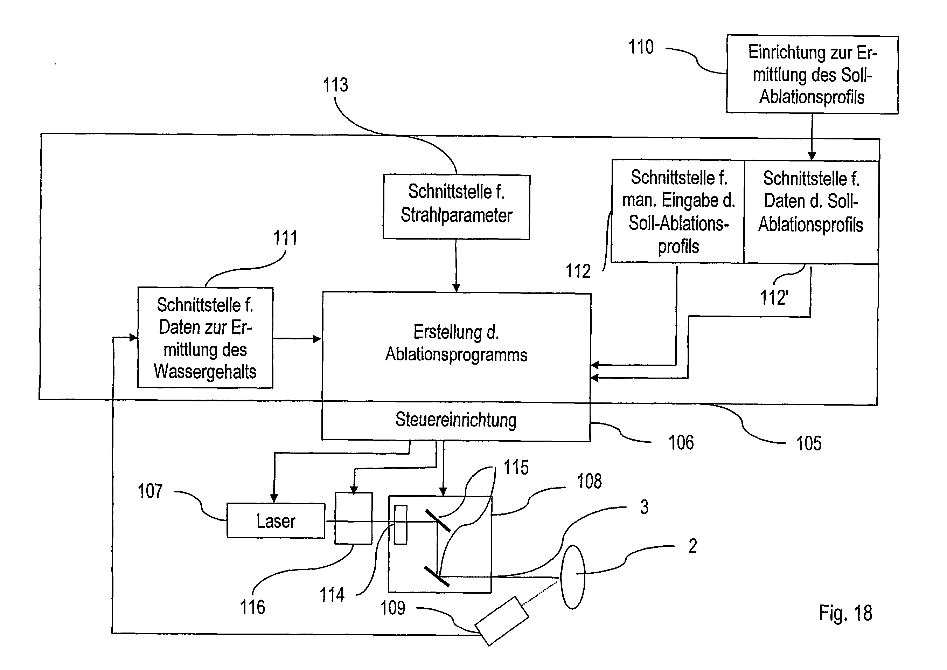

Eine dritte Ausführungsform, die eine Variante des zuvor geschilderten Ausführungsbeispiels darstellt, ist in Figur 18 gezeigt. Das hier schematisch dargestellte Instrument unterscheidet sich von dem Instrument des vorhergehenden Ausführungsbeispiels dadurch, daß im Strahlengang des Laserstrahls 3 zwischen dem Laser 107 und der Ablenkeinrichtung 108 ein Modulator 116, im Beispiel ein von der Steuereinrichtung 106' in seiner Transmission steuerbares Flüssigkristall-Element, angeordnet ist. Darüber hinaus weist die Steuereinrichtung 106' gegenüber der Steuereinrichtung 106 einen Ausgang zur Ansteuerung des Modulators 116 auf und das in der Datenverarbeitungseinrichtung 105 abgearbeitete Computerprogramm ist zur Steuerung der Fluence nicht durch Ansteuerung des Lasers 107, sondern durch Änderung der Transmission des Modulators 116 ausgebildet.

Die Fluence des Lasers 107 wird nun so eingestellt, daß bei maximaler Transmission des Modulators 116 die maximal erforderliche Fluence zur Ablation gemäß dem Ablationsprogramm erreicht werden kann. Der Modulator 116 wird bei der Ablation entsprechend dem Ablationsprogramm so gesteuert, daß die auf die Oberfläche auftreffenden Pulse die gewünschte Energie bzw. Fluence besitzen.

Bei einer anderen Variante kann statt des Modulators 116 eine strahlformende Einrichtung vorgesehen sein, die auf Signale von der Steuereinrichtung den Strahlquerschnitt und damit die Fluence ändert.

Bei anderen Ausführungsformen der zuvor beschriebenen Verfahren kann der Ablationsschwellwert $F_{thr}$ für die Fluence ebenfalls von dem Wassergehalt H abhängen.

Bei einer fünften bevorzugten Ausführungsform eines erfindungsgemäßen Steuersignalbildungsverfahrens gemäß dem zweiten Aspekt der Erfindung wird bei der Erstellung des Ablationsprogramms unmittelbar die Gleichung (9) verwendet, indem ein Muster von Zielorten vorgegeben wird und für jeden Zielort die Gleichung (9) als Funktion der Anzahl der abzugebenden Pulse $N_{W,i}$ gelöst wird.

Bei einer sechsten bevorzugten Ausführungsform eines Steuersignalbildungsverfahrens gemäß dem zweiten Aspekt der Erfindung wird über den Wassergehalt hinaus auch noch die Neigung der Oberfläche der Cornea gegenüber dem Laserstrahl 3 bzw. der z-Richtung als Näherung für die Richtung des Laserstrahls 3 in Verbindung mit der tatsächlichen Form des Strahlprofils berücksichtigt. Insofern stellt dieses Ausführungsbeispiel und Modifikationen hiervon insbesondere auch ein Ausführungsbeispiel zu dem ersten Aspekt der Erfindung dar. Der von dem Laser 107 abgegebene Laserstrahl 3 hat im Beispiel ein Strahlprofil mit Gaußform.

Während bei konventionellen Verfahren die wirksame Fluence F (x, y) als konstant über die Fläche des Pulses bzw. Spots angenommen wird, werden nun neben dem Einfluß des Wassergehalts zwei weitere Einflüsse gleichzeitig berücksichtigt. Zum einen wird berücksichtigt, daß durch die Neigung der zu bearbeitenden Oberfläche die wirksame Fluence entsprechend der Neigung gegenüber der Fluence des Laserstrahls 3 herabgesetzt wird. Wird die zu bearbeitende Oberfläche durch eine Höhenfunktion f(x, y), die die Höhe der Oberfläche über der x-y-Ebene angibt, beschrieben, kann der Neigungswinkel θ der Oberfläche relativ zu der z-Achse und damit näherungsweise zu dem auf die Oberfläche 2' einfallenden Laserstrahl 3 gemäß der Formel

$$\theta(x,y) = \arctan\big(|\text{grad}\, f(x,y)|\big) \qquad (12)$$

ermittelt werden. Die bei der Ablation wirksame Fluence F(x, y) auf der Oberfläche ergibt sich damit zu

$$F(x,y) = F_P(x,y) \cdot \cos\theta(x,y) \qquad (13)$$

wobei $F_P(x,y)$ die Fluence bei senkrechtem Einfall des Laserstrahls 3 auf die Oberfläche, d.h. bei einem Winkel θ=0 bezeichnet. $F_P$ entspricht daher der Fluence bzw. dem Strahlprofil des Laserstrahls 3.

Als zweiter Effekt wird berücksichtigt, daß die wirksame Fluence entsprechend dem Intensitäts- bzw. Energieprofil des Pulses in einer Ebene orthogonal zur Richtung des Laserstrahls 3 variiert und dabei insbesondere auch den Schwellwert $F_{thr}$ unterschreiten kann.

Damit ergibt sich das tatsächliche Einzelpuls-Ablationsvolumen aus der folgenden Formel:

$$V_{Puls} = \iint\limits_{Spot} D_P(x,y)\,dxdy = \mu \iint\limits_{Spot} \ln\frac{F(x,y)}{F_{thr}}\,dxdy = \mu \iint\limits_{Spot} \ln\frac{F_P(x,y)\cos\theta(x,y)}{F_{thr}}\,dxdy \qquad (14).$$

Das Einzelpuls-Ablationsvolumen hängt daher nichtlinear vom Neigungswinkel der Oberfläche und dem Fluenceprofil $F_P(x,y)$ ab.

Bei bekannten Einzelpuls-Ablationsvolumina kann mit bekannten einfachen Erstellungsverfahren wie sie beispielsweise in DE 197 27 573 C1 oder EP 1060710 A2 beschrieben sind, ein Ablationsprogramm erstellt werden, gemäß dem sich die mittlere Ablationstiefe $D_M$ gleichmäßig oder langsam variierend unter einem Abstand d auf die Oberfläche gelenkter Laserstrahlpulse gemäß

$$D_M = c\,\frac{V_{Puls}}{d^2} \qquad (15)$$

ergibt. c ist hierbei ein Proportionalitätsfaktor, der sich aus der Anordnung der Auftreffpunkte der Pulse des Laserstrahls ergibt. Beispielsweise gilt bei einem näherungsweise quadratischen Muster c=1, während bei einem näherungsweise hexagonalen Muster c=2/√3 gilt.

In folgenden wird davon ausgegangen, daß sich das Ablationsprofil wie auch der Wassergehalt räumlich langsam gegenüber der Länge des Abstands d oder die Neigung langsam gegenüber dem Durchmesser des Laserstrahls 3 an der Oberfläche verändert. Zur Beschreibung der räumlichen Abhängigkeit dieser räumlich langsam veränderlichen Größen werden im folgenden Koordinaten u und v verwendet, die in einem u-v-Koordinatensystem gegeben sind, das mit dem x-y-Koordinatensystem zusammenfällt. Das Einzelpuls-Ablationsvolumen ist damit ebenso wie die sich aus den nebeneinandergesetzten Pulsen ergebende mittlere Tiefe $D_M$ als Funktion von u und v aufzufassen.

Die effektive Ablationstiefe ergibt sich dann durch

$$D_{M,Ist}(u,v) = c\frac{V_{Puls,Ist}(u,v)\cos\theta(u,v)}{d^2} \tag{16}$$

In Formel (9) braucht nun nur noch $D_{P,W}$ durch $D_{M,Ist}$ ersetzt zu werden, wobei $V_{Puls,Ist}$ unter Verwendung von Formel (14), in der $D_P$ durch $D_{P,W}$ ersetzt ist, ermittelt wird.

Damit werden für alle verschiedenen zu ablatierende Bereiche der Oberfläche jeweils ein Wert der strahl- und/ oder neigungsabhängigen Modifikationsfunktion in Abhängigkeit von wenigstens der Form des Strahlprofils und/oder der Neigung der Oberfläche in dem jeweiligen Bereich und ein Wert der wassergehaltabhängigen Modifikationsfunktion ermittelt werden und das vorkompensierte Soll-Ablationsprofil unter Verwendung des Soll-Ablationsprofils und der Werte der Modifikationsfunktionen, insbesondere des Produkts der Modifikationsfunktionen, ermittelt.

Zu Ermittlung der Orte, auf die Laserpulse abzugeben sind, können verschiedene bekannte Verfahren verwendet werden, beispielsweise die bereits zuvor erwähnten, in DE 19727573 C1 und EP 1060710 A2 beschriebenen Verfahren. In DE 19727573 C1 erfolgt die Ablation schichtweise, das heißt es werden Auftreff- bzw. Zielorte für Pulse schichtweise ermittelt, wobei sich das gewünschte Profil dann durch Überlagerung dieser Schichten ergibt. Bei dem Verfahren in EP 1060710 A2 wird der Spotabstand quasi- kontinuierlich variiert, um die gewünschte Ablationstiefe zu erreichen.

Die Berechnung der Modifikationsfunktion sei am Beispiel der Ablation von einer sphärischen Oberfläche mit einem Laserstrahl mit einem Strahlprofil mit Gauß-Form erläutert (vgl. Fig. 3).

Das Strahlprofil bzw. die Fluence ist durch die Formel

$$F_P(r) = F_0 e^{-\frac{r^2}{w^2}} \tag{17}$$

beschrieben, wobei $F_0$ der Spitzenwert der Fluence, $r$ der radiale Abstand zum Zentrum des Strahlprofils und $w$ der Abstand ist, nach dem das Profil relativ zu dem Wert im Zentrum auf 1/e abgefallen ist.

Damit ergibt sich für das Ist-Ablationsprofil eines Laserpulses, der orthogonal auf eine ebene Fläche auftrifft:

$$D_{Ist}(r) = \mu\ln\frac{F_0}{F_{thr}} - \mu\frac{r^2}{w^2}, \tag{18}$$

so daß sich das Einzelpuls-Ablationsvolumen für diesen Puls zu

$$V_{Puls,E} = \frac{\pi}{2}\mu(H)w^2\left[\ln\frac{F_0}{F_{thr}}\right]^2 \tag{19}$$

berechnet. Als mittlere Tiefe des gewünschten Profils bei einem quadratischen Muster der Auftreffpunkte der Pulse bzw. Spot-Muster mit Kantenlänge a ist eine mittlere Tiefe der Ablation gemäß

$$D_E(r) = \frac{V_{s,E}}{d^2} = \frac{\pi}{2}\mu\frac{w^2}{d^2}\left[\ln\frac{F_0}{F_{thr}}\right]^2 \tag{20}$$

zu erwarten. Setzte man nun $D_E$ gleich $D_{soll}$ so könnte d als Funktion des Ortes ermittelt werden.

Tatsächlich ist die sphärische Oberfläche jedoch außer im Zentrum gegenüber dem als in hinreichend guter Näherung parallel zur z-Achse einfallend angenommen Laserstrahl 3 geneigt. Wie sich aus Abbildung bzw. Figur 3 ergibt, kann der Neigungswinkel in Abhängigkeit von dem Abstand ρ eines Ortes auf der Oberfläche des Auges 2 folgendermaßen berechnet werden:

$$\theta(\rho) = \arcsin\left(\frac{-\rho}{R}\right) = \arctan\left(\frac{-\rho}{\sqrt{R^2 - \rho^2}}\right). \tag{21}$$

Die Neigung der Oberfläche am Ort ρ führt nun dazu, daß sowohl der Spotabstand in der Neigungsrichtung als auch die Spotbreite w in Neigungsrichtung um den Faktor $1/\cos(\theta(\rho))$ vergrößert werden. Daher ändert sich das Verhältnis w/d in der Gleichung für D nicht mit der Neigung. Es ändert sich jedoch die Fluence in dieser Gleichung. Weiterhin hängt μ nun vom Wassergehalt H, beispielsweise gemäß den Gleichungen (6) und (7), ab. Man erhält für das zu erwartende Tiefenprofil auf der sphärischen Fläche daher folgendes Ergebnis:

$$D_{M,Ist}(\rho) = c\frac{V_{Puls,Ist}(\rho)\cos\theta(\rho)}{d^2} = c\frac{\pi}{2}\mu(H)\frac{w^2}{d^2}\left[\ln\frac{F_0 \cdot \cos\theta(\rho)}{F_{thr}}\right]^2 \tag{22}$$

Systematisch kann dieses Resultat auch dadurch erhalten werden, daß in der Formel für das Einzelpuls-Ablationsvolumen $\cos(\theta)$ und $\mu=\mu(H)$ als über die Spotfläche konstant angesehen wird.

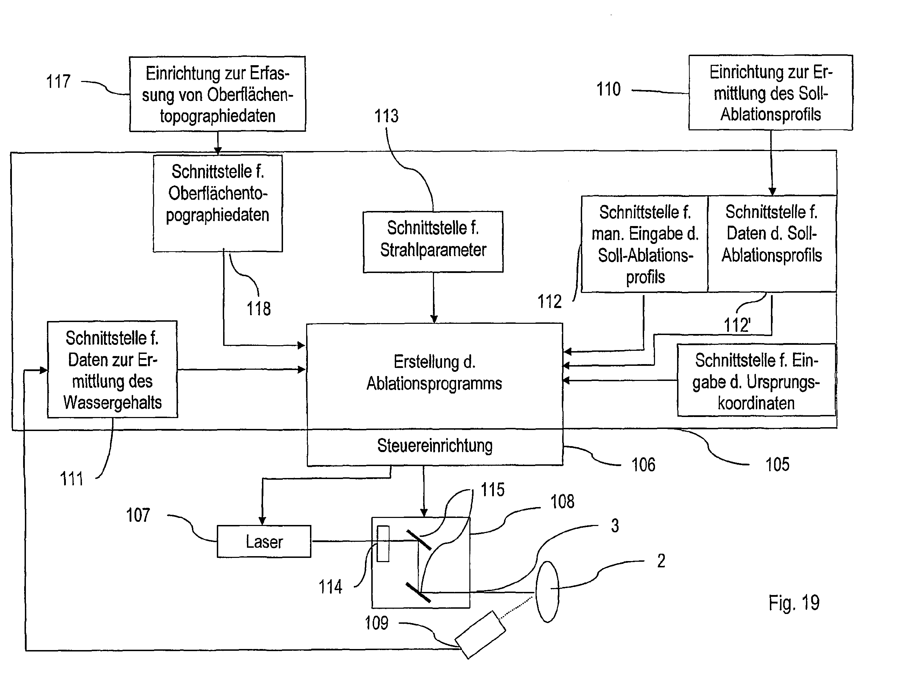

In Fig. 19 ist eine Laserablationsvorrichtung mit einer Erstellungsvorrichtung bzw. einer Steuersignalbildungsvorrichtung nach einer weiteren bevorzugten Ausführungsform der Erfindung gezeigt, die sich von der Laserablationsvorrichtung des ersten Ausführungsbeispiels zum zweiten Aspekt der Erfindung darin unterscheidet, daß die Datenverarbeitungseinrichtung 105 durch eine Datenverarbeitungseinrichtung 105' ersetzt ist und daß eine Einrichtung 117 zur Erfassung der Oberflächentopographie des Auges 2 vorgesehen ist, die über eine Datenverbiridung mit einer Schnittstelle 118 der Datenverarbeitungseinrichtung 105' für das Einlesen von Oberflächentopographiedaten in die Datenverarbeitungseinrichtung 105' verbunden ist. Über diese Schnittstelle sind auch Steuerbefehle zur Erfassung von Oberflächentopographiedaten an die Einrichtung 117 abgebbar. Die anderen Komponenten sind unverändert, so daß für diese die gleichen Bezugszeichen verwendet werden und die Ausführungen im Ausführungsbeispiel zu diesen Komponenten auch hier entsprechend gelten.

Die Einrichtung 117 zur Erfassung der Oberflächentopographie des Auges kann im Beispiel einen optischen Kohärenztomographen umfassen.

Die Datenverarbeitungseinrichtung 105' unterscheidet sich von der Datenverarbeitungseinrichtung 105 nur durch die Schnittstelle 118 und das Computerprogramm, mit dem die Datenverarbeitungseinrichtung 105' bzw. deren Prozessor programmiert ist. Das Computerprogramm weist Programmcode auf, so daß bei Ausführung des Computerprogramms in der Datenverarbeitungeinrichtung 105' das im folgende geschilderte Verfahren abgearbeitet wird.

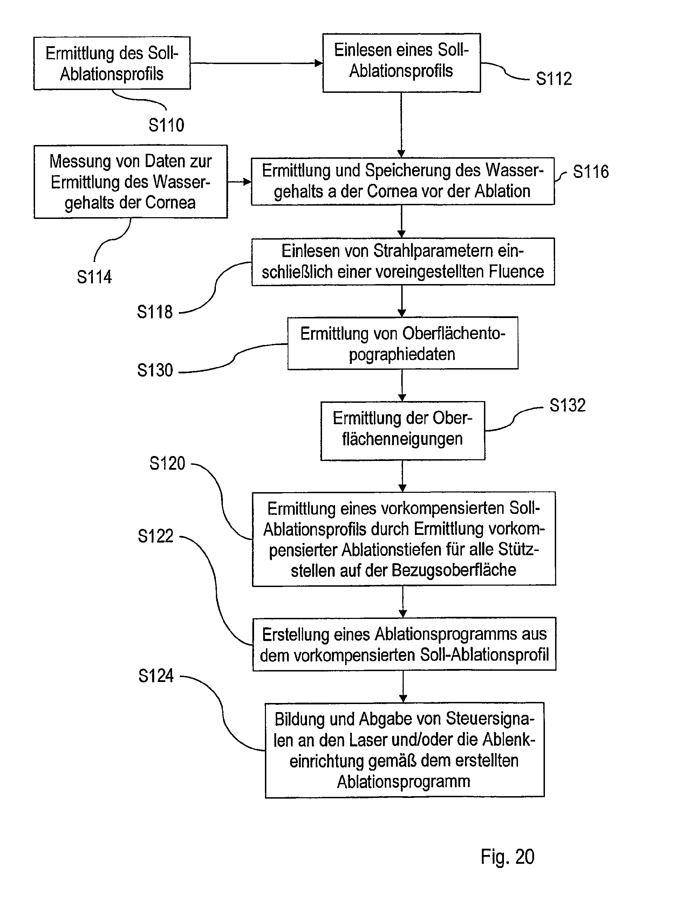

Zur Ermittlung des Ablationsprogramms und zur Bildung und Abgabe der Steuersignale werden die in dem Ablaufdiagramm in Fig. 20 skizzierten Verfahrensschritte durchgeführt, von denen die mit den gleichen Bezugszeichen wie in Fig. 15 bezeichneten Schritte wie im ersten Ausführungsbeispiel zum zweiten Aspekt der Erfindung ausgeführt werden und die Erläuterungen zu diesen Schritten auch hier entsprechend gelten.

In den Schritten S110 und S112 bzw. S114 und S116 werden also Daten zu dem Soll-Ablationsprofil bzw. dem Wassergehalt des Cornea-Gewebes vor der Ablation ermittelt und gespeichert.

In dem dann folgenden Schritt S118 werden ein Strahlradius w und ein voreingestellter Fluence-Wert $F_0$ ein-gelesen, der den Spitzenwert der Fluence über das Gaußsche Strahlprofil des Lasers 107 darstellt. Die Form des Strahlprofils ist durch die verwendeten Formeln berücksichtigt.

Dann wird in dem folgenden Schritt S130 auf Ansteuerung durch die Datenverarbeitungseinrichtung 105' die Oberflächentopographie des zu behandelnden Bereichs mittels der Einrichtung 117 zu Erfassung der Oberflächentopographiedaten ermittelt. Die entsprechenden Daten können beispielsweise über einem Punktraster in der x-y-Ebene erfaßte Höhen der Oberfläche gegenüber der x-y-Ebene umfassen.

In Schritt S132 werden diesen Daten dann über die Schnittstelle 118 für die Oberflächentopographiedaten in die Datenverarbeitungseinrichtung 105' eingelesen. Aus den Höhendaten werden dann Oberflächenneigungen durch numerische Ermittlungen von Gradienten und die oben angegebene Formel für den Neigungswinkel ermittelt. Die entsprechenden Daten werden dann in dem Speicher der Datenverarbeitungseinrichtung 105' gespeichert.

Die folgenden Schritte entsprechen dann den Schritten des Verfahrens in dem ersten Ausführungsbeispiel, wobei jedoch $N_{W,i}$ nun unter Verwendung der Formel (22) für $D_{P,W}$ in Gleichung (8) ermittelt wird.

Auf diese Weise findet eine Vorkompensation sowohl in Bezug auf den Wassergehalt als auch die Neigung der Oberfläche und der Form des Strahlprofils derart statt, daß deren Einflüsse, die bei der Erstellung des Ablationsprogramms mit einem einfachen Erstellungsverfahren vernachlässigt werden, vorab ausgleichend bzw. vorkompensierend berücksichtigt werden und das Ist-Ablationsprofil dem Soll-Ablationsprofil sehr nahe kommt.

Das Ablationsverfahren eignet sich sowohl für die photorefraktive Keratektomie als auch LASIK. Da bei diesen Verfahren Material in unterschiedlichen Schichten des Auges abgetragen wird, müssen entsprechend gegebenenfalls unterschiedliche Soll-Ablationsprofile verwendet werden.

Der Kohärenztomograph der Einrichtung 117 kann weiter dazu verwendet werden, die Dicke der Cornea vor und/oder während der Ablation zu ermitteln, d.h. eine pachymetrische Messung durchzuführen. Damit kann insbesondere vermieden werden, daß bei der Ablation die Restdikke der Cornea einen vorgegebenen Mindestwert unterschreitet.

In einem anderen Ausführungsbeispiel kann statt der Einrichtung 109 zur Durchführung konfokaler Raman-Spektroskopie eine Temperaturmeßeinrichtung, beispielsweise mit einer Infrarotkamera, deren optische Achse in einem spitzen Winkel zu der Richtung des Laserstrahls 3 geneigt ist, verwendet werden. Diese erfaßt Daten, aus denen die Temperatur der Cornea und hieraus deren Wassergehalt ortsaufgelöst ermitteltbar ist.

Alternativ kann statt der Einrichtung 109 zur Durchführung konfokaler Raman-Spektroskopie ein optischer Kohärenztomograph verwendet werden, mittels dessen Daten, aus denen der Brechungsindex des Cornea-Gewebes errechenbar ist, erfaßt werden können. Aus den Brechungsindexdaten kann dann mittels der Datenverarbeitungseinrichtung 105 wiederum der Wassergehalt ermittelt werden. Der Kohärenztomograph kann zusätzlich zu einem Kohärenztomographen zur Ermittlung der Oberflächentopographie, soweit dieser vorgesehen ist, vorhanden sein. Es ist jedoch auch möglich denselben Kohärenztomographen für beide Funktionen zu verwenden. Der Kohärenztomograph kann weiter dazu verwendet werden, die Dicke der Cornea vor und/oder während der Ablation zu ermitteln, d.h. eine pachymetrische Messung durchzuführen. Damit kann insbesondere vermieden werden, daß bei der Ablation die Restdicke der Cornea einen vorgegebenen Mindestwert unterschreitet.

Weiter kann eine Einrichtung zur Ermittlung der Luftfeuchte im Bereich der Cornea vorgesehen sein, die über eine Datenverbindung Feuchtedaten an die Datenverarbeitungseinrichtung 105 überträgt, wo sie in das Modell für die Ablationstiefe einfließen kann.

Bei weiteren Ausführungsbeispielen kann in Abhängigkeit vom Wassergehalt auch die Wiederholrate, mit der Pulse auf das Material, bzw. die Cornea abgegeben werden, modifiziert werden.

**Patentansprüche**

1. Verfahren zur Erstellung eines Ablationsprogramms für eine Ablation von Material von einer Oberfläche (2') eines Körpers (2) gemäß einem vorgegebenen Soll-Ablationsprofil durch die Abgabe von Pulsen eines gepulsten Laserstrahls (3) auf die Oberfläche (2'), wobei das Ablationsprogramm aus dem Soll-Ablationsprofil erstellt wird, **dadurch gekennzeichnet, daß** das Ablationsprogramm wenigstens eine Angabe einer Folge von verschiedenen Zielorten oder -richtungen umfaßt, auf die bzw. in die Pulse des Laserstrahls abzugeben sind, und daß bei der Erstellung des Ablationsprogramms die Form eines Strahlprofils des Laserstrahls (3) berücksichtigt wird, wobei das Strahlprofil einen Verlauf der Intensität, der Energie oder der Fluence über einen Querschnitt des Laserstrahles (3) nahe der Oberfläche (2') angibt.

2. Verfahren nach Anspruch 1, **dadurch gekennzeichnet, daß** das Ablationsprogramm auch in Abhängigkeit von einer Neigung der zu ablatierenden Oberfläche (2') erstellt wird.

3. Verfahren nach Anspruch 2, bei dem zur Berücksichtigung der Neigung der Oberfläche (2') und der Form des Strahlprofils aus dem Soll-Ablationsprofil in Abhängigkeit von wenigstens der Form des Strahlprofils und der Neigung der Oberfläche (2') an einem jeweiligen Zielort auf der Oberfläche (2') ein vorkompensiertes Soll-Ablationsprofil ermittelt wird, und aus dem vorkompensierten Soll-Ablationsprofil das Ablationsprogramm erstellt wird.

4. Verfahren nach Anspruch 3, bei dem für wenigstens zwei zu ablatierende Bereiche der Oberfläche (2') jeweils ein Wert einer Modifikationsfunktion in Abhängigkeit von wenigstens der Form des Strahlprofils und der Neigung der Oberfläche (2') in dem jeweiligen Bereich ermittelt werden und das vorkompensierte Soll-Ablationsprofil unter Verwendung des Soll-Ablationsprofils und der Werte der Modifikationsfunktion ermittelt wird.

5. Verfahren nach Anspruch 4, bei dem die Modifikationsfunktion zusätzlich von der Intensität, der Energie oder der Fluence der bei der Ablation zu verwendenden Pulse abhängt.

**6.** Verfahren nach Anspruch 2, bei dem aus dem Soll-Ablationsprofil ein vorläufiges Ablationsprogramm ermittelt wird, für wenigstens einen der abzugebenden Pulse ein Soll-Wert für dessen Energie oder Fluence in Abhängigkeit von der Form des Strahlprofils, der Neigung der Oberfläche (2') in dem Bereich, auf den der Puls abgegeben werden soll, und von einer Vorhersage der Ablationstiefe bei Verwendung des vorläufigen Ablationsprogramms ermittelt wird, und das erstellte Ablationsprogramm den Zielort des Pulses gemäß dem vorläufigen Ablationsprogramm und den ermittelten Soll-Wert für die Energie oder Fluence des Pulses umfaßt.

**7.** Verfahren nach Anspruch 2, bei dem aus dem Soll-Ablationsprofil ein vorläufiges Ablationsprogramm ermittelt wird, unter Verwendung des vorläufigen Ablationsprogramms ein vorhergesagtes Ablationsprofil in Abhängigkeit von der Form des Strahlprofils und der Neigung der Oberfläche (2') vorhergesagt wird, und unter Verwendung des vorhergesagten Ablationsprofils das zu verwendende Ablationsprogramm erstellt wird.

**8.** Verfahren nach Anspruch 7, bei dem das vorhergesagte Ablationsprofil an wenigstens zwei Punkten ermittelt wird, die weniger als der Durchmesser des Laserstrahls (3) auf der Oberfläche (2') des Körpers (2) voneinander beabstandet sind.

**9.** Verfahren nach Anspruch 7 oder 8, bei dem unter Verwendung des vorhergesagten Ablationsprofils und des vorgegebenen Soll-Ablationsprofils ein vorkompensiertes Soll-Ablationsprofil ermittelt und das zu verwendende Ablationsprogramm aus dem vorkompensierten Soll-Ablationsprofil erstellt wird.

**10.** Verfahren einem der Ansprüche 7 bis 9, bei dem die Erstellung des zu verwendenden Ablationsprogramms iterativ erfolgt, indem in einer aktuellen Iterationsschleife aus einem in einer vorhergehenden Iterationsschleife ermittelten modifizierten Soll-Ablationsprofil ein vorläufiges Ablationsprogramm ermittelt wird, ausgehend von dem vorläufigen Ablationsprogramm in Abhängigkeit von der Form des Strahlprofils ein aktuelles vorhergesagtes Ablationsprofil vorhergesagt wird, unter Verwendung des aktuellen vorhergesagten Ablationsprofils ein aktuelles modifiziertes Soll-Ablationsprofil ermittelt wird und nach der letzten Iterationsschleife das zu verwendende Ablationsprogramm in Abhängigkeit von dem vorgegebenen Soll-Ablationsprofil und wenigstens einem der modifizierten Soll-Ablationsprofile erstellt wird.

**11.** Verfahren nach einem der vorhergehenden Ansprüche, bei dem ein Modell verwendet wird, mittels dessen für einen Puls in Abhängigkeit von der Form des Strahlprofils der Verlauf der Ablationstiefe vorhersagbar ist, oder bei dem zur Erstellung des Ablationsprogramms das Strahlprofil vermessen wird.

**12.** Verfahren nach einem der vorhergehenden Ansprüche, bei dem zur Erstellung des Ablationsprogramms Topographiedaten der Oberfläche (2') erfaßt werden.

**13.** Verfahren nach einem der vorhergehenden Ansprüche, bei dem die Vorkompensation in Abhängigkeit von einer von dem individuellen Körper abhängigen Ablationseigenschaft des Materials und/oder einer zu erwartenden Änderung der Oberflächen- und/oder Materialeigenschaften bei und/oder infolge der Ablation durchgeführt wird.

**14.** Verfahren nach einem der vorhergehenden Ansprüche, bei dem das Material wasserhaltig ist, bei dem bei der durchzuführenden Ablation der Laserstrahl über die Oberfläche geführt wird, und bei dem das Ablationsprogramm ausgehend von dem Soll-Ablationsprofil zusätzlich unter Berücksichtigung eines Wassergehalts des zu ablatierenden Materials erstellt wird.

**15.** Verfahren nach Anspruch 14, bei dem bei der Erstellung des Ablationsprogramms der Wassergehalt in Abhängigkeit vom Ort auf der Oberfläche (2') oder in einem zu ablatierenden Bereich berücksichtigt wird.

**16.** Verfahren nach Anspruch 14 oder 15, bei dem ein Modell verwendet wird, das die Abhängigkeit der durch wenigstens einen auf einen Zielort auf der Oberfläche (2') abgegebenen Puls erzielten Ablationstiefe oder des durch wenigstens einen auf einen Zielort auf der Oberfläche (2') abgegebenen Puls erzielten Ablationsvolumens von dem Wassergehalt des mit dem Puls zu ablatierenden Materials wiedergibt.

**17.** Verfahren nach einem der Ansprüche 14 bis 16, bei dem ein weiteres Modell verwendet wird, das für einen vorgegebenen Bereich des Materials den Einfluß von auf diesen Bereich oder benachbarte Bereiche aufgetroffenen Pulsen des gepulsten Laserstrahls (3) auf den Wassergehalt wiedergibt.

**18.** Verfahren nach einem der Ansprüche 14 bis 17, bei dem zur Berücksichtigung des Wassergehalts bei der Erstellung des Ablationsprogramms ein Modell für den Wassergehalt oder die Änderung des Wassergehalts des Materials in Abhängigkeit von wenigstens der Anzahl und/oder Lage vorher auf den gleichen Ort und/oder benachbarte Orte abgegebener Pulse verwendet wird.

19. Verfahren nach einem der Ansprüche 14 bis 18, bei dem der Wassergehalt aus an dem Körper (2) gemessenen Daten ermittelt wird, insbesondere indem zur Ermittlung des Wassergehalts Daten verwendet werden, die die Temperatur der Oberfläche (2') oder die Eigenschaften von optischer Strahlung wiedergeben, die von dem Material im zu ablatierenden Bereich des Körpers (2) ausgeht.

20. Verfahren nach Anspruch 19, bei dem zur Ermittlung des Wassergehalts Daten, die durch konfokale Raman-Spektroskopie von optischer Strahlung von der Oberfläche (2') erhältlich sind und/oder Eigenschaften von Fluoreszenzstrahlung wiedergeben, die von dem zu ablatierenden Bereich des Körpers (2) ausgeht, und/oder die den Brechungsindex in dem Material wiedergeben.

21. Verfahren nach einem der Ansprüche 14 bis 20, bei dem zur Berücksichtigung des Wassergehalts, aus dem vorgegebenen Soll-Ablationsprofil ein in Abhängigkeit von dem Wassergehalt vorkompensiertes Ablationsprofil ermittelt wird und aus dem vorkompensierten Ablationsprofil das Ablationsprogramm erstellt wird.

22. Verfahren nach Anspruch 21, bei dem zur Ermittlung des vorkompensierten Ablationsprofils eine Modifikationsfunktion verwendet wird, die explizit oder implizit von dem Wassergehalt des zu ablatierenden Materials abhängt, und wobei vorzugsweise der Wert der Modifikationsfunktion an einem jeweils vorgegebenen Ort von einer durch das Soll-Ablationsprofil gegebenen Soll-Ablationstiefe an dem Ort abhängt.

23. Verfahren nach einem der Ansprüche 14 bis 22, bei dem aus dem Soll-Ablationsprofil ein vorläufiges Ablationsprogramm erstellt wird und zur Bildung des zu erstellenden Ablationsprogramms in Abhängigkeit von dem Wassergehalt wenigstens ein durch das vorläufige Ablationsprogramm implizit oder explizit vorgegebener Fluence-Wert oder eine durch das vorläufige Ablationsprogramm implizit oder explizit vorgegebene Pulsenergie eines auf einen durch das vorläufige Ablationsprogramm gegebenen Zielort abzugebenden Pulses in Abhängigkeit von dem Wassergehalt an dem Zielort geändert und als Angabe dem Zielort zugeordnet wird.

24. Verfahren nach Anspruch 14, bei dem zur Berücksichtigung der Form des Strahlprofils und/oder der Neigung der Oberfläche (2') aus dem Soll-Ablationsprofil unter Verwendung einer Modifikationsfunktion, die von der Form des Strahlprofils und/oder der Neigung der Oberfläche (2') abhängt, ein in Bezug auf Einflüsse der Form des Strahlprofils und/oder der Neigung der Oberfläche (2') vorkompensiertes Soll-Ablationsprofil ermittelt wird, und daß aus dem ermittelten in Bezug auf Einflüsse der Form des Strahlprofils und/oder der Neigung der Oberfläche (2') vorkompensiertes Soll-Ablationsprofil das Ablationsprogramm erstellt wird.

25. Verfahren nach Anspruch 24 und Anspruch 22, bei dem für wenigstens zwei zu ablatierende Bereiche der Oberfläche (2') jeweils ein Wert der strahl- und/oder neigungsabhängigen Modifikationsfunktion in Abhängigkeit von wenigstens der Form des Strahlprofils und/oder der Neigung der Oberfläche (2') in dem jeweiligen Bereich und ein Wert der wassergehaltabhängigen Modifikationsfunktion ermittelt werden und das vorkompensierte Soll-Ablationsprofil unter Verwendung des Soll-Ablationsprofils und der Werte der Modifikationsfunktionen, insbesondere des Produkts der Modifikationsfunktionen, ermittelt wird.

26. Verfahren zur Bildung von Steuersignalen zur Steuerung eines Lasers (7) einer Laserablationsvorrichtung zur Abgabe eines gepulsten Laserstrahls (3) und/oder einer Ablenkeinrichtung (8) der Laserablationsvorrichtung zur Ablenkung des Laserstrahls (3), um wasserhaltiges Material von einer Oberfläche (2') eines Körpers (2) gemäß einem vorgegebenen Soll-Ablationsprofil mittels Pulsen eines gepulsten Laserstrahls (3) zu ablatieren, bei dem mit dem Erstellungsverfahren nach einem der Ansprüche 14 bis 25 ein Ablationsprogramm für das vorgegebene Soll-Ablationsprofil erstellt wird und entsprechend dem Ablationsprogramm Steuersignale an den Laser (7) und/oder die Ablenkeinrichtung (8) abgegeben werden.

27. Verfahren nach Anspruch 26, bei dem Steuersignale gebildet werden, mittels derer ein einen zur Ablation verwendeten Laserstrahl (3) schwächenden Modulator (16) in seiner Transmission steuerbar ist, und/oder mittels derer die Fluence oder Pulsenergie von dem Laser (7) abgegebener Puls gesteuert wird, und/oder bei dem Steuersignale zur Steuerung einer strahlformenden optischen Einrichtung im Strahlengang des Laserstrahls (3), die zur Einstellung des Strahlquerschnitt dient, gebildet und an die strahlformende optische Einrichtung abgegeben werden.

**28.** Verfahren nach einem der Ansprüche 26 oder 27, bei dem nach der Erstellung eines ersten Teils des Ablationsprogramms und Abgabe entsprechender Steuersignale wenigstens ein weiterer, noch abzuarbeitender Teil des Ablationsprogramms erstellt wird und entsprechende Steuersignale abgegeben werden.

**29.** Verfahren nach einem der Ansprüche 26 bis 28, bei dem ausgehend von dem Soll-Ablationsprofil ein vorläufiges Ablationsprogramm erstellt wird und zur Erstellung des wenigstens einen weiteren Teils des Ablationsprogramms für wenigstens einen durch das vorläufige Abtationsprogramm gegebenen Zielort auf der Oberfläche (2') der Wassergehalt ermittelt wird, und das vorläufige Ablationsprogramm unter Bildung des Ablationsprogramms in Abhängigkeit von dem ermittelten Wassergehalt verändert wird.

**30.** Verfahren zur Ablation von Material von einer Oberfläche (2') eines Brillenglases, einer Kontaktlinse oder eines Linsenimplantates, gemäß einem vorgegebenen Soll-Ablationsprofil mittels eines über die Oberfläche (2') geführten, gepulsten Laserstrahls (3) mit einer vorgegebenen Form des Strahtprofits, bei dem mit einem Erstellungsverfahren nach einem der vorhergehenden Ansprüche, 1 bis 25 ein Ablationsprogramm für die Ablation von Material von der Oberfläche (2') des Brillenglases, der Kontaktlinse bzw. des Linsenimplantats erstellt wird, und Pulse des Laserstrahls (3) entsprechend dem erstellten Ablationsprogramm auf die Oberfläche (2') gelenkt werden.

**31.** Verfahren nach Anspruch 30, bei dem zur Einstellung der Energie oder Fluence eines Pulses der Laserstrahl (3) abgeschwächt oder der zur Abgabe des gepulsten Laserstrahls (3) verwendete Laser gesteuert wird.

**32.** Verfahren nach Anspruch 31, bei dem für wenigstens zwei Pulse deren Energie oder Fluence in Abhängigkeit von der Form des Strahlprofils und der Neigung der Oberfläche (2') in dem jeweiligen Bereich, auf den der jeweilige Puls abgegeben wird, während der Ablation ermittelt wird.

**33.** Computerprogramm mit Programmcode, um das Erstellungsverfahren nach einem der Ansprüche 1 bis 29 durchzuführen, wenn das Programm auf einem Computer (5; 5'; 105; 105') ausgeführt wird.

**34.** Computerprogramm nach Anspruch 33, das als Computerprogrammprodukt ausgebildet ist und auf einem computerlesbaren Datenträger gespeichert ist.

**35.** Vorrichtung zur Erstellung eines Ablationsprogramms für die Ablation von Material von einer Oberfläche (2') gemäß einem Soll-Ablationsprofil durch Abgabe von Pulsen eines gepulsten Laserstrahls (3) auf die Oberfläche (2'), wobei die Vorrichtung eine Datenverarbeitungseinrichtung (5; 105) aufweist, die zur Durchführung eines Erstellungsverfahrens nach einem der Anspruche 1 bis 25 ausgebildet ist und dazu einen Speicher, in dem ein Computerprogramm gemäß Anspruch 33 oder 34 gespeichert ist, und einen Prozessor zur Abarbeitung des Computerprogramms aufweist, mittels dessen das Ablationsprogramm entsprechend einem Erstellungsverfahren gemäß einem der Ansprüche 1 bis 25 erstellbar ist.

**36.** Vorrichtung nach Anspruch 35 mit einer Steuereinrichtung (6; 6'; 106, 106'), mittels derer ein Laser (7; 107) zur Abgabe des Laserstrahls (3) und/oder eine Ablenkeinrichtung (8; 108) zur Ablenkung des Laserstrahls (3) und/oder ein den Laserstrahl (3) schwächender Modulator gemäß einem erstellten Ablationsprogramm ansteuerbar sind.

**37.** Vorrichtung nach einem der Ansprüche 35 oder 36, bei der Datenverarbeitungseinrichtung (5; 105) eine Schnittstelle (13, 13'; 113, 113') zur Eingabe von Daten, die die Form des Strahlprofils des Laserstrahls (3) charakterisieren, umfaßt, und/oder die aufweist: eine Einrichtung (16; 116) zur Erfassung des Strahlprofils des Laserstrahls (3), und/oder eine Einrichtung (9; 109) zur Erfassung von Topographiedaten der zu ablatierenden Oberfläche (2').

**38.** Vorrichtung zur Ablation von Material von einer Oberfläche (2') eines Körpers (2), die einen Laser zur Abgabe eines gepulsten Laserstrahls (3), eine Ablenkeinrichtung zur gesteuerten Ablenkung des Laserstrahls (3) und eine Erstellungsvorrichtung nach einem der Ansprüche 35 bis 37 umfaßt.

**39.** Vorrichtung nach einem der Ansprüche 35 bis 37 die umfaßt:

- eine Schnittstelle (111) zum Erfassen von Daten, die den Wassergehalt des Materials wiedergeben oder aus denen der Wassergehalt ermittelbar ist,
- eine Steuerschnittstelle für eine Meßeinrichtung (109) zur Messung von Daten an dem Körper (2), die den Wassergehalt wiedergeben oder aus denen der Wassergehalt ermittelt werden kann, aufweist, über die Steuerbefehle zur Erfassung von Meßdaten an die Meßeinrichtung (109) abgebbar sind,

- eine Einrichtung zur Erfassung einer Temperatur der Oberfläche aufweist, die zur Übermittlung von erfaßten Temperaturdaten an die Datenverarbeitungseinrichtung über eine Datenverbindung mit der Datenverarbeitungseinrichtung verbunden ist,

- ein mit der Datenverarbeitungseinrichtung (105; 105') verbundenes Spektrometer (109) zur Analyse von von dem zu ablatierenden Bereich des Körpers (2) ausgehender optischer Strahlung aufweist,

- eine Einrichtung zur Durchführung von konfokaler Raman-Spektroskopie aufweist, die zur Übermittlung von erfaßten Spektroskopiedaten an die Datenverarbeitungseinrichtung (105; 105') mit der Datenverarbeitungseinrichtung (105; 105') verbunden ist, und

- eine mit der Datenverarbeitungseinrichtung verbundene Einrichtung zur Detektion von Fluoreszenzstrahlung aufweist, die bei Bestrahlung der zu ablatierenden Oberfläche des Körpers von Material an der Oberfläche des Körpers abgegeben wird.

40. Vorrichtung zur Bildung von Steuersignalen für einen Laser und/oder eine Ablenkeinrichtung einer Laserablationsvorrichtung, um wasserhaltiges Material von einer Oberfläche (2') eines Körpers (2) gemäß einem vorgegebenen Soll-Ablationsprofil mittels Pulsen eines gepulsten Laserstrahls (3) zu ablatieren, wobei die Vorrichtung eine Erstellungsvorrichtung nach einem der Ansprüche 35 bis 37 und 39 zur Erstellung eines Ablationsprogramms aus dem vorgegebenen Soll-Ablationsprofil und eine Steuereinrichtung (106; 106') zur Abgabe von Steuersignalen gemäß dem erstellten Ablationsprogramm an den Laser (107) und/oder die Ablenkeinrichtung (108) zur Ablenkung des von dem Laser (107) abgegebenen Laserstrahls (3) umfaßt.

41. Vorrichtung nach Anspruch 40, bei der die Steuereinrichtung (106') zur Bildung und Abgabe von Steuersignalen zur Ansteuerung eines den Laserstrahl (3) schwächenden Modulators (116) gemäß einem erstellten Ablationsprogramm und/oder zur Bildung und Abgabe von Steuersignalen zur Ansteuerung einer strahlformenden optischen Einrichtung im Strahlengang des Laserstrahls, die zur Einstellung des Strahlquerschnitt dient, gemäß einem erstellten Ablationsprogramm ausgebildet ist.

**Claims**

1. A method for generating an ablation program for ablation of material from a surface (2') of a body (2) according to a predetermined desired ablation profile by emission of pulses of a pulsed laser beam (3) onto the surface (2'), wherein the ablation program is generated from the desired ablation profile **characterized in that** the ablation program comprises at least an indication of a series of target locations or target directions, to which the pulses of the laser beam have to be emitted and that in generating the ablation program the shape of a beam profile of the laser beam (3) is taken into account, wherein the beam profile specifies a gradient of the intensity, of the energy or of the fluence over a cross section of the laser beam (3) close to the surface (2').

2. The method as claimed in claim 1, **characterized in that** the ablation program is generated while taking into account an inclination of the surface (2') to be ablated.

3. The method as claimed in claim 2, wherein in order to consider the inclination of the surface (2') and the shape of the beam profile, the desired ablation profile is used to determine a pre-compensated desired ablation profile as a function of at least the shape of the beam profile and the inclination of the surface (2') at a respective target location on the surface (2'), and the ablation program is generated on the basis of the pre-compensated ideal ablation program.

4. The method as claimed in claim 3, wherein for at least two regions to be ablated from the surface (2') one value of a modification function is respectively determined as a function of at least one of the shape of the beam profile and the inclination of the surface (2') in the respective region and the pre-compensated desired ablation profile is determined using the desired ablation profile and the values of the modification function.

5. The method as claimed in claim 4, wherein the modification function additionally depends on the intensity, the energy, or the fluence of the pulses to be used for ablation.

6. The method as claimed in claim 2, wherein a preliminary ablation program is determined from the desired ablation profile, for energy or fluence of at least one of the pulses to be emitted a desired value is determined depending on the shape of the beam profile, on the inclination of the surface (2') in the region onto which the pulse is to be emitted, and from a prediction of the ablation depth using the preliminary ablation program, and the generated ablation program comprises the target location of the pulse according to the preliminary ablation program and the determined ideal value for the energy or fluence of the pulse.

7. The method as claimed in claim 2, wherein the a preliminary ablation program is determined from the desired ablation profile, the preliminary ablation program is used to predict a predicted ablation profile as a function of the beam profile shape and of the inclination of the surface (2'), and the ablation program to be used is generated using the predicted ablation profile.

8. The method as claimed in claim 7, wherein the predicted ablation profile is determined at at least two locations which are spaced apart from each other by less than the diameter of the laser beam (3) on the surface (2') of the body (2).

9. The method as claimed in claim 7 or 8, wherein the predicted ablation profile and the predetermined desired ablation profile are used to determine a pre-compensated desired ablation profile, and the ablation program to be used is generated from the pre-compensated desired ablation profile.

10. The method as claimed in any one of claims 7 to 9, wherein the ablation program to be used is generated in an iterative manner in that, in an actual iteration step, a preliminary ablation program is determined from a modified desired ablation profile determined in a preceding iteration step, an actual predicted ablation profile is predicted on the basis of the preliminary ablation program as a function of the beam profile shape and of the inclination of the surface (2'), an actual modified desired ablation profile is determined using the actual predicted ablation profile, and the ablation program to be used is generated as a function of the predetermined desired ablation profile and at least one of the modified desired ablation profiles after the last iteration loop.

11. The method as claimed in any one of the preceding Claims, wherein a model is used by means of which the course of the ablation depth can be predicted for a pulse as a function of the shape of the beam profile or wherein the beam profile is measured in order to generate the ablation program.

12. The method as claimed in any one of the preceding claims, wherein topographical data of the surface (2') are acquired in order to generate the ablation program.

13. The method as claimed in any one of the preceding claims, wherein the pre-compensation is effected as a function of an ablation property of the material, which property depends on the individual body, and/or as a function of a change in surface properties and/or material properties to be expected during and/or as a result of ablation.

14. The method as claimed in any one of the preceding claims, wherein the material contains water, wherein the laser beam is guided over the surface during the ablation to be effected, and wherein the ablation program is generated starting from the desired ablation profile, additionally considering a water content of the material to be ablated.

15. The method as claimed in claim 14, wherein in generating the ablation program the water content is taken into consideration as a function of the location on the surface (2') or in a region to be ablated.

16. The method as claimed in claim 14 or 15, wherein a model is used which indicates the dependence of the ablation depth, which is achieved by at least one pulse emitted onto a target location on the surface (2'), or of the ablation volume, which is achieved by at least one pulse emitted onto a target location on the surface (2'), on the water content of the material to be ablated by said pulse.

17. The method as claimed in any one of claims 14 to 16, wherein a further model is used, which indicates, for a predetermined region of the material, the influence that pulses of the pulsed laser beam (3) impinging on this region or on adjacent regions have on the water content.

18. The method as claimed in any one of claims 14 to 17, which uses a model for the water content or the change in the water content of the material as a function of at least the number and/or the position of pulses previously emitted onto the same location and/or adjacent locations in order to take the water content into consideration when generating the ablation program.

19. The method as claimed in any one of claims 14 to 18, wherein the water content is determined from data measured on the body (2), in particular wherein data which indicate the temperature of the surface (2') or properties of optical radiation originating from the material in the region to be ablated from the body (2) are used to determine the water content.

**20.** The method as claimed in claim 19, wherein data which are available by confocal Raman spectroscopy of optical radiation from the surface (2') and/or data which indicate the properties of fluorescent radiation originating from the region to be ablated from the body (2) are used to determine the water content.

**21.** The method as claimed in any one of claims 14 to 20, wherein the water content is takein into account by determining from the predetermined desired ablation profile a pre-compensated ablation profile as a function of the water content and generating the ablation program from the pre-compensated ablation profile.

**22.** The method as claimed in claim 21, wherein a modification function is used in order to determine the pre-compensated ablation profile, said modification function depending explicitly or implicitly on the water content of the material to be ablated and wherein the value of the modification function at a respectively predetermined location depends on a desired ablation depth given by the desired ablation profile at said location.

**23.** The method as claimed in any one of claims 14 to 22, wherein a preliminary ablation program is generated from the desired ablation profile and, in order to generate the ablation program as a function of the water content, at least one fluence value implicitly or explicitly given by the preliminary ablation program, or a pulse energy of a pulse to be emitted onto the target location implicitly or explicitly given by the preliminary ablation program is modified as a function of the water content at the target location and is assigned to the target location as an indication.

**24.** The method as claimed in claim 2314 wherein in order to consider the shape of the beam profile and/or the inclination of the surface (2'), a desired ablation profile, which is pre-compensated with respect to the influences of the beam profile shape and/or of the inclination of the surface (2'), is determined from the desired ablation profile, using a modification function which depends on the shape of the beam profile and/or on the inclination of the surface (2'), and wherein the ablation program is generated from the determined desired ablation profile which has been pre-compensated with respect to the influences of the beam profile shape and/or of the inclination of the surface (2').

**25.** The method as claimed in claims 24 and 22, wherein for at least two regions of the surface (2') to be ablated one value each of the beam-dependent and/or inclination-dependent modification function is/are determined as a function of at least the shape of the beam profile and/or the inclination of the surface (2') in the respective region, and a value of the water content-dependent modification function is determined, and the pre-compensated desired ablation profile is determined using the desired ablation profile and the values of the modification functions, in particular of the modification functions' product.

**26.** A method for forming control signals for controlling a laser (7) of a laser ablation device to emit a pulsed laser beam (3) and/or for controlling a deflecting device (8) of the laser ablation device to deflect the laser beam (3) in order to ablate water-containing material from a surface (2') of a body (2) according to a predetermined desired ablation profile by means of pulses of a pulsed laser beam (3), wherein the generating method as claimed in any one of claims 14 to 25 is used to generate an ablation program for the predetermined desired ablation profile and control signals are output to the laser (7) and/or to the deflecting device (8) according to the ablation program.

**27.** The method as claimed in claim 26, wherein the control signals are formed by means of which the transmission of a modulater (16) attenuating the laser beam (3) can be controlled and/or by means of which the fluence or the pulse energy of pulses emitted by the laser (7) is controlled, and/or wherein control signals controlling a beam-shaping optical device located in the beam path of the laser beam (3), said device serving to adjust the beam cross-section, are formed and are output to the beam-shaping optical device.

**28.** The method as claimed in claims 26 or 27, wherein after generating a first part of the ablation program and emission of corresponding control signals at least one further executable part of the ablation program is generated and corresponding control signals are output.

**29.** The method as claimed in any one of claims 26 to 28, wherein a preliminary ablation program is generated on the basis of the desired ablation profile and the water content is determined in order to generate the at least one further part of the ablation program for at least one target location on the surface (2') given by the preliminary ablation program, and the preliminary ablation program is changed by generating the ablation program as a function of the determined water content.

**30.** A method for ablation of material from a surface (2') of a spectacle lens, a contact lens or an intraocular lens according to a predetermined desired ablation profile by means of a pulsed laser beam (3), which is guided over the surface (2') and has a beam profile with a predetermined shape, wherein an ablation program for ablating material from the surface (2') of the spectacle lens, the contact lens or the intraocular lens is generated by a generating method as claimed in any one of the preceding claims 1 to 25, and pulses of the laser beam (3) are directed onto the surface (2') according to the generated ablation program.

**31.** The method as claimed in claim 30, wherein the laser beam (3) is attenuated in order to set the energy or fluence of a pulse or the laser used to emit the pulsed laser beam (3) is controlled so as to set the energy or fluence of a pulse.

**32.** The method as claimed in claim 31, wherein for at least two pulses the energy or fluence of said pulses is determined during ablation as a function of the shape of the beam profile and of the inclination of the surface (2') in the region onto which the respective pulse is emitted.

**33.** A computer program comprising program code to carry out the generating method as claimed in any one of claims 1 to 29, when the program is being executed on a computer (5; 5'; 105; 105').

**34.** A computer program according to claim 33 being provided as computer program product and being stored on a computer-readable data carrier.

**35.** An apparatus for generating an ablation program for ablation of material from a surface (2') according to a desired ablation profile by emission of pulses of a pulsed laser beam (3) onto the surface (2'), said apparatus comprising a data processing device (5; 105) which is provided to carry out a generating method according to any one of claims 1 to 25 and, for this purpose, comprises a memory in which a computer program according to claim 33 or 34 is stored, and a processor for executing the computer program by which an ablation program can be generated according to a method for generating according to any of the claims 1 to 25.

**36.** The device as claimed in claim 35, comprising a control unit (6; 6'; 106, 106') by means of which a laser (7; 107) for emitting the laser beam (3) and/or a deflecting device (8; 108) for deflecting the laser beam (3) and/or a modulator attenuating the laser beam (3) can be controlled according to an ablation program generated.

**37.** The apparatus as claimed in any one of claims 35 or 36, wherein the data processing device (5; 105) comprises an interface (13, 13'; 113, 113') for input of data which characterize the shape of the beam profile of the laser beam (3) and/or wherein the apparatus comprises a device (16; 116) for detection of the beam profile of the laser beam (3) and/or a device (9; 109) for acquisition of topographical data of the surface (2') to be ablated.

**38.** An apparatus for ablation of material from a surface (2') of a body (2), said apparatus comprising a laser for emission of a pulsed laser beam (3), a deflecting device for controlled deflection of the laser beam (3), and a generating apparatus as claimed in any one of claims 35 to 37.

**39.** The apparatus as claimed in any one of claims 35 to 37, which comprises:

- an interface (111) for acquiring data which indicate the water content of the material or from which the water content can be determined,
- a control interface for a measuring device (109) measuring data on a body (2), which data indicate the water content or from which the water content can be determined, said control interface enabling the output of control commands for acquisition of measurement data to the measuring device (109),
- a device for detecting a temperature of the surface, said device being connected to the data processing device via a data link so as to transmit acquired temperature data to the data processing device,
- a spectrometer (109) for analysis of optical radiation coming from the region to be ablated from the body (2), said spectrometer (109) being connected to the data processing device (105; 105'),
- a device for carrying out confocal Raman spectroscopy, which device is connected to the data processing device (105; 105') so as to transmit acquired spectroscopic data to the data processing device (105; 105'), and
- a device for detection of fluorescent radiation emitted by material at the surface of the body upon irradiation of the surface to be ablated from the body, said detection device being connected to the data processing device.

**40.** An apparatus for forming control signals for a laser and/or for a deflecting device of a laser ablation device in order to ablate water-containing material from a surface (2') of a body (2) according to a predetermined desired ablation profile by means of pulses from a pulsed laser beam (3), wherein the apparatus comprises a generating apparatus as claimed in any one of claims 35 to 37 and 39 for generating an ablation program from the predetermined desired ablation profile and a control unit (106; 106') for outputting control signals according to the generated ablation program to the laser (107) and/or the deflecting device (108) for deflecting of the laser beam (3) emitted by the laser (107).

**41.** The apparatus as claimed in claim 40, wherein the control unit (106') is provided to generate and output control signals to control a modulator (116) attenuating the laser beam (3) according to a generated ablation program and/or to form and output control signals to control a beam-shaping optical device in the beam path of the laser beam according to a generated ablation program, said optical device serving to adjust the beam cross-section.

**Revendications**

**1.** Procédé destiné à l'élaboration d'un programme d'ablation en vue d'une ablation de matière sur une surface (2') d'un corps (2) selon un profil d'ablation théorique prédéfini, par l'émission d'impulsions d'un rayon laser (3) pulsé sur la surface (2'), le programme d'ablation étant élaboré à partir du profil d'ablation théorique, **caractérisé en ce que** le programme d'ablation comprend au moins une donnée d'une succession de différents sites de visée ou directions de visée sur lesquels ou dans lesquelles doivent être émises les impulsions du rayon laser, et **en ce que** la forme d'un profil du rayon laser (3) est prise en compte lors de l'élaboration du programme d'ablation, le profil du rayon indiquant un tracé de l'intensité, de l'énergie ou de la fluence sur une section du rayon laser (3) proche de la surface (2').

**2.** Procédé selon la revendication 1, **caractérisé en ce que** le programme d'ablation est également élaboré en fonction d'une inclinaison de la surface (2') à ablater.

**3.** Procédé selon la revendication 2, dans lequel un profil d'ablation théorique précompensé est déterminé pour tenir compte de l'inclinaison de la surface (2') et de la forme du profil du rayon à partir du profil d'ablation théorique en fonction au moins de la forme du profil du rayon et de l'inclinaison de la surface (2') au niveau d'un site de visée respectif sur la surface (2'), et le programme d'ablation est élaboré à partir du profil d'ablation théorique précompensé.

**4.** Procédé selon la revendication 3, dans lequel pour au moins deux zones à ablater sur la surface (2') est déterminée respectivement une valeur d'une fonction de modification en fonction au moins de la forme du profil du rayon et de l'inclinaison de la surface (2') dans la zone concernée, et le profil d'ablation théorique précompensé est déterminé moyennant l'utilisation du profil d'ablation théorique et des valeurs de la fonction de modification.

**5.** Procédé selon la revendication 4, dans lequel la fonction de modification dépend, en plus, de l'intensité, de l'énergie ou de la fluence des impulsions à utiliser lors de l'ablation.

**6.** Procédé selon la revendication 2, dans lequel un programme d'ablation provisoire est déterminé à partir du profil d'ablation théorique, pour au moins une des impulsions à émettre est déterminée une valeur de consigne pour l'énergie ou la fluence desdites impulsions en fonction de la forme du profil du rayon, de l'inclinaison de la surface (2') dans la zone, dans laquelle doit être émise l'impulsion, et d'une prédiction de la profondeur d'ablation lors de l'utilisation du programme d'ablation provisoire, et le programme d'ablation élaboré comprend le site de visée de l'impulsion selon le programme d'ablation provisoire et la valeur de consigne déterminée pour l'énergie ou la fluence de l'impulsion.

**7.** Procédé selon la revendication 2, dans lequel un programme d'ablation provisoire est déterminé à partir du profil d'ablation théorique, un profil d'ablation prédit en fonction de la forme du profil du rayon et de l'inclinaison de la surface (2') est prédit moyennant l'utilisation du programme d'ablation provisoire, et le programme d'ablation à utiliser est élaboré moyennant l'utilisation du profil d'ablation prédit.

**8.** Procédé selon la revendication 7, dans lequel le profil d'ablation prédit est déterminé en au moins deux points qui sont écartés l'un de l'autre sur la surface (2') du corps (2) sur une distance inférieure au diamètre du rayon laser (3).

**9.** Procédé selon la revendication 7 ou 8, dans lequel un profil d'ablation théorique précompensé est déterminé moyennant l'utilisation du profil d'ablation prédit et du profil d'ablation théorique prédéfini, et le programme d'ablation à utiliser est élaboré à partir du profil d'ablation théorique précompensé.

**10.** Procédé selon l'une quelconque des revendications 7 à 9, dans lequel l'élaboration du programme d'ablation à utiliser est effectuée de manière itérative, du fait qu'un programme d'ablation provisoire est déterminé dans une boucle d'itération actuelle à partir d'un profil d'ablation théorique modifié, déterminé dans une boucle d'itération antérieure, un profil d'ablation prédit actuel est prédit à partir du programme d'ablation provisoire en fonction de la forme du profil du rayon, un profil d'ablation théorique modifié actuel est déterminé moyennant l'utilisation du profil d'ablation prédit actuel, et, après la dernière boucle d'itération, le programme d'ablation à utiliser est élaboré en fonction du profil d'ablation théorique prédéfini et au moins un des profils d'ablation théoriques modifiés.

**11.** Procédé selon l'une quelconque des revendications précédentes, dans lequel est utilisé un modèle, au moyen duquel le tracé de la profondeur d'ablation peut être prédit pour une impulsion en fonction de la forme du profil du rayon, ou dans lequel le profil du rayon est mesuré pour l'élaboration du programme d'ablation.

**12.** Procédé selon l'une quelconque des revendications précédentes, dans lequel des données de topographie de la surface (2') sont collectées pour l'élaboration du programme d'ablation.

**13.** Procédé selon l'une quelconque des revendications précédentes, dans lequel la précompensation est effectuée en fonction d'une propriété d'ablation de la matière, laquelle dépend du corps individuel, et/ou en fonction d'une variation à escompter des propriétés de la surface et/ou de la matière pendant l'ablation et/ou par suite de l'ablation.

**14.** Procédé selon l'une quelconque des revendications précédentes, dans lequel la matière contient de l'eau, dans lequel le rayon laser est guidé au-dessus de la surface pendant l'ablation à effectuer, et dans lequel le programme d'ablation est élaboré à partir du profil d'ablation théorique et en plus en tenant compte d'une teneur en eau de la matière à ablater.

**15.** Procédé selon la revendication 14, dans lequel lors de l'élaboration du programme d'ablation, la teneur en eau est prise en compte en fonction du site sur la surface (2') ou dans une zone à ablater.

**16.** Procédé selon la revendication 14 ou 15, dans lequel est utilisé un modèle qui reproduit la dépendance la dépendance entre la profondeur d'ablation obtenue par au moins une impulsion émise sur un site de visée sur la surface (2') ou le volume d'ablation obtenu par au moins une impulsion émise sur un site de visée sur la surface (2') et la teneur en eau de la matière à ablater au moyen de l'impulsion.

**17.** Procédé selon l'une quelconque des revendications 14 à 16, dans lequel est utilisé un modèle supplémentaire qui, pour une zone prédéfinie de la matière, reproduit l'influence que les impulsions du rayon laser (3) pulsé, projetées sur ladite zone ou des zones adjacentes, exercent sur la teneur en eau.

**18.** Procédé selon l'une quelconque des revendications 14 à 17, dans lequel, afin de tenir compte de la teneur en eau lors de l'élaboration du programme d'ablation, il est utilisé un modèle pour la teneur en eau ou pour la variation de la teneur en eau de la matière en fonction au moins du nombre et/ou de la position des impulsions émises précédemment sur le même site et/ou sur des sites adjacents.

**19.** Procédé selon l'une quelconque des revendications 14 à 18, dans lequel la teneur en eau est déterminée à partir de données mesurées sur le corps (2), en particulier en utilisant, pour la détermination de la teneur en eau, des données qui reproduisent la température de la surface (2') ou les propriétés du rayonnement optique qui est émis par la matière dans la zone à ablater du corps (2).

**20.** Procédé selon la revendication 19, dans lequel pour la détermination de la teneur en eau sont utilisées des données qui sont obtenues par la spectroscopie Raman confocale du rayonnement optique partant de la surface (2') et/ou qui reproduisent des propriétés du rayonnement de fluorescence qui est émis par la zone à ablater du corps (2), et/ou qui reproduisent l'indice de réfraction dans la matière.

**21.** Procédé selon l'une quelconque des revendications 14 à 20, dans lequel, afin de tenir compte de la teneur en eau à partir du profil d'ablation théorique prédéfini, un profil d'ablation précompensé est déterminé en fonction de la teneur en eau, et le programme d'ablation est élaboré à partir du profil d'ablation précompensé.

**22.** Procédé selon la revendication 21, dans lequel pour déterminer le profil d'ablation précompensé est utilisée une fonction de modification qui dépend explicitement ou implicitement de la teneur en eau de la matière à ablater, et dans lequel de préférence la valeur de la fonction de modification sur un site prédéfini dans chaque cas dépend d'une profondeur d'ablation théorique sur ledit site, définie par le profil d'ablation théorique.

23. Procédé selon l'une quelconque des revendications 14 à 22, dans lequel un programme d'ablation provisoire est élaboré à partir du profil d'ablation théorique et, pour la constitution du programme d'ablation à élaborer en fonction de la teneur en eau, au moins une valeur de fluence prédéfinie implicitement ou explicitement par le programme d'ablation provisoire ou une énergie, prédéfinie implicitement ou explicitement par le programme d'ablation provisoire, d'une impulsion à émettre sur un site de visée défini par le programme d'ablation provisoire sont modifiées en fonction de la teneur en eau au niveau du site de visée et en tant que données sont associées au site de visée.

24. Procédé selon la revendication 14, dans lequel, afin de tenir compte de la forme du profil du rayon et/ou de l'inclinaison de la surface (2'), un profil d'ablation théorique précompensé en relation avec les influences de la forme du profil du rayon et/ou de l'inclinaison de la surface (2') est déterminé à partir du profil d'ablation théorique moyennant l'utilisation d'une fonction de modification qui dépend de la forme du profil du rayon et/ou de l'inclinaison de la surface (2'), et dans lequel le programme d'ablation est élaboré à partir du profil d'ablation théorique précompensé, déterminé en relation avec les influences de la forme du profil du rayon et/ou de l'inclinaison de la surface (2').

25. Procédé selon la revendication 24 et la revendication 22, dans lequel pour au moins deux zones à ablater sur la surface (2') sont déterminées respectivement une valeur de la fonction de modification, qui dépend du rayon et/ou de l'inclinaison, en fonction au moins de la forme du profil du rayon et/ou de l'inclinaison de la surface (2') dans la zone respective et une valeur de la fonction de modification, qui dépend de la teneur en eau, et le profil d'ablation théorique précompensé est déterminé moyennant l'utilisation du profil d'ablation théorique et des valeurs des fonctions de modification, en particulier du produit des fonctions de modification.

26. Procédé pour générer des signaux de commande destinés à commander un laser (7) d'un dispositif d'ablation par laser, destiné à émettre un rayon laser (3) pulsé, et/ou un dispositif de déflexion (8) du dispositif d'ablation par laser, destiné à défléchir le rayon laser (3), afin de procéder à l'ablation d'une matière contenant de l'eau sur une surface (2') d'un corps (2) selon un profil d'ablation théorique prédéfini, au moyen des impulsions d'un rayon laser (3) pulsé, dans lequel procédé, au moyen du procédé d'élaboration selon l'une des revendications 14 à 25, est élaboré un programme d'ablation pour le profil d'ablation théorique prédéfini et des signaux de commande sont délivrés, conformément au programme d'ablation, vers le laser (7) et/ou le dispositif de déflexion (8).

27. Procédé selon la revendication 26, dans lequel sont générés des signaux de commande, au moyen desquels un modulateur (16), qui atténue le rayon laser (3) utilisé pour l'ablation, peut être commandé sur le plan de sa transmission et/ou au moyen desquels la fluence ou l'énergie de l'impulsion émise par le laser (7) sont commandées, et/ou dans lequel sont générés des signaux de commande, destinés à commander un dispositif optique formant le rayon, dans la trajectoire du rayon laser (3), lequel est utilisé pour régler la section du rayon, et lesdits signaux de commande sont envoyés vers le dispositif optique formant le rayon.

28. Procédé selon l'une des revendications 26 ou 27, dans lequel, après l'élaboration d'une première partie du programme d'ablation et l'émission de signaux de commande correspondants, est élaborée au moins une partie supplémentaire, encore à affiner, du programme d'ablation et des signaux de commande correspondants sont délivrés.

29. Procédé selon l'une quelconque des revendications 26 à 28, dans lequel un programme d'ablation provisoire est élaboré à partir du profil d'ablation théorique et, pour l'élaboration de ladite au moins une partie supplémentaire du programme d'ablation, la teneur en eau est déterminée pour au moins un site de visée sur la surface (2'), défini par le programme d'ablation provisoire, et le programme d'ablation provisoire est modifié moyennant l'élaboration du programme d'ablation en fonction de la teneur en eau déterminée.

30. Procédé pour l'ablation de matière sur une surface (2') d'un verre de lunetterie, d'une lentille de contact ou d'un implant lenticulaire, selon un profil d'ablation théorique prédéfini, au moyen d'un rayon laser (3) pulsé, guidé au-dessus de la surface (2'), avec une forme prédéfinie du profil du rayon, dans lequel procédé, au moyen d'un procédé d'élaboration selon l'une des revendications précédentes 1 à 25, est élaboré un programme d'ablation pour l'ablation de la matière sur la surface (2') du verre de lunetterie, de la lentille de contact ou de l'implant lenticulaire, et les impulsions du rayon laser (3) sont dirigées sur la surface (2') conformément au programme d'ablation élaboré.

31. Procédé selon la revendication 30, dans lequel, pour le réglage de l'énergie ou de la fluence d'une impulsion, le rayon laser (3) est atténué ou le laser utilisé pour émettre le rayon laser (3) pulsé est commandé.

**32.** Procédé selon la revendication 31, dans lequel l'énergie ou la fluence pour au moins deux impulsions sont déterminées, pendant l'ablation, en fonction de la forme du profil du rayon et/ou de l'inclinaison de la surface (2') dans la zone respective, sur laquelle est émise l'impulsion respective.

**33.** Programme d'ordinateur avec un code programme pour la mise en oeuvre du procédé d'élaboration selon l'une quelconque des revendications 1 à 29, lorsque le programme est exécuté sur un ordinateur (5; 5'; 105; 105').

**34.** Programme d'ordinateur selon la revendication 33 qui est conçu sous la forme d'un produit programme d'ordinateur et est stocké sur un support de données propre à être lu par un ordinateur.

**35.** Dispositif destiné à l'élaboration d'un programme d'ablation pour une ablation de matière sur une surface (2') d'un corps (2) selon un profil d'ablation théorique prédéfini, par l'émission d'impulsions d'un rayon laser (3) pulsé sur la surface (2'), ledit dispositif comportant un dispositif de traitement des données (5 ; 105), qui est configuré pour la mise en oeuvre d'un procédé d'élaboration selon l'une des revendications 1 à 25 et qui, à cet effet, comporte une mémoire, dans laquelle est stocké un programme d'ordinateur selon la revendication 33 ou 34, et un processeur pour l'affinement du programme d'ordinateur, au moyen duquel le programme d'ablation peut être élaboré conformément au procédé d'élaboration selon l'une quelconque des revendications 1 à 25.

**36.** Dispositif selon la revendication 35, comportant un dispositif de commande (6 ; 6' ; 106, 106'), au moyen duquel il est possible de commander, selon un programme d'ablation élaboré, un laser (7 ; 107) destiné à émettre un rayon laser (3), et/ou un dispositif de déflexion (8 ; 108) destiné à défléchir le rayon laser (3), et/ou un modulateur atténuant le rayon laser (3).

**37.** Dispositif selon la revendication 35 ou 36, dans lequel le dispositif de traitement des données (5 ; 105) comprend une interface (13, 13' ; 113, 113') pour saisir des données qui caractérisent la forme du profil du rayon laser (3), et/ou qui comporte un dispositif (16 ; 116) destiné à détecter le profil du rayon laser (3), et/ou un dispositif (9 ; 109) destiné à détecter les données de topographie de la surface (2') à ablater.

**38.** Dispositif destiné à l'ablation de matière sur une surface (2') d'un corps (2), lequel comporte un laser destiné à émettre un rayon laser (3) pulsé, un dispositif de déflexion pour la déflexion commandée du rayon laser (3) et un dispositif d'élaboration selon l'une quelconque des revendications 35 à 37.

**39.** Dispositif selon l'une quelconque des revendications 35 à 37, lequel comprend :

- une interface (111) destinée à saisir des données qui reproduisent la teneur en eau de la matière ou à partir desquelles peut être déterminée la teneur en eau,
- une interface de commande pour un dispositif de mesure (109) destiné à mesurer des données au niveau du corps (2) qui reproduisent la teneur en eau de la matière ou à partir desquelles peut être déterminée la teneur en eau, par l'intermédiaire de laquelle des instructions pour la saisie des données de mesure peuvent être envoyées vers le dispositif de mesure (109),
- un dispositif destiné à enregistrer une température de la surface, lequel est relié au dispositif de traitement des données via une ligne de transmission des données en vue de transmettre les données de température enregistrées vers le dispositif de traitement des données,
- un spectromètre (109) relié au dispositif de traitement des données (105 ; 105') pour l'analyse du rayonnement optique émis par la zone à ablater du corps (2),
- un dispositif destiné à la mise en oeuvre de la spectroscopie Raman confocale, lequel est relié au dispositif de traitement des données (105 ; 105') en vue de transmettre les données de spectroscopie enregistrées vers le dispositif de traitement des données (105 ; 105'), et
- un dispositif, relié au dispositif de traitement des données et destiné à détecter le rayonnement de fluorescence qui est émis par la matière à la surface du corps au moment de l'irradiation de la surface à ablater du corps.

**40.** Dispositif destiné à générer des signaux de commande pour un laser et/ou un dispositif de déflexion d'un dispositif d'ablation par laser pour l'ablation d'une matière contenant de l'eau sur une surface (2') d'un corps (2), selon un profil d'ablation théorique prédéfini, au moyen des impulsions d'un rayon laser (3) pulsé, ledit dispositif comprenant un dispositif d'élaboration selon l'une quelconque des revendications 35 à 37 et 39 destiné à l'élaboration d'un programme d'ablation à partir du profil d'ablation théorique prédéfini, et un dispositif de commande (106 ; 106') destiné à délivrer des signaux de commande conformément au programme d'ablation élaboré vers le laser (107) et/ou le dispositif de déflexion (108) en vue de défléchir le rayon laser (3) émis par le laser (107).

**41.** Dispositif selon la revendication 40, dans lequel le dispositif de commande (106') est configuré pour générer et délivrer des signaux de commande pour la commande d'un modulateur (116), atténuant le rayon laser (3), selon un programme d'ablation élaboré, et/ou pour générer et délivrer des signaux de commande pour la commande d'un dispositif optique formant le rayon dans la trajectoire du rayon laser, lequel est destiné à régler la section du rayon selon un programme d'ablation élaboré.

Fig. 1

Fig. 3

Einrichtung zur Erfassung von Oberflächentopographiedaten — 9

10 — Einrichtung zur Ermittlung des Soll-
Ablationsprofils

Schnittstelle f.
man. Eingabe d.
Oberflächentopographiedaten

Schnittstelle f.
Oberflächentopographiedaten

Schnittstelle f.
man. Eingabe
d. Strahlparameter

Schnittstelle f.
Daten d. Strahlparameter

Schnittstelle f.
man. Eingabe d.
Soll-Ablationsprofils

Schnittstelle f.
Daten d. Soll-
Ablationsprofils

11

11'

13

13'

12

12'

Schnittstelle f.
Eingabe d. Ursprungskoordinaten

11"

Erstellung d.
Ablationsprogramms

Steuereinheit

Schnittstelle f.
Eingabe d. Ursprungskoordinaten

12"

5

6

7

Laser

14

15

8

3

2

Fig. 2

EP 1 848 389 B1

S10

Ermittlung von
Oberflächentopographiedaten

Ermittlung der Oberflächenneigungen — S12

Ermittlung des Soll-
Ablationsprofils

Einlesen des Soll-
Ablationsprofils — S16

S14

Einlesen der
Strahlparameter — S18

Eingabe der zu verwendenden Fluence — S20

Ermittlung der Werte der
Modifikationsfunktion — S22

Ermittlung des modifizierten
bzw. vorkompensierten Soll-
Ablationsprofils — S24

Erstellung eines zu verwendenden Ablationsprogramms auf der Basis des modifizierten Soll-Ablationsprofils, der Fluence und der Strahlparameter — S26

Ausgabe von Steuerbefehlen
an den Laser und/oder die
Ablenkeinrichtung gemäß dem
Ablationsprogramm — S28

Fig. 4

Fig. 5

Fig. 6

Fig. 7

S10

Ermittlung von
Oberflächentopographiedaten

Ermittlung der Oberflächenneigungen — S12

S14

Ermittlung des Soll-
Ablationsprofils

Einlesen des Soll-
Ablationsprofils — S16

Einlesen der Strahlparameter und einer voreingestellten Fluence — S30

Erstellung eines vorläufigen Ablationsprogramms auf der Basis des Soll-
Ablationsprofils, der voreingestellten
Fluence und der Strahlparameter — S32

Zur Erstellung des zu verwendenden Ablationsprogramms:
Ermittlung einer modifizierten
Fluence für jeden Puls — S34

Ausgabe von Steuerbefehlen an den Laser und/oder
die Ablenkeinrichtung gemäß Ablationsprogramm — S36

Fig. 8

| Einrichtung zur Erfassung von Oberflächentopographiedaten | 9 | 10 | Einrichtung zur Ermittlung des Soll-Ablationsprofils |

| Schnittstelle f. man. Eingabe d. Oberflächentopographiedaten | Schnittstelle f. Oberflächentopographiedaten | Schnittstelle f. man. Eingabe d. Strahlparameter | Schnittstelle f. Daten d. Strahlparameter | Schnittstelle f. man. Eingabe d. Soll-Ablationsprofils | Schnittstelle f. Daten d. Soll-Ablationsprofils |

11    11'    13    13'    12    12'

| Schnittstelle f. Eingabe d. Ursprungskoordinaten | Erstellung d. Ablationsprogramms | Schnittstelle f. Eingabe d. Ursprungskoordinaten |

11"    12"

Steuereinheit

5    6'

27    15    8    3

7    Laser    14    2

Fig. 9

**Fig. 10**

- S10 — Ermittlung von Oberflächentopographiedaten
- S12 — Ermittlung der Oberflächenneigungen
- S14 — Ermittlung des Soll-Ablationsprofils
- S16 — Eingabe des Soll-Ablationsprofils $D_{Soll,1}$
- S18 — Eingabe der Strahlparameter
- S20 — Eingabe der zu verwendenden Fluence
- S38 — $j=1$
- S40 — Erstellung eines vorläufigen Ablationsprogramms aus $D_{Soll,j}$
- S42 — Berechnung eines aktuellen vorhergesagten Ist-Ablationsprofils $D_{Ij}$ auf der Basis des aktuellen vorläufigen Ablationsprogramms
- S44 — Berechnung und Speicherung einer Teilmodifikationsfunktion $K_j = D_{Ij}/D_{Soll,1}$
- S46 — $j=j_{max}$ oder Für alle $(x,y)$ $|K_j(x,y)-1| < e$
- NEIN
- S48 — Bilde neues, modifiziertes Soll-Ablationsprofil $D_{Soll,j+1} = D_{Soll,j}/K_j$
- S50 — $j=j+1$
- JA
- S52 — Ermittle Modifikationsfunktion $K(x,y) = K_1(x,y)...K_j(x,y)$
- S54 — Ermittle vorkompensiertes Soll-Ablationsprofil $D_{Soll, mod} = D_{Soll,1}/K$
- S56 — Erstellung des zu verwendenden Ablationsprogramms aus $D_{Soll,mod}$
- S58 — Ausgabe von Steuerbefehlen an den Laser und/oder die Ablenkeinrichtung gemäß dem Ablationsprogramm

Fig. 11A

Fig. 11B

Fig. 12

Einrichtung zur Er-mittlung des Soll-Ablationsprofils — 110

Schnittstelle f. Daten d. Soll-Ablationsprofils — 112'

Schnittstelle f. man. Eingabe d. Soll-Ablations-profils — 112

Schnittstelle f. Strahlparameter — 113

Schnittstelle f. Daten zur Er-mittlung des Wassergehalts — 111

Erstellung d. Ablationsprogramms — 106

Steuereinrichtung — 105

Laser — 107

115, 108, 114, 109, 3, 2

Fig. 13

Fig. 14

Ermittlung des Soll-
Ablationsprofils

S110

Einlesen eines Soll-
Ablationsprofils

S112

Messung von Daten zur
Ermittlung des Wassergehalts der Cornea

S114

Ermittlung und Speicherung des Wassergehalts a der Cornea vor Ablation

S116

Einlesen von Strahlparametern einschließlich einer voreingestellten Fluence

S118

Ermittlung eines vorkompensierten Soll-
Ablationsprofils durch Ermittlung vorkompensierter Ablationstiefen für alle Stützstellen auf der Bezugsoberfläche

S120

Erstellung eines Ablationsprogramms aus
dem vorkompensierten Soll-Ablationsprofil

S122

Bildung und Abgabe von Steuersignalen an den Laser und/oder die Ablenkeinrichtung gemäß dem erstellten
Ablationsprogramm

S124

Fig. 15

Ermittlung des Soll-
Ablationsprofils

S110

Einlesen eines Soll-
Ablationsprofils — S112

Messung von Daten zur
Ermittlung des Wassergehalts der Cornea

S114

S116

Ermittlung des Wassergehalts
a der Cornea vor Ablation

Einlesen der Strahlparameter einschließlich einer
voreingestellten Fluence

S118

Erstellung eines vorläufigen Ablationsprogramms auf der Basis des Soll-
Ablationsprofils, der voreingestellten
Fluence und der Strahlparameter

S126

Sukzessive Bildung und Ausgabe von Steuerbefehlen an den Laser und/oder die Ablenkeinrichtung nach Modifizierung der Fluence entsprechend dem aktuellen Wassergehalt

S128

Fig. 16

Ermittlung eines Zielorts $(x_i, y_i)$ gemäß
vorläufigem Ablationsprogramm

S128a

Messung von Daten zur Ermittlung
des Wassergehalts am Zielort $(x_i, y_i)$

S128b

Ermittlung des Wassergehalts H am Zielort $(x_i, y_i)$

S128c

Ermittlung der geänderten Fluence für den
Zielort $(x_i, y_i)$ unter Erstellung des zu verwendenden Ablationsprogramms

S128d

Fig. 17

Fig. 18

EP 1 848 389 B1

117 — Einrichtung zur Erfassung von Oberflächentopographiedaten

110 — Einrichtung zur Ermittlung des Soll-Ablationsprofils

113

Schnittstelle f. Oberflächentopographiedaten

Schnittstelle f. Strahlparameter

Schnittstelle f. man. Eingabe d. Soll-Ablationsprofils

Schnittstelle f. Daten d. Soll-Ablationsprofils

112

112'

118

Schnittstelle f. Daten zur Ermittlung des Wassergehalts

Erstellung d. Ablationsprogramms

Schnittstelle f. Eingabe d. Ursprungskoordinaten

Steuereinrichtung

111

106

105

107

115

108

3

Laser

2

114

109

Fig. 19

Ermittlung des Soll-
Ablationsprofils

S110

Einlesen eines Soll-
Ablationsprofils

S112

Messung von Daten zur
Ermittlung des Wassergehalts der Cornea

S114

Ermittlung und Speicherung des Wassergehalts a der Cornea vor der Ablation

S116

Einlesen von Strahlparametern einschließlich einer voreingestellten Fluence

S118

Ermittlung von Oberflächentopographiedaten

S130

Ermittlung der Oberflächenneigungen

S132

Ermittlung eines vorkompensierten Soll-
Ablationsprofils durch Ermittlung vorkompensierter Ablationstiefen für alle Stützstellen auf der Bezugsoberfläche

S120

Erstellung eines Ablationsprogramms aus
dem vorkompensierten Soll-Ablationsprofil

S122

Bildung und Abgabe von Steuersignalen an den Laser und/oder die Ablenkeinrichtung gemäß dem erstellten
Ablationsprogramm

S124

Fig. 20

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0185075 A
- WO 0185075 A1
- WO 0187201 A1
- WO 0150945 A1
- WO 9527534 A
- DE 19727573 C1
- EP 1060710 A2
- US 6666855 B
- WO 0112113 A1
- WO 0108075 A1
- WO 9920429 A1
- DE 19623749 A1

**In der Beschreibung aufgeführte Nicht-Patentliteratur**

- Refraktive Chirurgie der Hornhaut. Georg Thieme Verlag, 2000, 150
- **R. Srinivasan.** Ablation of Polymers and Biological Tissue by Ultraviolet Lasers. *Science,* 31. Oktober 1986, vol. 234, 559-565
- **Brian T. Fischer et al.** Assessment of Transient Changes in Corneal Hydration Using Confocal Raman Spectroscopy. *Cornea,* 2003, vol. 22 (4), 363-370